(19) Europäisches Patentamt
European Patent Office
Office européen des brevets

(11) EP 1 468 284 B1

(12) EUROPEAN PATENT SPECIFICATION

(45) Date of publication and mention of the grant of the patent:
**06.08.2008 Bulletin 2008/32**

(21) Application number: **02777526.1**

(22) Date of filing: **13.11.2002**

(51) Int Cl.:
***G01N 33/50*** *(2006.01)* ***C12Q 1/66*** *(2006.01)*
***C12N 15/62*** *(2006.01)*

(86) International application number:
**PCT/GB2002/005120**

(87) International publication number:
**WO 2003/042693 (22.05.2003 Gazette 2003/21)**

(54) **METHOD FOR MEASURING INTRACELLULAR ATP AND/OR GENE EXPRESSION**

VERFAHREN ZUM NACHWEIS VON INTRAZELLULÄREN ATP UND/ODER GENEXPRESSION

TECHNIQUE PERMETTANT DE DOSER L'ATP INTRACELLULAIRE ET/OU L'EXPRESSION DES GENES

(84) Designated Contracting States:
**AT BE BG CH CY CZ DE DK EE ES FI FR GB GR IE IT LI LU MC NL PT SE SK TR**

(30) Priority: **14.11.2001 GB 0127292**
**06.03.2002 GB 0205201**

(43) Date of publication of application:
**20.10.2004 Bulletin 2004/43**

(73) Proprietor: **THE SECRETARY OF STATE FOR DEFENCE**
**Salisbury**
**Wiltshire SP4 0JQ (GB)**

(72) Inventors:
- **DAY, John, Cavendish**
  **Oxford OX1 3SR (GB)**
- **SQUIRREL, David, James**
  **Salisbury,**
  **Wiltshire SP4 0JQ (GB)**
- **BAILEY, Mark, John**
  **Oxford OX1 3SR (GB)**
- **WHITE, Peter, John**
  **Salisbury,**
  **Wiltshire SP4 0JQ (GB)**

(74) Representative: **Greaves, Carol Pauline et al**
**Greaves Brewster,**
**Indigo House,**
**Cheddar Business Park,**
**Wedmore Road,**
**Cheddar, Somerset BS27 3EB (GB)**

(56) References cited:
**WO-A-96/22376** **WO-A-98/46729**

- **DATABASE EMBL [Online] 26 June 2001 (2001-06-26), XP002256478 retrieved from EBI Database accession no. AAB82087 & WO 01/025426 A (KIKKOMAN CORP.) 12 April 2001 (2001-04-12)**
- **DATABASE EMBL [Online] 1 December 2001 (2001-12-01), XP002256479 Database accession no. Q95YI4 & GOMI K ET AL: J BIOL CHEM, vol. 276, no. 39, 28 September 2001 (2001-09-28), pages 36508-13, XP002227525**
- **DAY J C ET AL: "Structure and evolution of the luciferin-regenerating enzyme (LRE) gene from the firefly Photinus pyralis." INSECT MOLECULAR BIOLOGY, vol. 12, no. 4, 20 August 2003 (2003-08-20), pages 365-372, XP002256477 ISSN: 0962-1075**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to applications for a bioluminescent signalling system, in particular comprising luciferase and luciferin, as well as to certain novel genes and proteins used in the process, and to the production of elements used in the system.

Bioluminescent signalling systems are well known and have a wide range of applications in biotechnology, in particular in the fields of detection of micro-organisms, or as physiological reporters in investigations of cell activity.

One of the commonest bioluminescent signalling systems utilises a combination of the enzyme luciferase, found in nature in organisms such as fireflies and glow-worms, and the enzyme substrate, luciferin. In the presence of adenosine triphosphate (ATP) found in all cells, luciferase oxidises luciferin to produce oxyluciferin and cysteine as well as a bioluminescent signal, which may be monitored for example using a luminometer.

The signalling reaction can be represented as follows:

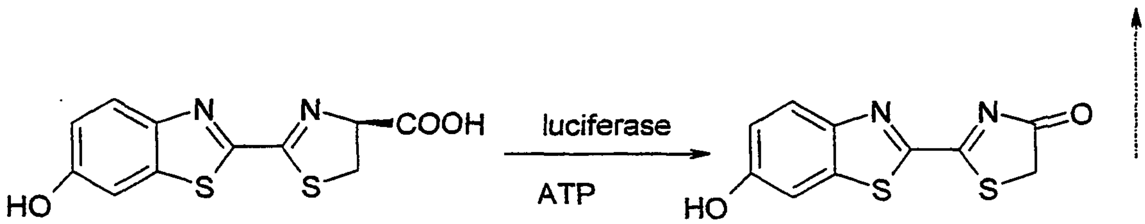


D-luciferin　　Oxyluciferin

where the dotted arrow indicates a bioluminescent signal.

Oxyluciferin is however an inhibitor of the reaction, which means that any signal generated in such a system is very short lived. Furthermore, it is necessary to provide a considerable excess of D-luciferin to any reaction system to ensure that an adequate and measurable signal is generated.

Examples of assays for the detection of microorganisms and the like, which use such a system include those described for example in WO 94/17202 and WO 96/02665.

Intracellular ATP concentrations can vary 10-fold or more depending upon a cell's state of health or developmental stage. It is of great value to be able to measure fluctuations in intracellular ATP levels as a means of investigating e.g. the effects of drugs, toxins, hormones, environmental agents or disease on cells.

Various methods for analysing the concentration of ATP *in vivo* are suggested in the art. For instance, in Dementieva et al (1996) Biochemistry (Moscow) Vol 61, No. 7., the intracellular concentration of ATP was measured in *E. coli* by calculating the total amount of ATP present using a recombinant luciferase, and dividing by an estimated total cell volume.

Such an indirect approach can at best produce only an estimate of the actual ATP concentration.

Luciferase has sometimes been used as a marker for gene expression (*in vivo*) where its production in a cell is linked to a particular genetic control element. Luciferin is added exogenously and intracellular ATP concentrations, under almost all conditions, will be such that the enzyme is saturated. Thus the switching on of gene expression is signalled by light that is emitted in a quantitative manner according to the amount of active luciferase that is generated.

It is generally the concentration of luciferase which is measured; this concentration is then correlated with a different event e.g. the efficiency of a promoter. It is known that using luciferases with reduced Michaelis-Menten constant $K_m$ for ATP (see e.g. WO 96/22376) ensures that changes in the ambient [ATP] does not interfere with the assay.

A mutant luciferase, which may be particularly useful in cellular ATP assays conducted *in vivo* because of the higher than normal Michaelis-Menten constant $K_m$, is described in WO 98/46729. By using luciferases which have an increased $K_m$ compared with those already known in the art, the possibility.of using these enzymes to measure steady state ATP concentrations over a wide range. This is because, generally speaking, the relationship between enzyme velocity (V, as measured by light intensity) and substrate concentration (of ATP, where luciferin is in excess) is as follows:

$$V = V_m . [ATP] / K_m + [ATP]$$

It can therefore be seen that only when the $K_m$ is greater than (or of a similar order as) the ambient [ATP] will there be a degree of proportionality between changes in [ATP] and changes in light intensity. Where the $K_m$ is much less than the ambient [ATP], any changes in [ATP] will not tangibly effect the measured light intensity. Clearly the more sensitive the light detection is, the smaller the measurable changes in 'V' can be, and the smaller the $K_m$ can be with

respect to the [ATP] range being assessed.

For certain applications, e.g. *in vivo* measurements, it may be advantageous to have a luciferase wherein the $K_m$ is of the order of between 400 μm to 1.4 mM e.g. 500 μm, 600 μm, 1 mM etc. However, as can be appreciated from the discussion above, the main criterion is that the $K_m$ is not much less than the expected [ATP] range to be assessed.

A particular expected [ATP] range which is important for physiological assays of blood cells is between 300 μm and 1 mM, or more particularly 380 μm and 620 μm, (cf. Sigma Diagnostic Kit, Catalog No. 366 discussed above). For other mammalian cells such as hepatocytes, the [ATP] range is 2.5 mM - 6 mM (see Dementieva et al (1996) discussed above). Use of the recombinant luciferases such as those described in WO 96/22376 are particularly suitable for continuous assays in these ranges.

In all such assays, however, as well as those used as physiological reporters of cell health, for example during screening for drugs etc., the cell is suitably transformed so that it expresses a luciferase enzyme. However, as mentioned above, luciferin must be applied exogenously. Unfortunately, luciferin is only cell-permeable at pH<5, which is not generally regarded as being a physiologically acceptable pH. Thus addition of this reagent to a conventional cellular assay is not easy. In order to monitor the ATP levels of the cell over a protracted period, it is necessary to maintain this low pH over that period, which could in itself have a detrimental effect on the cells. This limits application of assays of this type, in particular in high-throughput screening where robust methodology is required.

Furthermore, although luciferase enzyme may be produced using recombinant DNA technology, D-luciferin is generally produced synthetically, as a mixture of the desired D-isomer and the unwanted L-isomer, which must be separated prior to use. This is a wasteful procedure.

US Patent No. 5,814,504 describes a 40kD protein, isolated from firefly species, which produces firefly D-luciferin when combined with oxyluciferin and D-cysteine. This protein is said to be useful in improving the durability of the luminescent signal from the luciferase/luciferin reaction system and in reducing the amount of luciferase and luciferin used in the reaction. Methods of producing firefly luciferin using this protein are also described. The amino acid sequence of this protein and the corresponding mRNA from *Photinus pyralis* are available on the NCBI database as Accession Number BAB60700 and Accession Number AB062786 respectively.

The sequence of other luciferase recycling enzymes, derived from *Luciola cruciata* and *Luciola lateralis* have also been published (see BAB85479 and BAB85478 respectively).

According to the present invention there is provided a method for measuring intracellular ATP and/or gene expression, which method comprises transforming a cell with a construct which encodes a luciferase and a luciferin recycling protein, introducing luciferin into said cell, and monitoring bioluminescent signals from said cell.

The expression "luciferin recycling protein" or LRE refers to proteins which convert oxyluciferin and cysteine to luciferin. An example of such a protein is that described in USP 5,814,504 .

The method of the invention can be used to monitor intracellular ATP levels. Alternatively or additionally it may be used to monitor expression of either the luciferase gene or the luciferin recycling gene since the signal produced will be related to expression levels of either of these, although more directly related to the expression of luciferase.

The method of the invention has significant advantages in allowing *in vivo* gene expression to be reported with a stable light output. A single luciferin charge may be introduced into the cell at the beginning of the cell assay, during a brief exposure to pH 5. Thereafter the cell can be restored to physiological pH during the assay. Although the luciferin will be used rapidly in the system, the oxyluciferin produced will be converted back to D-luciferin by the action of the luciferin recycling protein expressed within the cell, and cysteine, in particular D-cysteine, present within the cell.

As a result, a relatively stable light output is achievable, which provides a signal that is easier to read. There may be particular advantages to a system of this type.

The luciferase enzyme and the luciferin recycling protein may be expressed within the cell as two separate proteins. In this case, the construct used to transform the cell may be a two part construct, one part containing the gene encoding the luciferase enzyme, and the second part containing the gene encoding the luciferin recycling protein. Preferably the luciferase enzyme and the luciferin recycling protein are expressed together as a fusion protein. By genetically linking the luciferase activity and the recycling activity, the kinetics of the reaction, (either in-vivo or in-vitro) become more favourable. Such fusion proteins form a further aspect of the invention.

If necessary, the cellular content of D-cysteine may be increased, for example by further transforming the cell so that it expresses an L-cysteine racemase.

In a preferred embodiment, the cell is transformed such that it expresses two luciferase enzymes, one with a relatively high $K_m$ value and one with a relatively low $K_m$ value. Suitably these have outputs at different wavelengths, which may be distinguished. In this system, the useful range of the assay may be extended. Alternatively or additionally, it allows a ratiometric assay to be conducted, where the activity of the high $K_m$ luciferase is compared with that of the low $K_m$ luciferase. In this way, cellular physiology may be continuously monitored, for example so that cell poisons or other adverse effects of a sample being screened, could be quickly detected.

Examples of luciferase mutants with a relatively low $K_m$ value are described in WO 96/22376, and these may be used in the context of the invention.

Novel constructs used in transforming the cells for use in the method of the invention form a further aspect of the invention. Thus, the invention further provides a DNA construct comprising (i) a nucleic acid sequence which encodes a luciferase enzyme, and (ii) a nucleic acid sequence which encodes a luciferin recycling protein. These elements are preferably under the control of a suitable promoter. Where the elements are expressed as two proteins, the nucleic acid sequence which encodes the luciferase enzyme will be under the control of a first promoter, and the nucleic acid sequence which encodes the luciferin recycling protein will be under the control of a second promoter.

Preferably, however, the nucleic acid sequences of these elements are linked so as to express a fusion protein of the luciferase and the luciferin recycling enzyme. In this case, the nucleic acid sequence is under the control of a single promoter.

As described above, the construct may suitably comprise one or more additional components selected from (iii) a nucleic acid sequence which encodes a further luciferase enzyme; and (iv) a nucleic acid sequence which encodes an L-cysteine racemase enzyme. Elements (iii) and (iv) are suitably under the control of third and fourth promoters respectively.

The construct is suitably in the form of one or more vectors that may be used in cell transformation. Where the elements of the construct are present on more than one vector, these may be used to co-transform the target cell.

Cells transformed in this way may be eukaryotic or prokaryotic. For cellular assays, it might be envisaged that eukaryotic cells, such as mammalian or plant cells would be required. Vectors and promoters are selected such that they are active in the target cell type, as is conventional in the art.

Nucleic acids used in the constructs of the invention encode proteins having the specified activity. It may be preferred that at least some of the codons used in these nucleic acids are "optimised" for the target cell species, as is conventional in the art. For example, particular cell types will express more effectively nucleic acids with a particular percentage GC content, and as a result of the degeneracy of the genetic code, codons may be selected so that the nucleic acids have a %GC content resembling this.

Examples of nucleic acid sequences of elements (i) and/or (iii) above are well known in the art. They suitably encode luciferases having desired properties such as thermostability, $K_m$ values and colorimetric properties. Examples of mutant luciferases which are suitably encoded by the nucleic acids used in the constructs of the invention are described in EP-A-0528448, WO95/25798 WO 96/22376, WO 98/46729, WO 00/24878, WO 01/31028, WO 99/14336 and WO 01/20002.

Examples of suitable L-cysteine racemase enzymes (element (iv) above) are known in the art. For example, the enzyme amino acid racemase, with low substrate specificity, from *Pseudomonas putida* (designated EC 5.1.1.10 on the EcoCyc database) is known to catalyse the conversion of L-amino acids to D-amino acids.

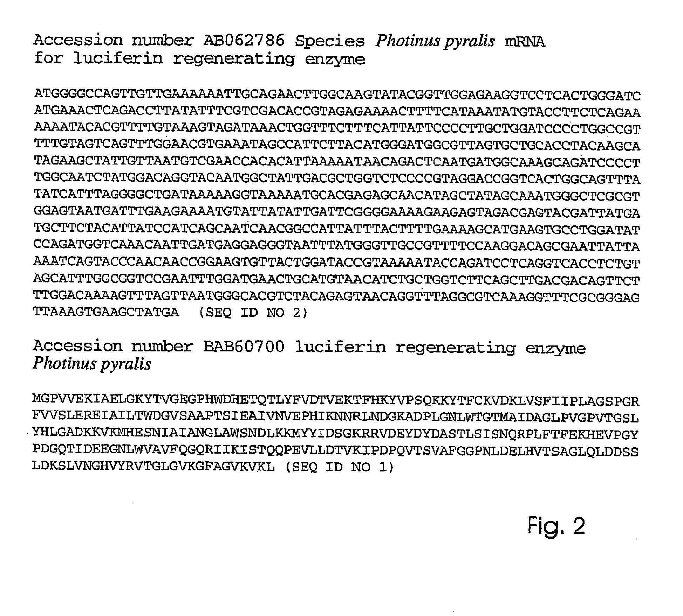

An example of a nucleic acid which may be used in element (ii) of the construct of the invention encodes a protein of SEQ ID NO 1 as illustrated hereinafter in Figure 2, or a luciferin recycling fragment or variant thereof. This protein is obtainable from *Photinus pyralis*.

Other known examples of luciferin recycling enzymes are proteins obtainable from Luciola species such as *Luciola cruciata* and *Luciola lateralis*. Particular examples of such sequences are given hereinafter as SEQ ID NO 62 and 63. Thus the nucleic acids included in the constructs of the invention may encode proteins of SEQ ID NOS 62 or 63, or luciferin recycling fragments thereof, or variants of any of these.

As used herein, the term "fragment" refers to one or more portions of the basic sequence which has the required enzyme activity. These may be deletion mutants. Generally speaking the portions will comprise at least 12 and preferably at least 20 amino acids of the basic sequence.

The expression "variant" includes allelic variants and variants found at different loci as a result of the presence of multiple gene copies. In addition, the term "variant" includes sequences of amino acids or nucleic acids, which encode them, which differ from the base amino acid sequence from which they are derived in that one or more amino acids within the sequence are substituted for other amino acids. Amino acid substitutions may be regarded as "conservative" where an amino acid is replaced with a different amino acid with broadly similar properties. Non-conservative substitutions are where amino acids are replaced with amino acids of a different type. Broadly speaking, fewer non-conservative substitutions will be possible without altering the biological activity of the polypeptide. Suitably variants will be at least 60% homologous, preferably at least 75% homologous, and more preferably at least 90% homologous to the base sequence. Homology in this instance can be judged for example using the algorithm of Lipman-Pearson, with Ktuple:2, gap penalty:4, Gap Length Penalty:12, standard PAM scoring matrix (Lipman, D.J. and Pearson, W.R., Rapid and Sensitive Protein Similarity Searches, Science, 1985, vol. 227, 1435-1441).

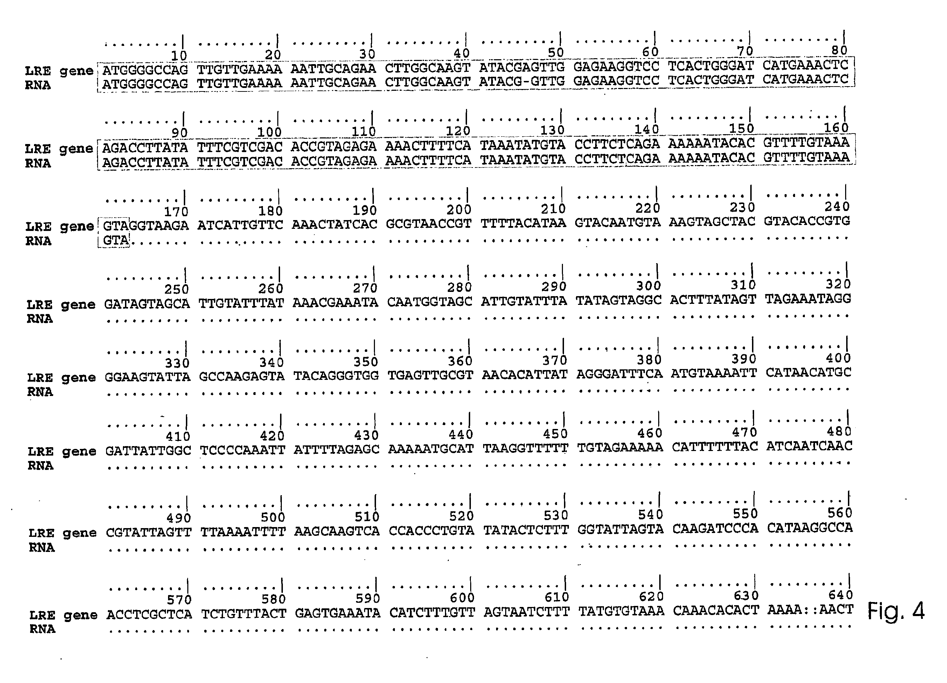

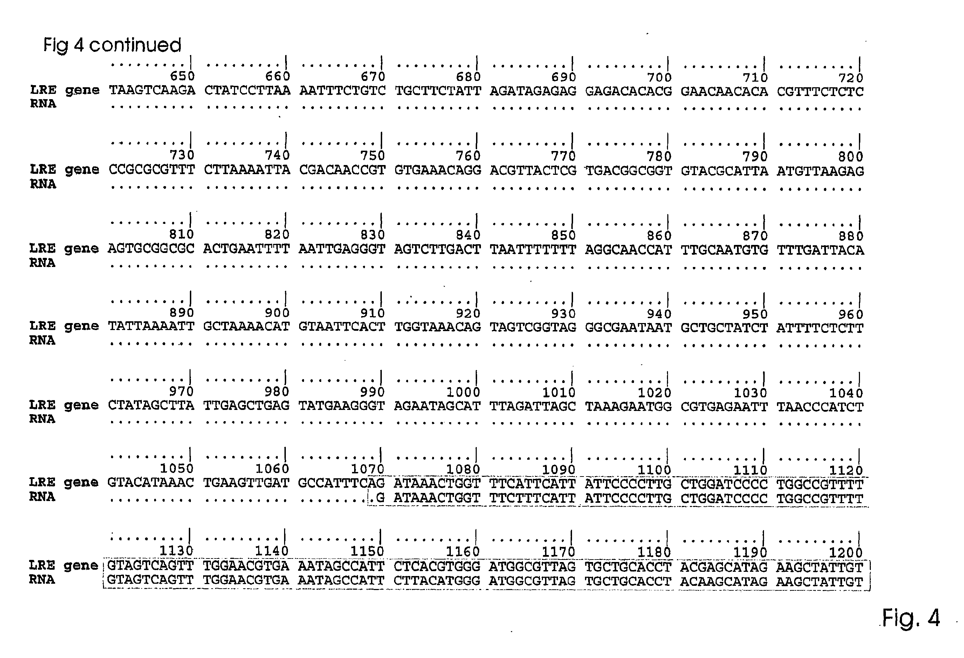

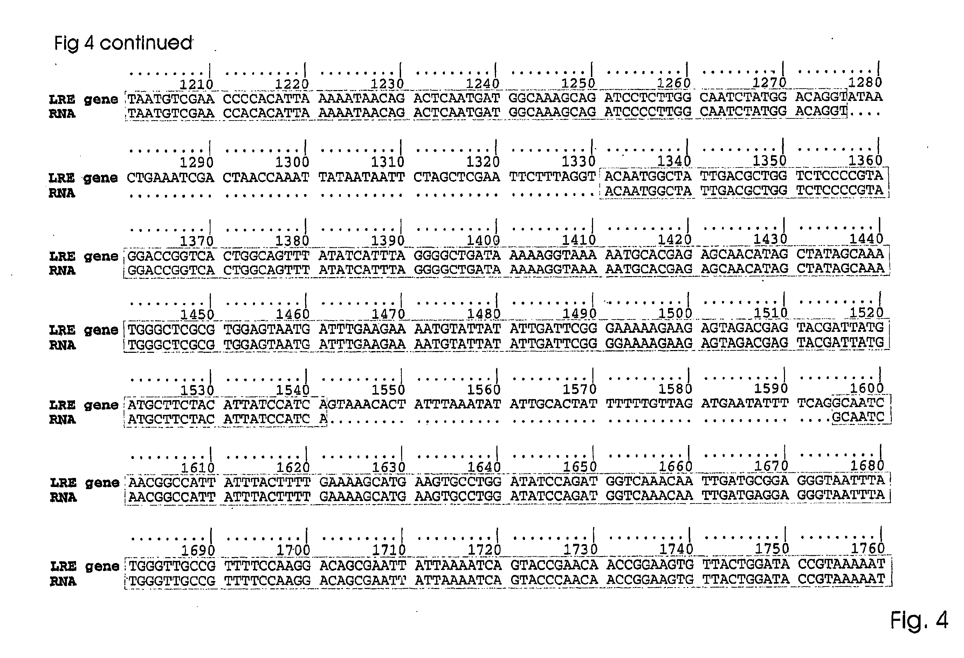

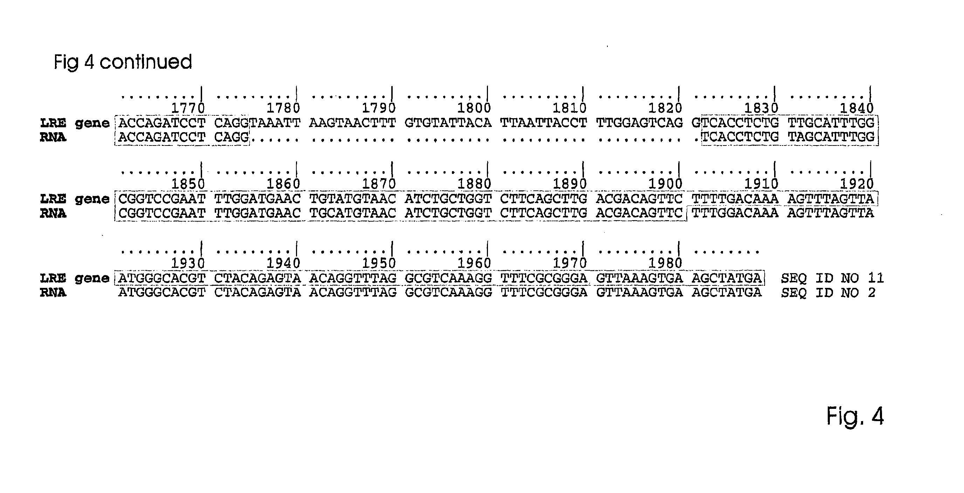

A particular example of a nucleic acid which encodes SEQ ID NO'1 is the mRNA sequence shown as SEQ ID NO 2 in Figure 2 hereinafter. An example of a suitable genomic sequence, which encodes the protein of SEQ ID NO 1, is SEQ ID NO 11 as illustrated in Figure 4 hereinafter.

In an alternative embodiment, the construct of the invention includes as element (ii) a nucleic acid which encodes a luciferin recycling protein obtained from a glow worm species such as Lampyris noctiluca, or luciferin recycling fragements thereof, or variants of any of these.

In particular, the nucleic acid encodes a luciferin recycling protein comprising SEQ ID NO 3, as shown in Figure 10 hereinafter, or a luciferin recycling protein comprising SEQ ID NO 39, as shown in Figure 12, or a luciferin recycling protein comprising SEQ ID NO 59, as shown in Figure 16 or a luciferin recycling fragment or variant of any of these.

Luciferin recycling protein comprising SEQ ID NO 3, SEQ ID NO 39 or SEQ ID NO 59, together with luciferin recycling fragments, and variants of any of these having at least 60%, more preferably at least 80%, yet more preferably at least 90% and most preferably at least 95% homology or identity, may be used in the present invention.

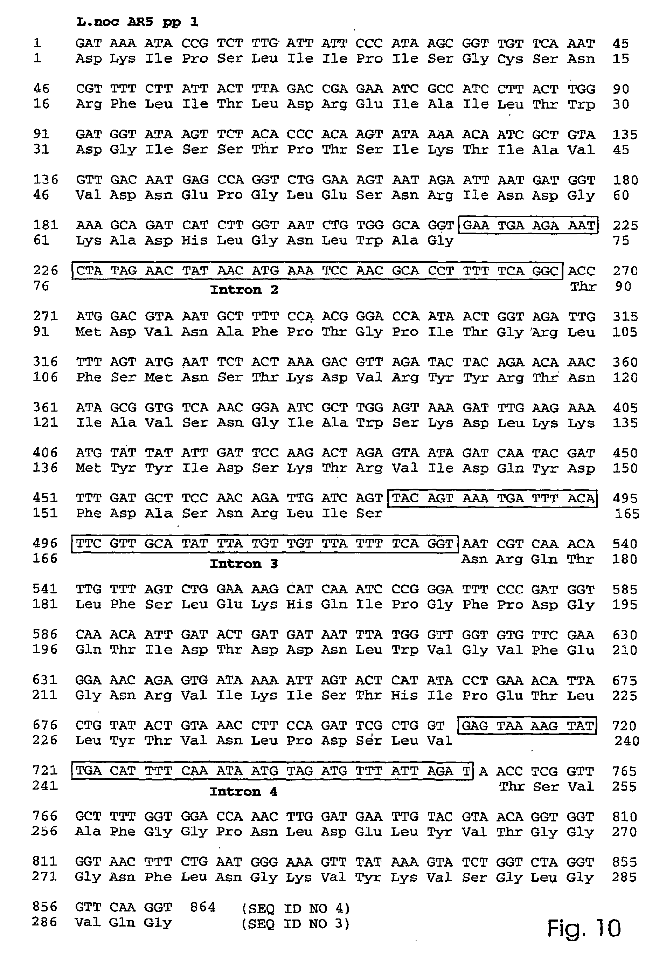

A particular embodiment of the invention makes use of a luciferin recycling protein comprising SEQ ID NO 3, as illustrated hereinafter in Figure 10.

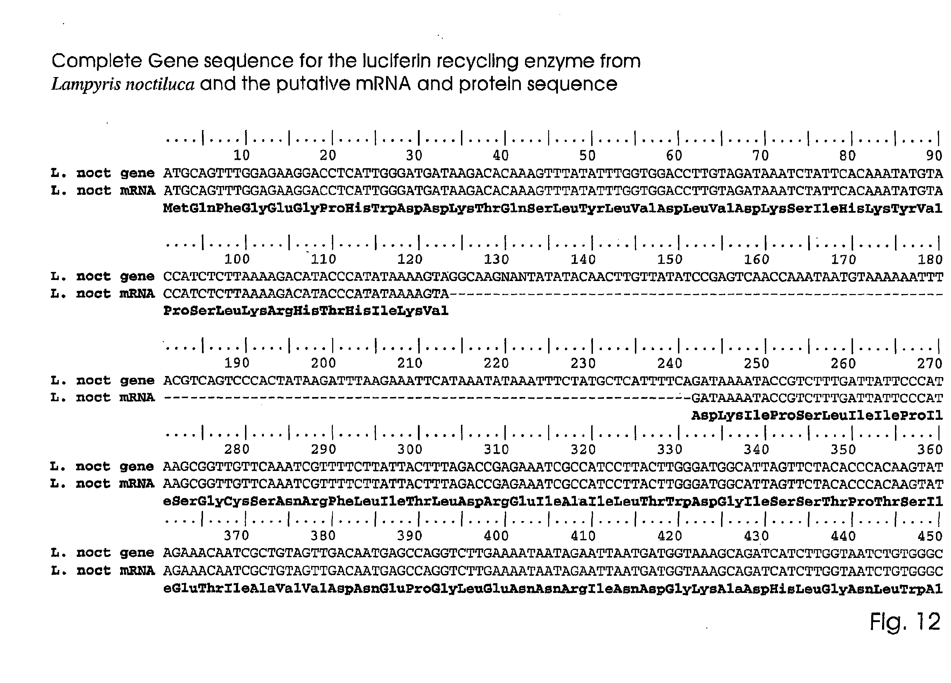

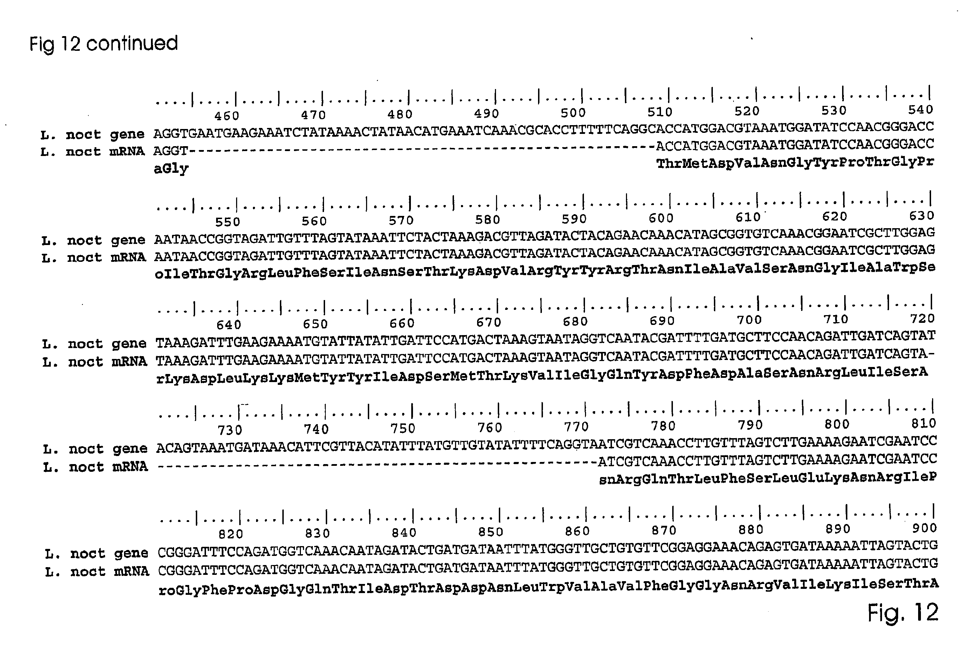

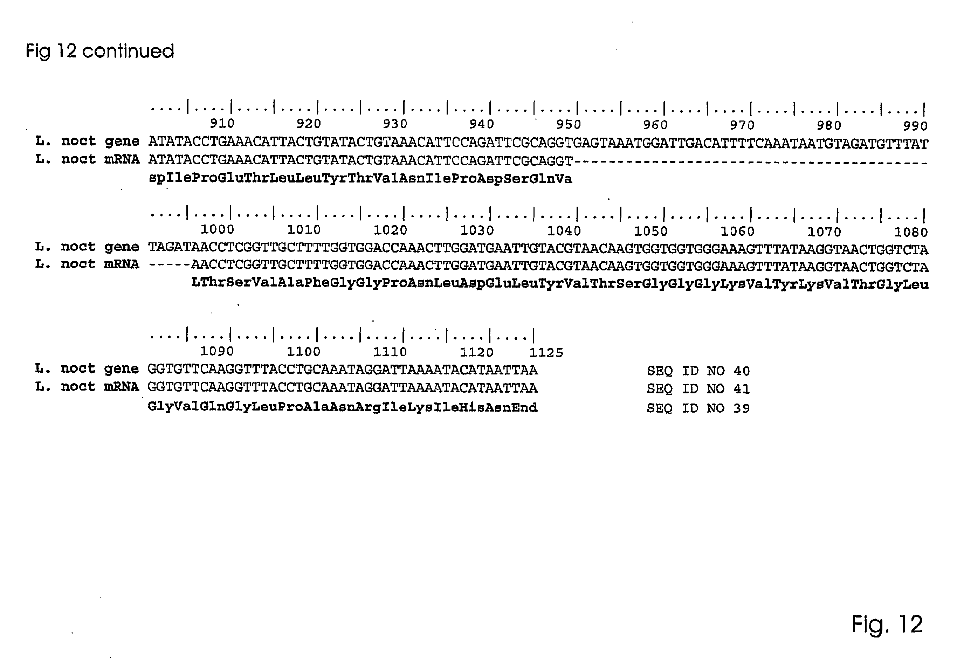

An alternative embodiment of the invention makes use of a luciferin recycling protein comprising SEQ ID NO 39 as illustrated in Figure 12.

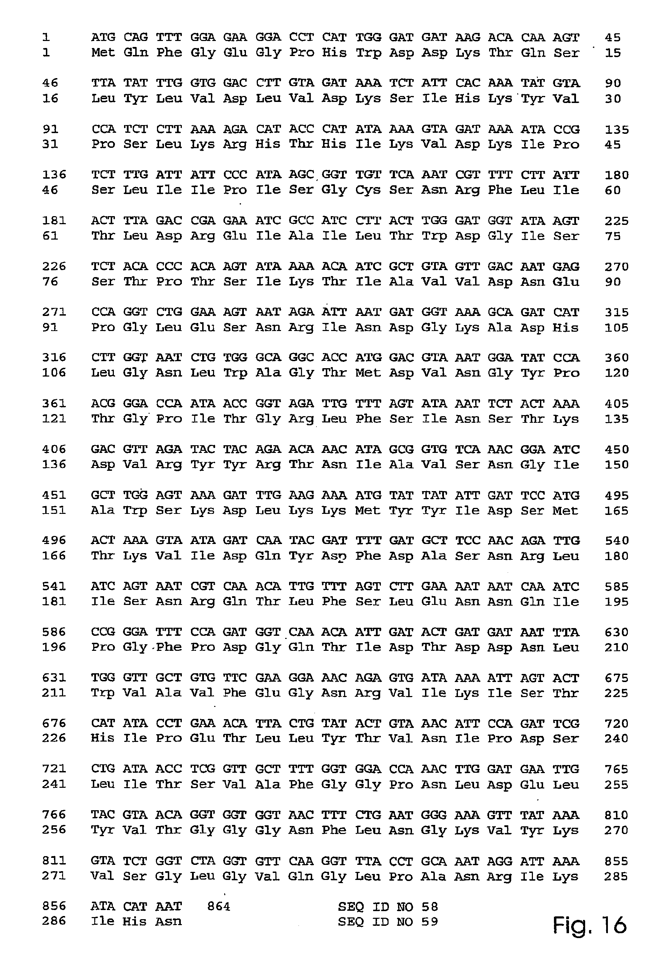

A preferred embodiment of the invention makes use of a luciferin recycling protein comprising SEQ ID NO 59 in Figure 16.

Such proteins and in particular proteins of SEQ ID NO 59, are obtainable from *Lampyris noctiluca* and therefore allelic variants are also obtainable from this species. The sequence was cloned and partially sequenced as described in Example 1 hereinafter. The cloning process however was not straightforward, due in part to the presence of large introns, which appear to be present in native genes for luciferin recycling proteins.

Further the present invention, may use a nucleic acid that encodes a luciferin recycling protein comprising SEQ ID NO 3, a luciferin recycling protein comprising SEQ ID NO 39, or a luciferin recycling protein comprising SEQ ID NO 59,or a luciferin recycling fragment thereof, or a variant of any of these having at least 60% sequence homology or identity. In particular, a nucleic acid which may be used in the invention encodes a luciferin recycling protein comprising SEQ ID NO 3, SEQ ID NO 39 or SEQ ID NO 59.

An example of such a sequence, which encodes SEQ ID NO 3, is the genomic sequence (including introns) SEQ ID NO 4 as shown in Figure 10. An example of a nucleic acid sequence which encodes a protein of SEQ ID NO 39 is SEQ ID NO 40 as shown in Figure 12. A suitable cDNA sequence comprises SEQ ID NO 4 without the illustrated introns (SEQ ID NO 41) or SEQ ID NO 38 as shown in Figure 12. In particular, the nucleic acid is of SEQ ID NO 58 as shown in Figure 16.

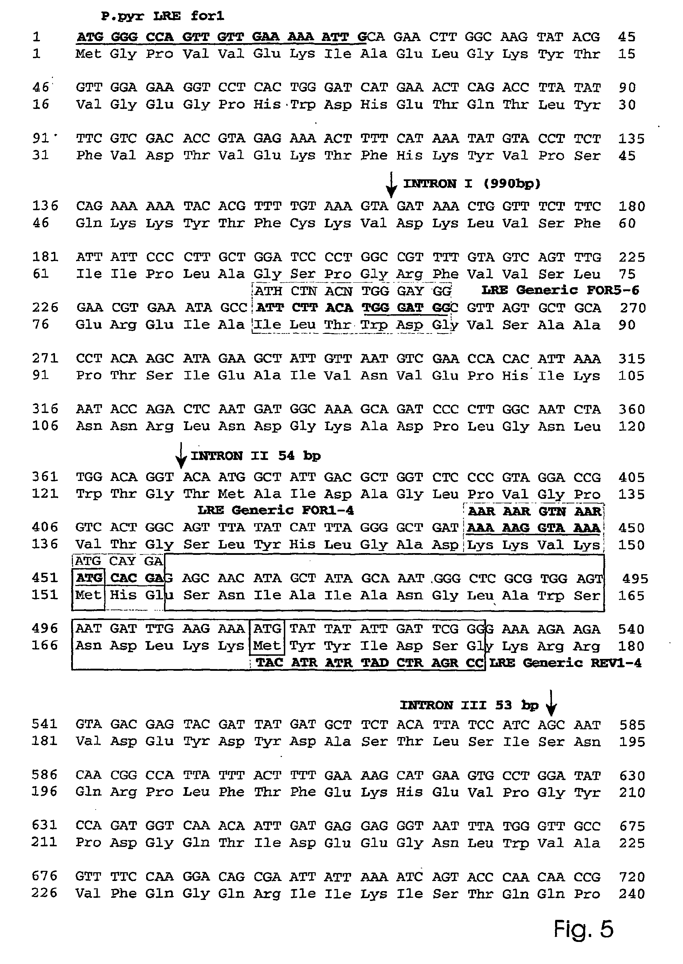

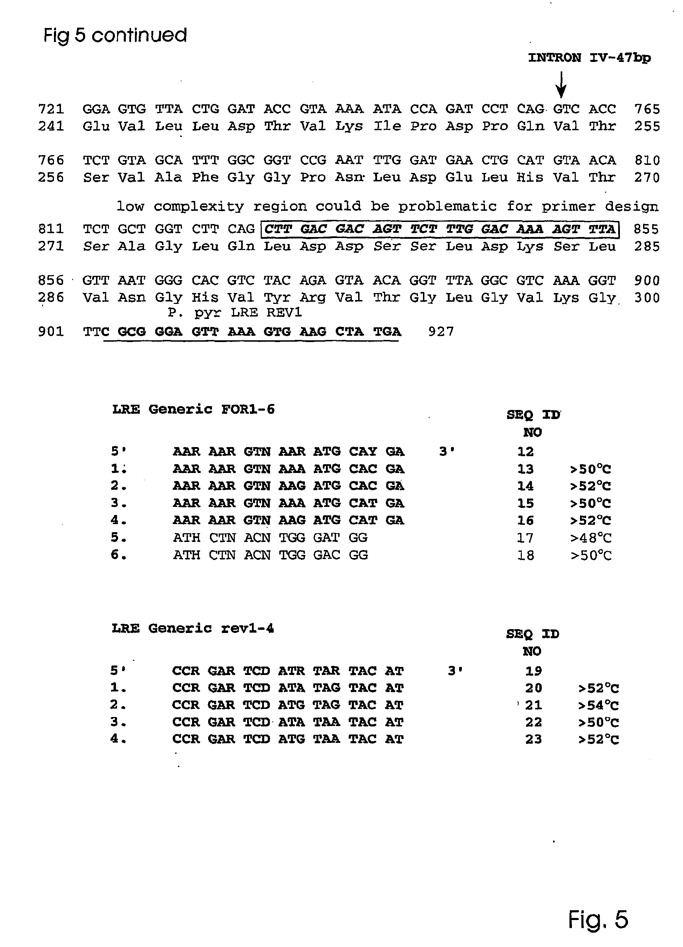

In addition to the proteins obtainable from particular species, chimeric luciferin recycling enzymes may be produced by combining fragments of enzymes from various species. In this context, the fragments suitably comprise regions encoded by individual exons found within the genomic sequences. It has been found for example, that generally the gene sequences encoding these proteins contain 5 exons, linked together by 4 introns of varying size, as illustrated hereinafter for example in Figures 5, 6, 7, 10, 12 and 14. Chimeric luciferin recycling proteins suitably comprise a combination of 5 individual exons, corresponding to exon I, II, III, IV and V within the sequences listed above.

Suitably, the chimeric enzyme contains at least one and preferably up to four fragments encoded by exons of a luciferin recycling gene from glowworm species such as *Lampyris noctiluca.* For example, the applicants have produced a luciferin recycling enzyme by splicing together exon 1 of a *Photinus pyralis* sequence, to a fragment of *Lampyris noctiluca* luciferin recycling enzyme corresponding to exons 2, 3, 4 and 5 within the gene.

An example of such an enzyme is illustrated hereinafter as SEQ ID NO 61. This sequence and luciferin recycling fragments and variants of either of these, together with nucleic acid sequences which encode these proteins, such as SEQ ID NO 60, may be used in the invention.

The novel proteins of the invention may be used in the production of synthetic D-luciferin, as well as other optically active enzyme substrates. For example they may be used in the regeneration of optically active substrates of phosphatase or β-galactosidase enzymes.

The invention will now be particularly described by way of example with reference to the accompanying drawings in which:

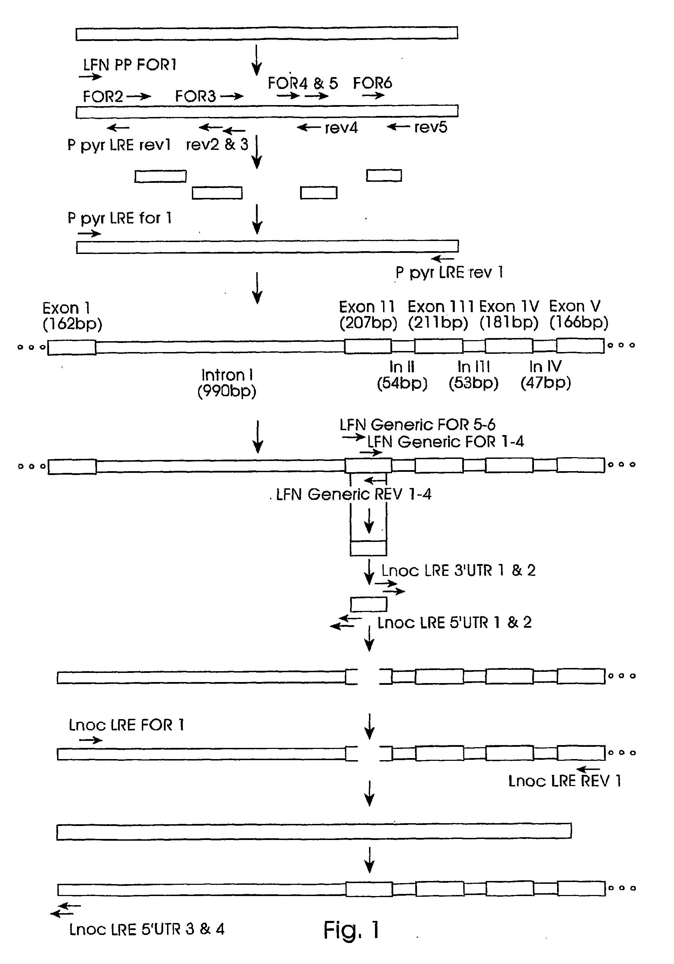

Figure 1 shows schematically the experimental design and results obtained for the characterisation of the gene coding for a luciferin-recycling protein (enzyme) (LRE) found in *L. noctiluca;*

Figure 2 shows the sequence of *Photinus pyralis* LRE mRNA and the corresponding protein sequence, obtained from the NCBA database (SEQ ID NO 2 and SEQ ID NO 1 respectively);

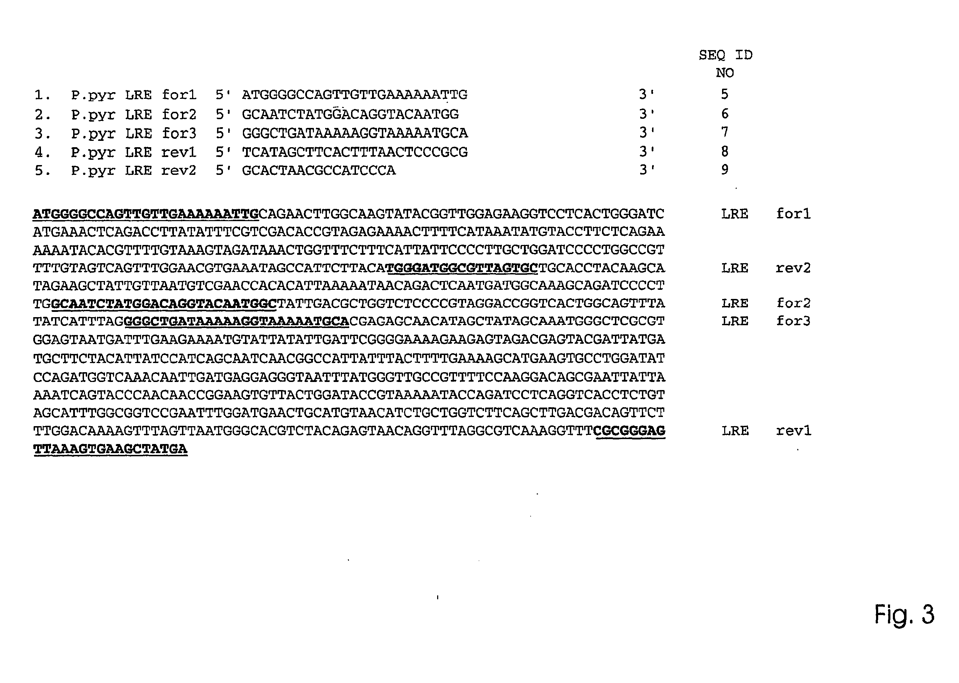

Figure 3 shows a series of primers designed to amplify the entire gene of *P. pyralis* LRE;

Figure 4 shows the complete sequence of the LRE gene from *P. pyralis* (SEQ ID NO 11) aligned against the mRNA sequence (SEQ ID NO 2); where exons are shown shaded;

Figure 5 illustrates the generic primers designed to amplify small region of a *L. noctiluca,* homologue or the *P. pyralis* LRE, based upon conserved amino acid sequences, and their position with respect to the amino acid sequence

(SEQ ID NO 1);

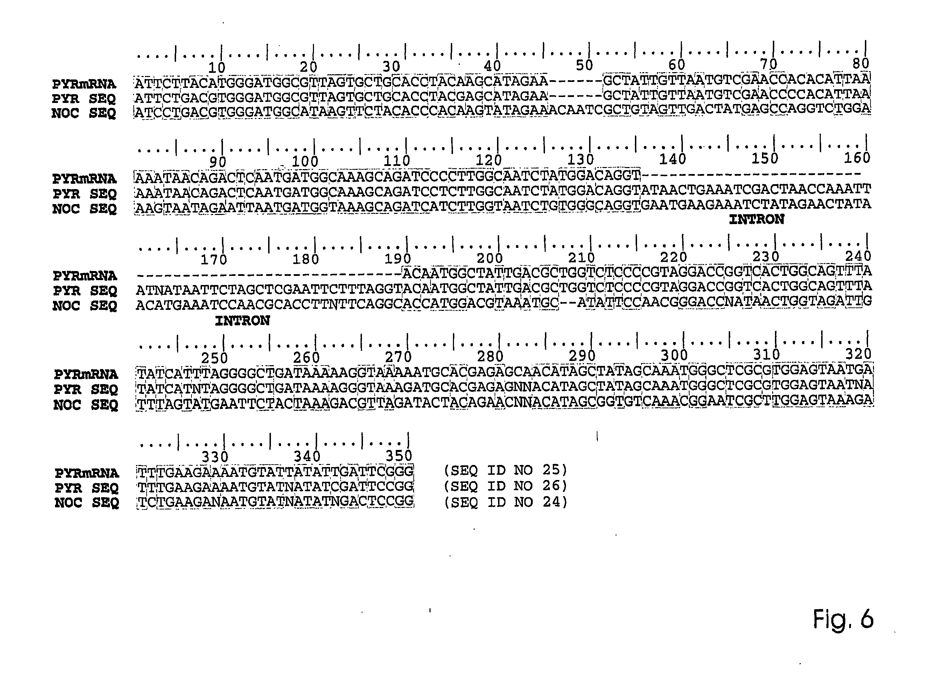

Figure 6 shows a 350 base pair PCR product from *L. noctiluca,* aligned with the *P*. *pyralis* mRNA and DNA sequences;

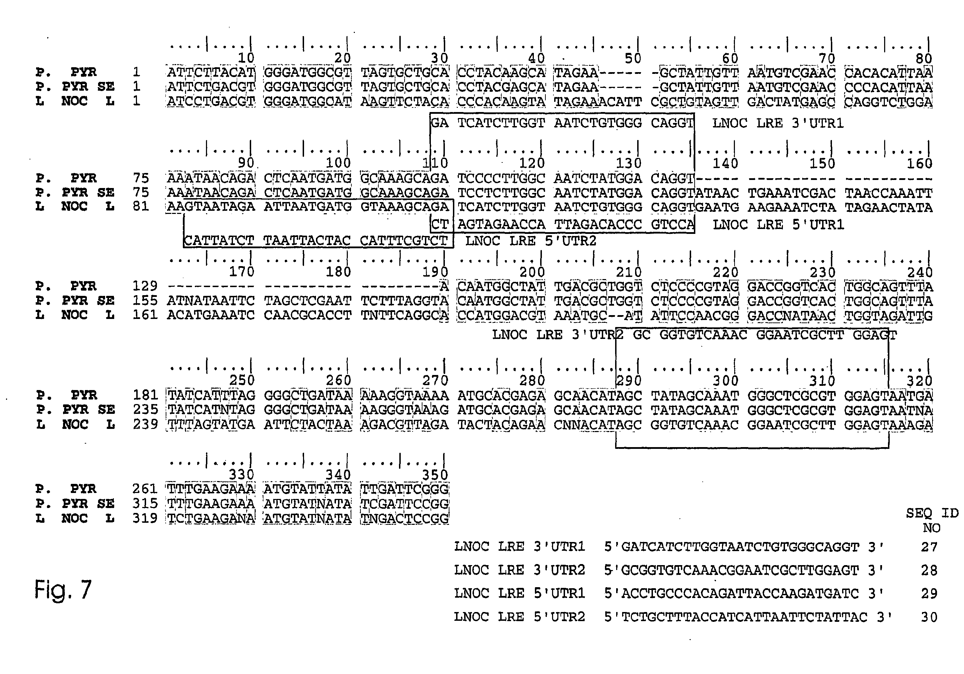

Figure 7 shows the primers used in a 5' and 3' genome walking experiment;

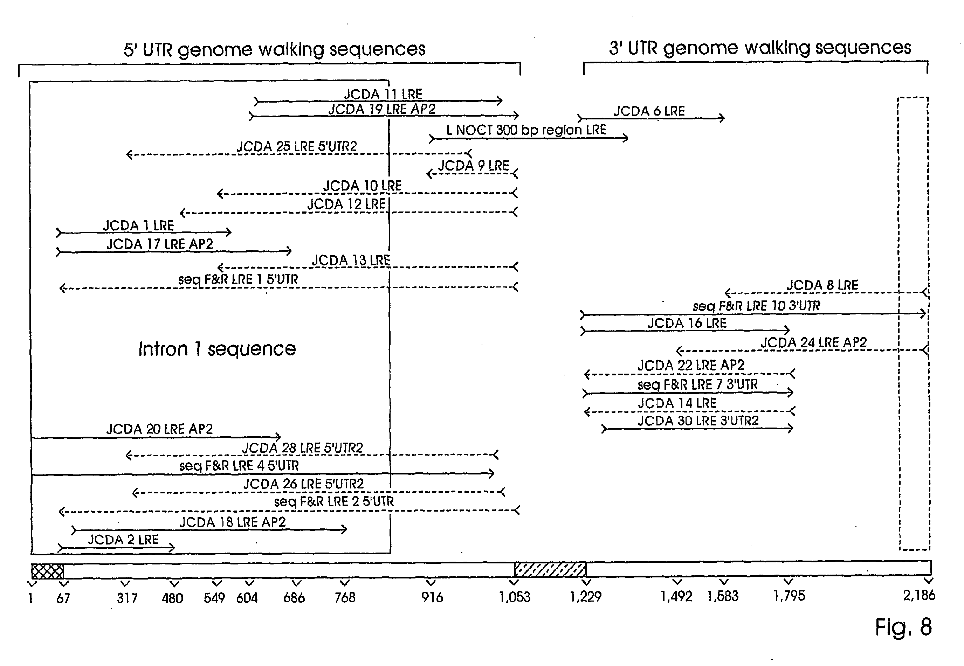

Figure 8 illustrates the contig constructed from the genome walking experiment;

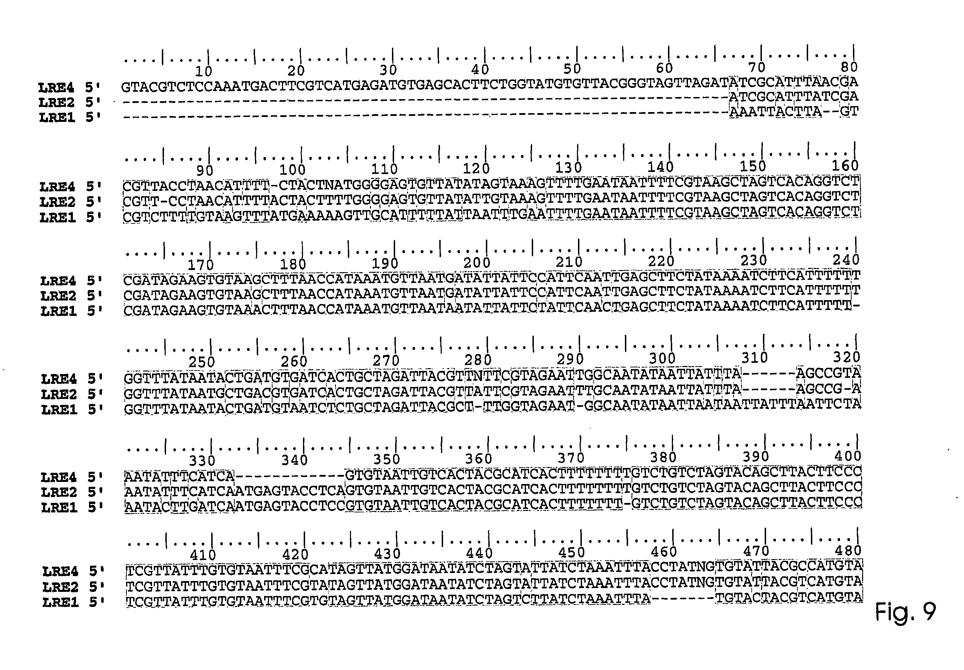

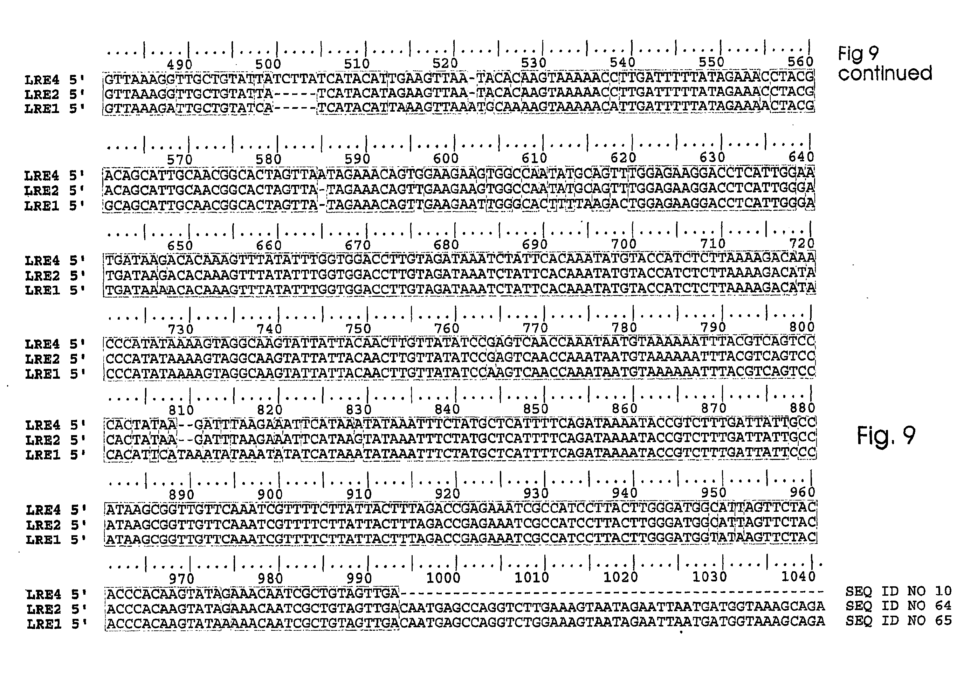

Figure 9 shows the 5' UTR genomic walking sequences from *L noctiluca* illustrating the presence of three forms of the gene;

Figure 10 shows a protein sequence (SEQ ID NO 3) and coding nucleic acid sequence (SEQ ID NO 4) comprising the LRE of *L*. *noctiluca;*

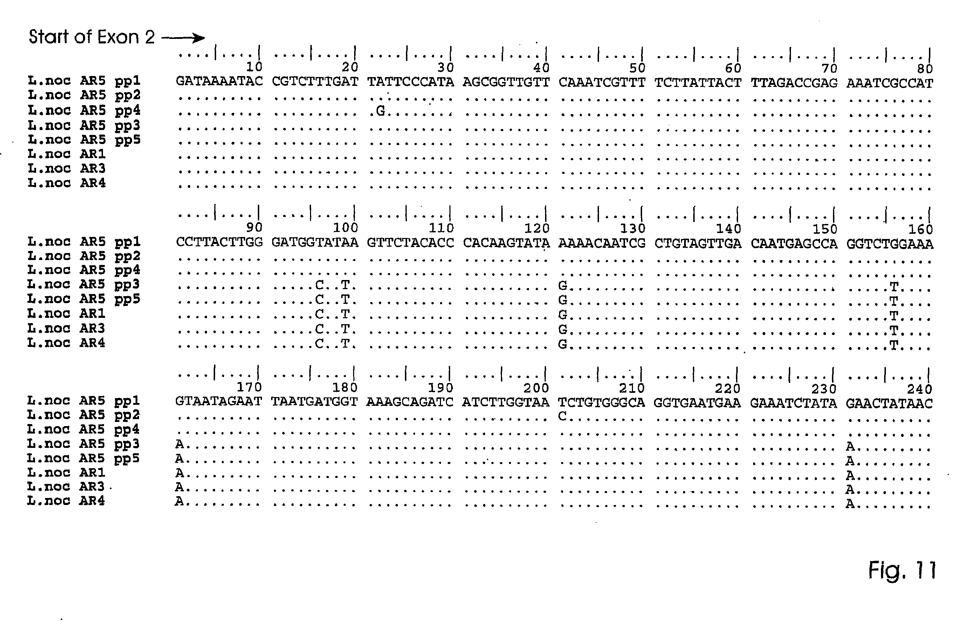

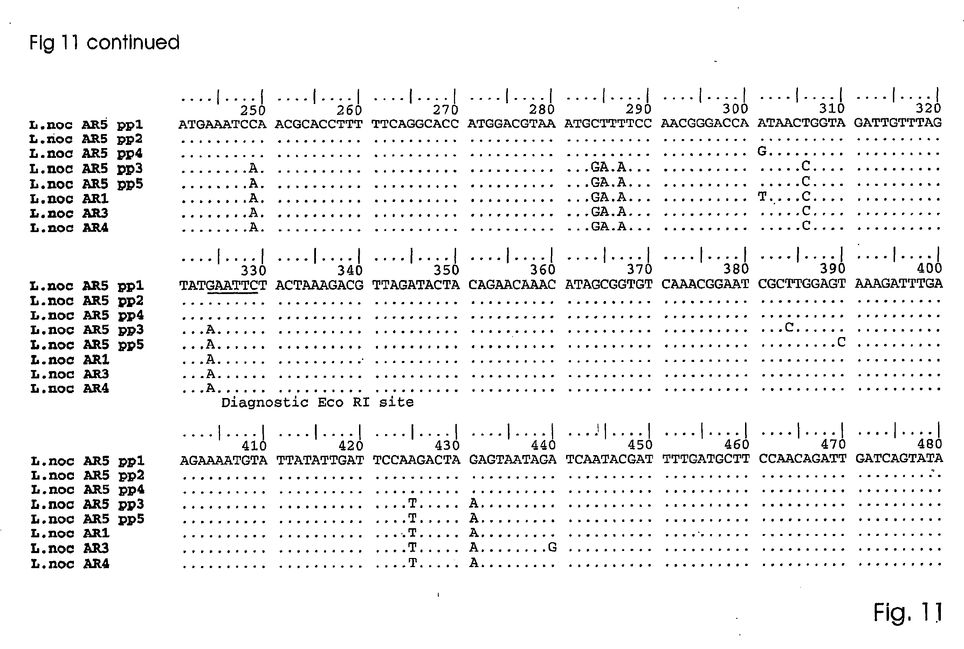

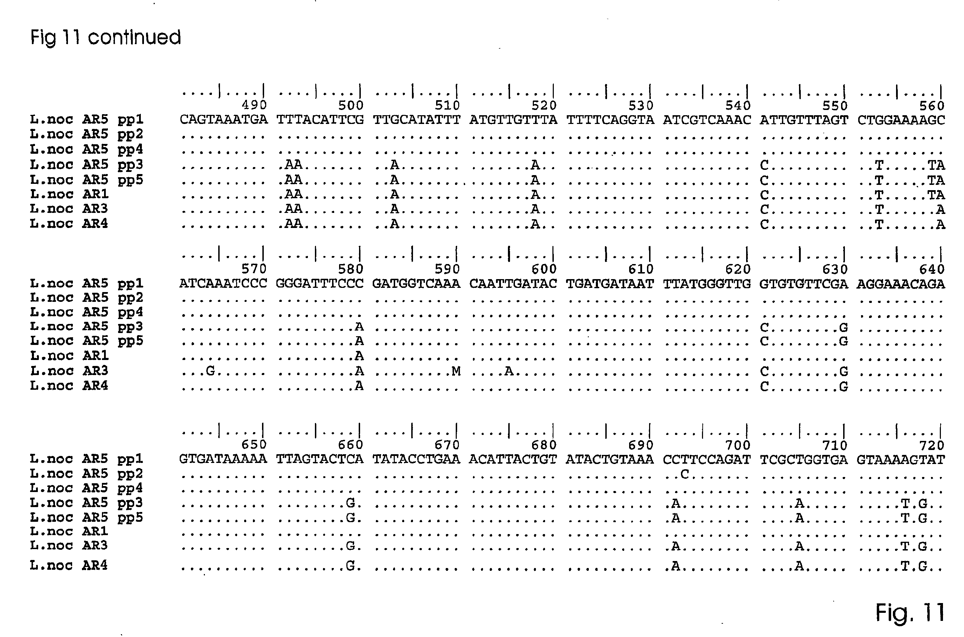

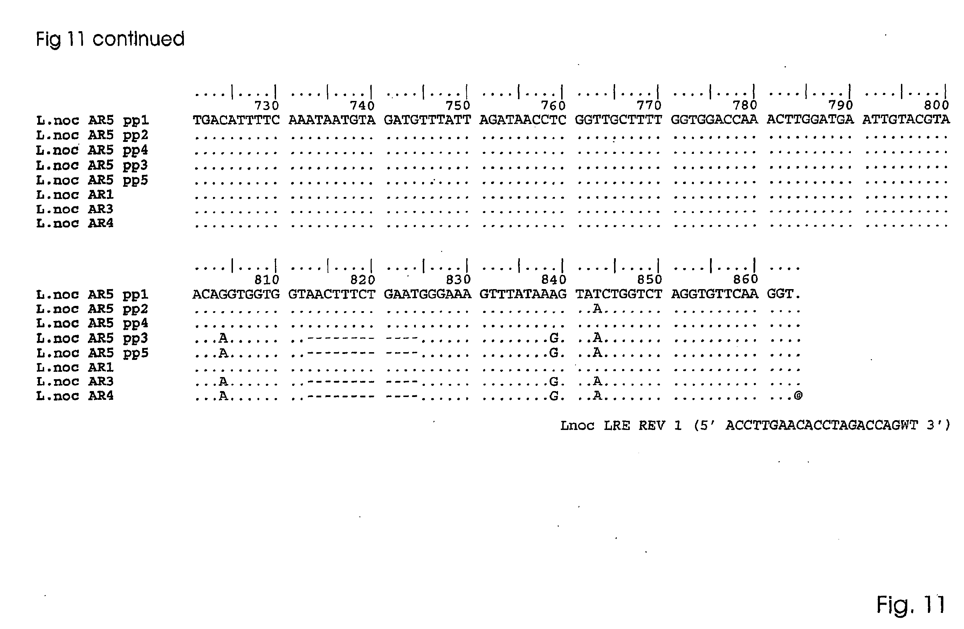

Figure 11 shows SEQ ID NO 4 and some allelic or other variants, derived from 4 different individuals;

Figure 12 shows a complete protein sequence (SEQ ID NO 39), the coding gene nucleic acid sequence (SEQ ID NO 40) and putative mRNA sequence (SEQ ID NO 38) comprising the LRE of *L*. *noctiluca*;

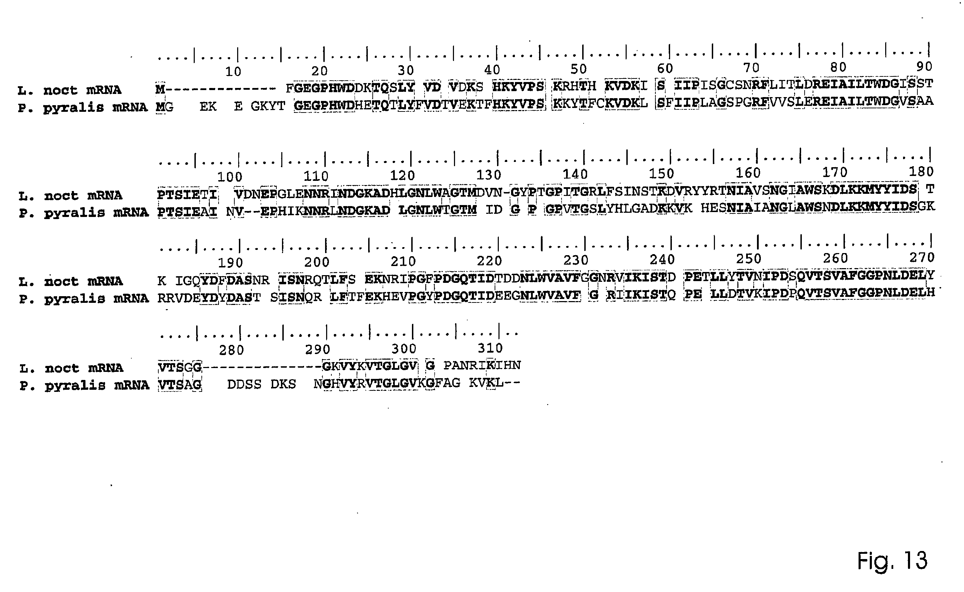

Figure 13 shows the putative amino acid sequence derived from the complete luciferin recycling enzyme gene sequence in *L*. *noctiluca* aligned against the LRE protein sequence from *Photinus pyralis* (Accession number BAB60700), which displays 55.7% amino acid homology;

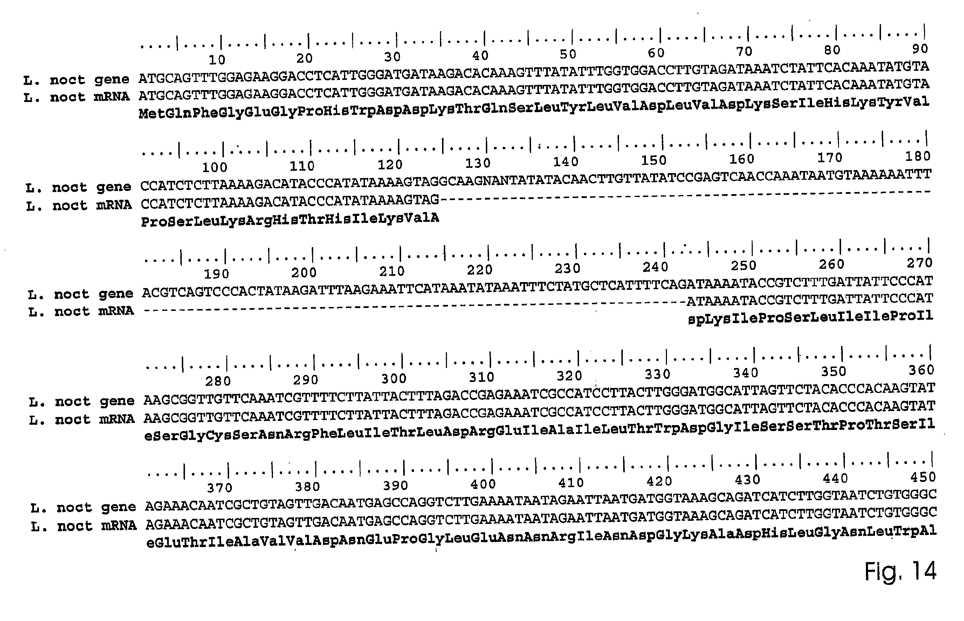

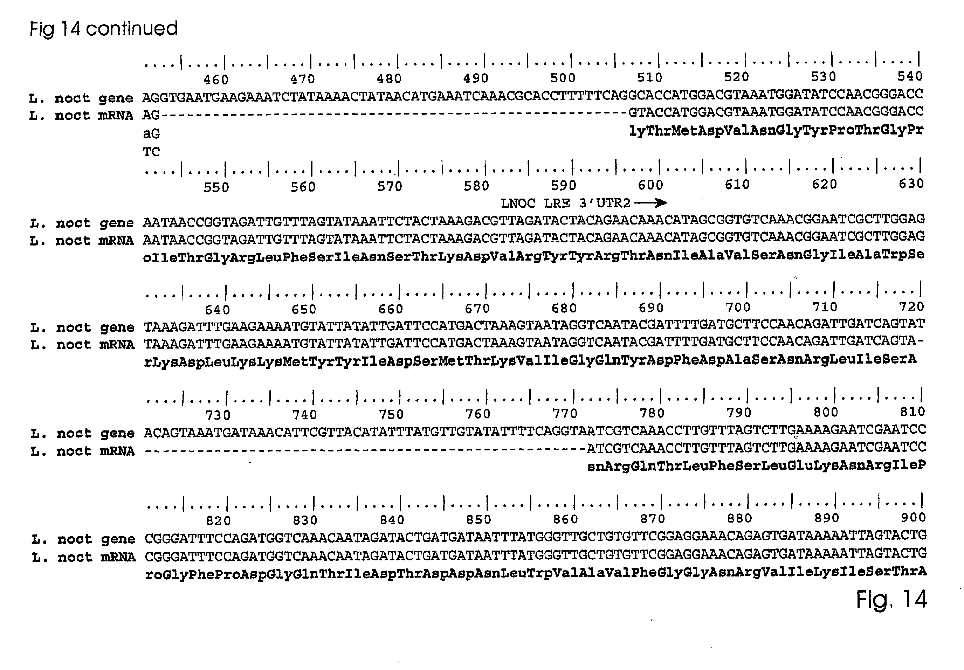

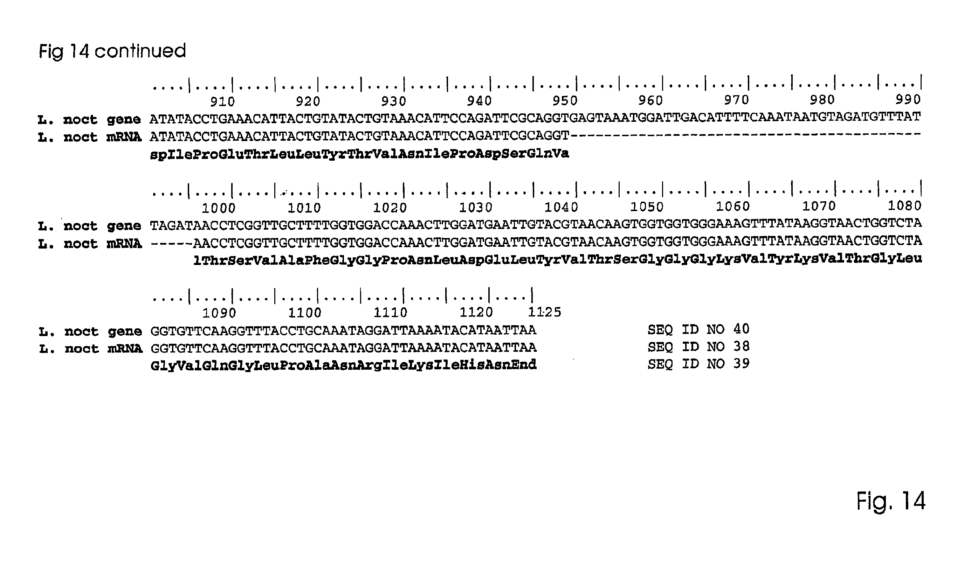

Figure 14 is similar to Figure 12 but showing a corrected position of intron regions within the genomic sequence (SEQ ID NO 40);

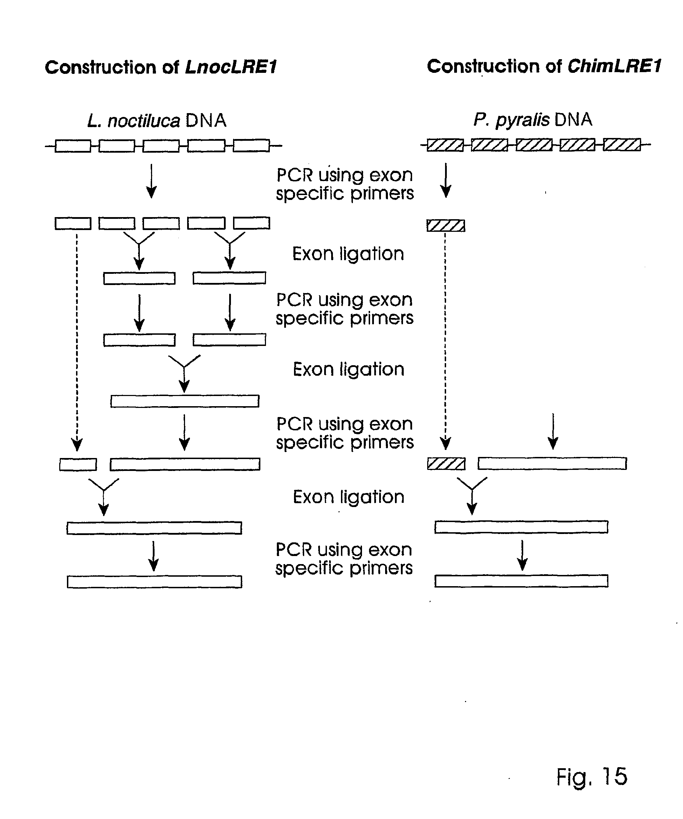

Figure 15 is a schematic showing luciferin regenerating enzyme (LRE) cDNA construction using exon-ligation mediated PCR;

Figure 16 shows the DNA sequence (SEQ ID NO 45) and putative amino acid sequence of L. noctiluca LRE1 (SEQ ID NO 46);

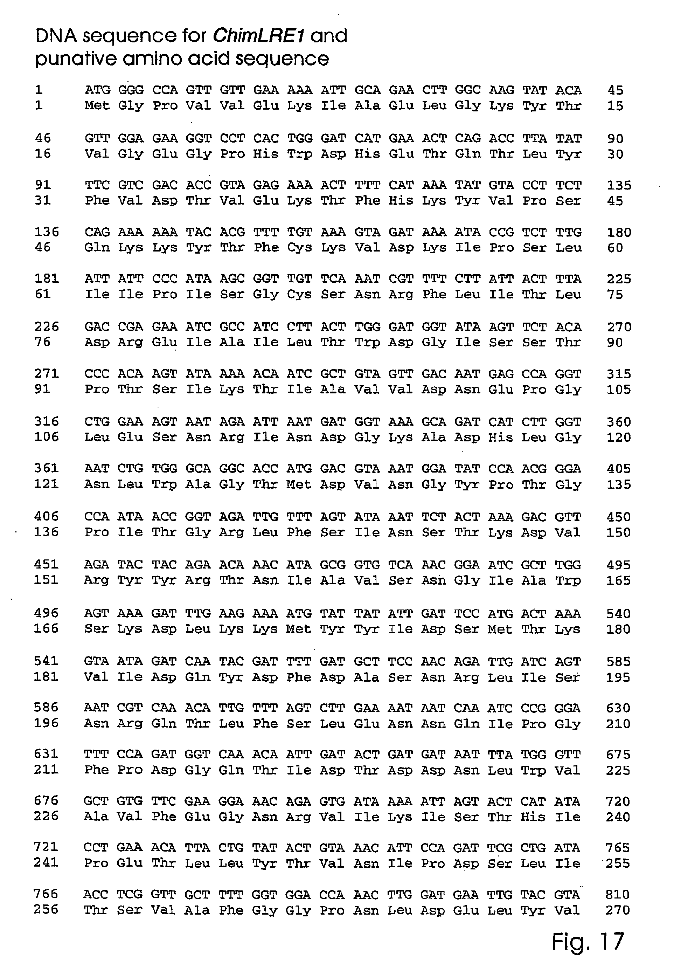

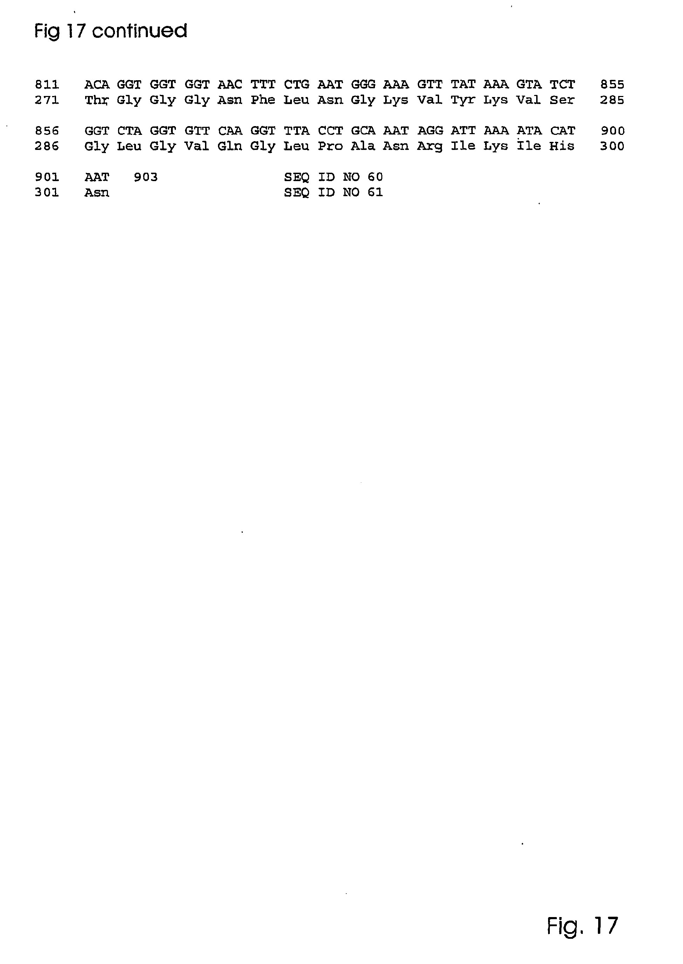

Figure 17 shows the DNA sequence (SEQ ID NO 47) and putative amino acid sequence of L. noctiluca LRE1 (SEQ ID NO 48);

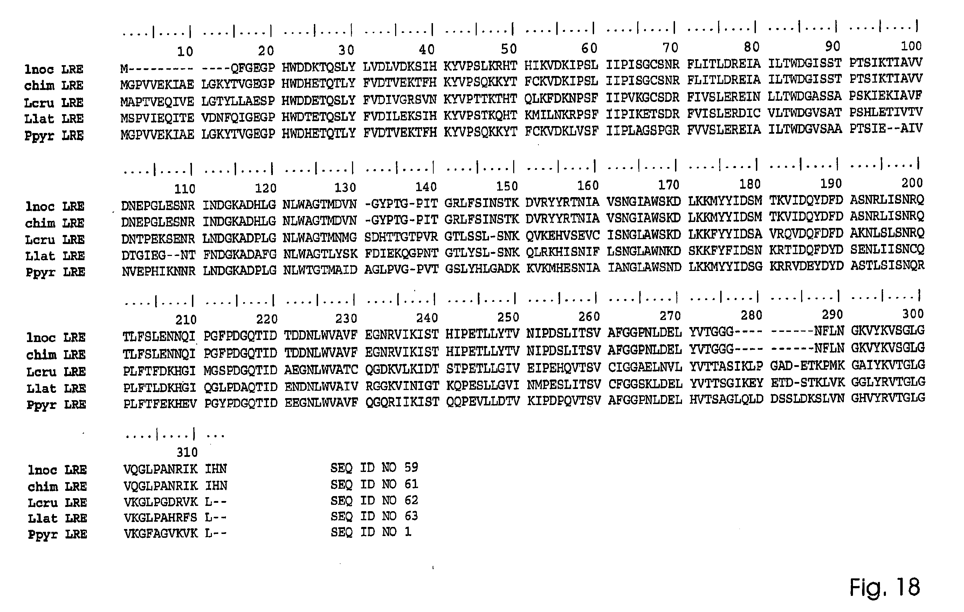

Figure 18 shows the amino acid sequence from *LnocLRE1* (lnoc LRE)(SEQ ID NO 59) and *ChimLREI* (chim LRE) (SEQ ID NO 61) aligned against three published LRE's; *Luciola cruciata* - Lcru LRE (BAB85479) (SEQ II NO 62), *Luciola lateralis* - Llat LRE (BAB85478) (SEQ ID NO 63) and Photinus pyralis - Ppyr LRE (BAB60700) (SEQ ID NO 1); and

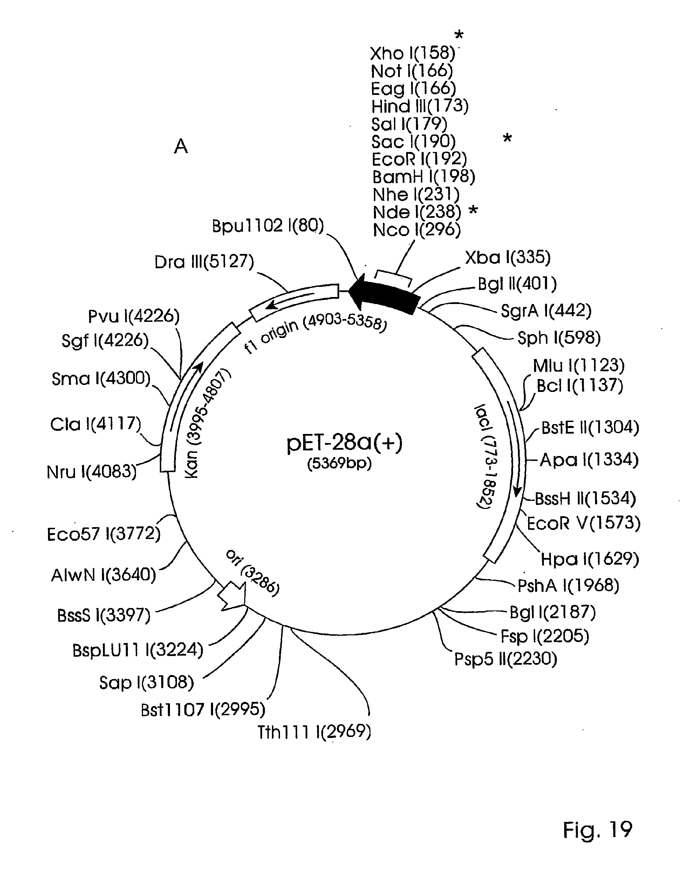

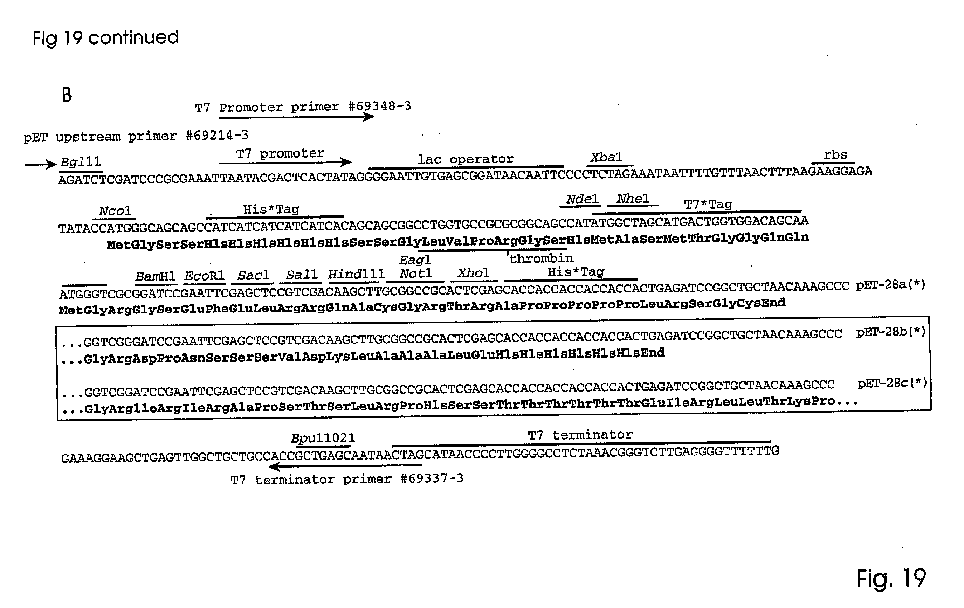

Figure 19 illustrates the pET-28a-c(+) cloning vector (Novagen) where A. is the plasmid map for pET-28a(+), and B. shows the sequence of the pET-28a-c(+) cloning/expression region, with insertion sites marked by *.

Example 1

Cloning and Sequencing of an LRE gene from *Lampyris noctiluca*

*Lampyris noctiluca,* the European glow-worm was collected from Southern England from 2000-2002. Total genomic DNA was extracting from female specimens using the High Pure PCR Template Preparation Kit (Roche). Genomic DNA was extracted in a similar manner from lyophilised *Photinus pyralis* specimens (Sigma).

The published *P*. *pyralis* LRE mRNA sequence (SEQ ID NO 2), obtained from GENEBANK, was used to design a range of generic primers in an attempt to amplify a homologous gene sequence from an extract of *L. noctiluca.* These attempts, however, were unsuccessful. Although amplification using a conventional polymerase chain reaction (PCR) yielded numerous products, these all appeared to be non-specific amplicons.

In order to overcome these difficulties, the applicants designed a set of five primers (SEQ ID NOS 5-9) to amplify the entire *P*. *pyralis* LRE gene, as illustrated in Figure 3 hereinafter. The positioning of these primers on SEQ ID NO 2 is also shown in this Figure.

As a result of amplification using these primers, additional sequence information was generated. By combining the initial sequence with these sequences, a complete sequence contig of the *P. pyralis* LRE gene was generated (SEQ ID NO 11) and is shown in Figure 4. In this Figure, the complete gene sequence is aligned against the mRNA sequence (SEQ ID NO 2). The exons were characterised and are shown shaded.

Generic primers were designed to amplify a small region of a *L. noctiluca* homologue of the *P. pyralis* LRE, based upon conserved amino acid sequences. These consisted of 6 forward primers (SEQ ID NOS 12-18) and 5 reverse primers (SEQ ID NOS 19-23). The sequence of the primers and their position with respect to the amino acid sequence (SEQ ID NO 1) is illustrated in Figure 5.

Using the LRE generic primers FOR5 (SEQ ID NO 17) and LRE generic REV 3 (SEQ ID NO 22), a 350 base pair PCR product was amplified from *L. noctiluca.* This was sequenced (SEQ ID NO 24) and shown to have homology with sequences in the *P. pyralis* LRE mRNA and genomic sequence (SEQ ID NOs 25 and 26 respectively) as illustrated in Figure 6. This gave assurance that there was indeed a homologue LRE in *L. noctiluca*.

With this information, 4 primers (SEQ ID NOs 27-30) were designed with a view to carrying out 5' and 3' genome walking along the *L. noctiluca* LRE gene. The sequence of these primers and the location on SEQ ID-NOs 24-26 is illustrated in Figure 7.

As a result of the 5' and 3' genome walking exercise with these primers, six clones were obtained from both the 5' and 3' ends and these were sequenced both forward and reverse. The resulting contig included the complete sequence downstream of the original 350bp sequence. The sequence at the 5'end has no homology to any regions of exon/intron I in *P. pyralis*. The contig, illustrated in Figure 8, of all the walking sequences produced more than two alleles in the alignment, suggesting that there might be more than one locus for this gene in *L. noctiluca.*

This is further illustrated in Figure 9 which shows the 5'UTR genomic walking sequences from *L. noctiluca.* Three different allelic forms of the gene are shown in one individual (SEQ ID NOS 10, 64 and 65), suggesting the presence of two or more copies of the gene. In this Figure, exon II begins at the arginine and position 855 in the illustrated sequence.

The 3'UTR genomic walking sequences (not shown) suggested that there were two different forms of the gene in one individual.

A sequence of a major part of the *L. noctiluca* LRE protein and the coding sequence as derived from this exercise are illustrated in Figure 10 as SEQ ID NO 3 and SEQ ID NO 4 respectively.

Some allelic variants from four different individuals are shown in Figure 11 as SEQ ID NOS 31-37.

It was surprisingly found that intron I of *L. noctiluca* was considerably smaller than intron I from *P. pyralis.* A complete read out beyond intron I and exon I in *L. noctiluca* was obtained. The complete gene sequence for the luciferin recycling protein from *Lampyris noctiluca,* the putative mRNA and protein sequence are shown in Figure 12 as SEQ ID NOS 40, 41 and 39 respectively.

Example 2

Preparation of Luciferin Recycling Proteins

Based upon the LRE gene sequence from *Lampyris noctiluca* (Fig 14) five sets of primers, NOC LRE EX1-5 were designed to amplify each exon individually (Fig 15, Table 1).

**Table** 1 List of-oligonucleotides and sequences. Underlined bases denote restriction enzyme sites.

| Oligonucleotide | Sequence 5'-3' | SEQ ID NO |
|---|---|---|
| NOC LRE EX1 for | ATGCAGTTTGGAGAAGGACCTCATTGGG | 42 |
| NOC LRE EX1 rev | CTACTTTTATATGGGTATGTCTTTTAAG | 43 |
| NOC LRE EX2 for | ATAAAATACCGTCTTTGATTATT | 44 |
| NOC LRE EX2 rev | CTGCCCACAGATTACCAAGATGATCTG | 45 |
| NOC LRE EX3 for | GCACCATGGACGTAAATG | 46 |
| NOC LRE EX3 rev | TGATCAATCTGTTGGAAGCATCA | 47 |
| NOC LRE EX4 for | GTAATCGTCAAACATTGTTTAGTC | 48 |
| NOC LRE EX4 rev | CAGCGAATCTGGAAKGTTTACAG | 49 |
| NOC LRE EX5 for | ATAACCTCGGTTGCTTTTGGTGACC | 50 |
| NOC LRE EX5 rev (1097) | ATTATRTATTTTWATCCTATTTGCAG | 51 |

(continued)

| Oligonucleotide | Sequence 5'-3' | SEQ ID NO |
|---|---|---|
| PYR LRE EX1 for | ATGGGGCCAGTTGTTGAAAAAATTGCAG | 52 |
| PYR LRE EX1 rev | TCATAGCTTCACTTTAACTCCCGC | 53 |
| NOC LRE Nco I START | GAGCTCCCATGGCCAGTTTGGAGAAGGACCTCATTGGG | 54 |
| NOC LRE Xho I END1 | GAGCTCTCGAGATTATRTATTTTWATCCTATTTGCAG | 55 |
| NOC LRE Xho I END2 | GAGCTCTCGAGTTAATTATRTATTTTWATCCTATTTGCAG | 56 |
| PYR LRE Nco I START | GAGCTCCCATGGGGCCAGTTGTTGAAAAAATTGC | 57 |

PCR was carried out using the proof reading PFU polymerase (Promega) in order to minimise the chance of a single deoxyadenosine residue being added to the 3' ends of the amplified fragments.

PCR products were separated on a 2% agarose gel, excised from the gel and extracted using agarose clean up columns (AB Gene). In the first ligation round, 2 exons pairs were ligated together. Ligation reactions were carried out at 16°C overnight. 0.2 µl of the ligation reaction was used in the second round of PCR. For each ligated pair of exons one forward and one reverse primer were used to amplify a contiguous product (Fig 15). Electrophoresis, gel excision and clean up was carried out as described above. In the second round of ligation, the two pairs of exon PCR products were ligated together. PCR amplification generated contiguous products that were subsequently used in a final ligation with the remaining exon.

The final PCR product containing all five exons ligated sequentially was cloned using the pGEM®-T Easy Vector System (Promega) and sequenced using a dye termination kit (Beckman) to confirm a continuous open reading frame and thus generating a complete transcript - *LnocLRE1* (Fig. 16). Due to the apparent differences in exon 1 of the LRE between *P. pyralis* and *L. noctiluca,* it was decided to amplify exon 1 from *P pyralis*, using *P. pyralis* specific primers (Table 1), and to splice this onto the exons 2-5 of *L. noctiluca* to produce a chimaeric LRE - *ChimLRE1* (Fig. 17).

*Sequence characteristics*

Two transcripts generated from exon ligation mediated PCR, *LnocLRE1* and *ChimLRE1* are 864 bp and 903 bp in length respectively. Both sequences (SEQ ID NOS 58 and 60 respectively) are shown, with putative amino acid sequences (SEQ ID NOS 59 and 61 respectively) in Figures 16 and 17. These amino acid sequences are shown aligned with other LRE's previously reported (Fig 18) and amino acid percentage identities are shown in table 2.

**Table 2** Percentage Amino acid indentity between *LnocLRE1* and *ChimLREI* against three published LRE's; *Luciola cruciata* - Lcru LRE (BAB85479), *Luciola laterlais* - Llat LRE (BAB85478) and *Photinus pyralis* - Ppyr LRE (BAB 60700).

| | *ChimLREI* | Lcru LRE | Llat LRE | Ppyr LRE |
|---|---|---|---|---|
| *LnocLRE1* | 90.6 | 51.5 | 50.6 | 54.1 |
| *ChimLREI* | | 53.2 | 51.6 | 63.1 |
| Lcru LRE | | | 57.2 | 56.2 |
| Llat LRE | | | | 50.8 |

*Transformation into stable and expression strains of* E. coli Both LRE sequences can be transferred to a suitable expression vector by using restriction enzyme site containing primers for PCR amplification to enable correct orientation and expression in the chosen vector. Primers were designed with a *Nco* I and a *Xho* I site (Table 1). Restriction enzyme digestion of PCR products will result in the conversion of sticky end cloning sites, a *Nco* I site at the 5' end and an *Xho* I site at the 3' end.

Particular vectors which may be used for expression of LRE sequences are pET-28a (Fig. 19) and 16b cloning vectors (Novagen).. The former will produce proteins with a C-terminal His tag. The latter provides for native protein

production (see cloning strategy above).

Target genes cloned in pET plasmids are under the control of a strong T7 promoter and require expression in a host containing the T7 RNA polymerase gene. This is provided by the DE3 lysogen of the BL21 (DE3) host strains. However, as pET recombinants can be unstable in the expression strains (DE3), plasmids are maintained in a stable *E. coli* strain (XL1-Blue). Recombinant plasmids can then be transformed into the expression strain BL21(DE3)pLysS prior to induction. The expression strain pLysS provides for high-stringency expression.

2μl of each ligation reaction is added to 100μl of competent cells (XL1-Blue) and subjected to 45second heat shock at 42˚C. Following 2 minutes incubation on ice 0.9ml of SOC is added and the sample incubated, with shaking at 37˚C, for 1hr. Recombinants are selected on LB plates containing 50μg/ml ampicillin (for pET) and 34μ/ml chloramphenicol (for pLysS).

Recombinants may be sequenced prior to transformation of 25ng into the expression strain BL21(DE3)pLysS.

*Over-expression*

An overnight culture (3ml) of each construct (in LB containing ampicillin, 50μg/ml, and chloramphenicol, 34μg/ml, is used to inoculate a 1L culture. Prior to induction cultures are split to provide a control (uninduced sample) and a test (induced sample). All test samples are induced at an optical density ($A_{600}$) of between 0.45-0.6 with 1mM isopropyl-β-D-thiogalactoside (IPTG), for 3-5 hours.
To check for protein expression 1ml samples of both uninduced and induced cultures were pelleted.

To each pellet, 100-200μl of 1X SDS solubilising buffer (SB) is added and the samples boiled for 5 minutes prior to sodium dodecyl sulfate-polyacrylamide gel analysis (SDS-PAGE, Laemmli, U. K. (1970). Cleavage of structural proteins during the assembly of the head of bacteriophage T4. Nature 227(259): 680-85).

To liquid fractions of any protein prep an equal volume of 2X SB is added prior to denaturing and SDS-PAGE.

Solubilising buffer:

| | 1X | 2X |
|---|---|---|
| 10% SDS | 100 | 200 |
| 1M Tris | 50 | 100 |
| 2-mercaptoethanol | 50 | 100 |
| PMSF | 20 | 40 |
| EDTA | 10 | 20 |
| Glycerol | 100 | 200 |
| $DH_2O$ | 670 | 340 |
| | 1000μl | 1000μl |
| Add about 50μl 10mg/ml bromophenol blue. | | |

SDS-PAGE gels are prepared using a 10% resolving gel and a 4% stacking gel. They are run for approximately 1 hour at 25-30mA/gel in Tris/glycine/SDS buffer [0.025M Tris, 0.192M glycine, 0.1% SDS (ICN)]. Proteins are visualised by incubating, with shaking, in coomassie blue (1hr) and destained until protein bands are seen clearly using destain (see below).

| | |
|---|---|
| 10% Resolving gel (makes 4-5): $dH_2O$ | 16.2ml |
| 1.5M Tris; 0.384% SDS (Protogel buffer from National Diagnostics) | 10ml |
| 10% SDS | 450μl |
| Ultra Pure Protogel (30% w/v acrylamide from National Diagnostics) | 13.3ml |
| 10% APS (made fresh each time) | 400μl |
| Temed | 40μl |

<u>4% stacking gel (makes 4-5):</u>

| | |
|---|---|
| $dH_2O$ | 12.2ml |
| 0.5M Tris; 0.4% SDS (Protogel buffer from National Diagnostics) | 5ml |
| 10% SDS | 200μl |

(continued)

| | |
|---|---|
| Ultra Pure Protogel (30% w/v acrylamide from National Diagnostics) | 2.7ml |
| 10% APS (make fresh each time) Temed | 200μl 40μl |

Coomassie Brilliant Blue

| | |
|---|---|
| Coomassie brilliant blue R-250 | 1g |
| Methanol | 450ml |
| Water | 450ml |
| Acetic acid | 100ml |

Destain

| | |
|---|---|
| Isopropanol | 125ml |
| Acetic acid | 50ml |
| Glycerol | 15ml |
| Water | 310ml |

*Protein purification*

Induced cultures (500ml) are collected and resuspended in 20ml buffer. Soluble crude extracts (clarified samples) are prepared by sonication (10 cycles of 25 sec on, 20 sec off) and collection of supernatants following centrifugation (14000 rpm, 20 min). His- tagged proteins are purified using the TALON affinity resin according to the manufacturer's instructions (BD biosciences). TELON resin utilises immobilised cobalt 2+ and provides enhanced selectivity for polyhistidine-tagged proteins.

Purified proteins can be visualised using SDS-PAGE and quantified according to the Bradford assay (Bradford, M. M. (1976) A rapid and sensitive method for the quantification of microgram quantities of protein utilizing the principle of protein-dye binding. Anal. Biochem. 72: 248-54).

Example 3

In-vivo Assay using Luciferase Recycling Enzyme

Whole cells are transformed using conventional methods, so that they express an LRE and firefly luciferase activities. These cells are incubated in a low pH buffer (max pH 5.0) containing 0.6mM D-luciferin, for 0.5 to 10min (depending on cell type).

The cells are then removed from the low pH buffer, washed, and resuspended in a neutral buffer. Alternatively, the low pH buffer is neutralised using a suitable alkali. Bioluminescence emitted from the cells can then be detected and measured using a luminometer or other light-detecting instrument. This provides an in vivo assay for measuring ATP intracellularly.

Example 4

In-vitro Assay using Luciferase Recycling Enzyme

A sample containing ATP to be assayed will be added to a reaction mixture containing 25mM Tricine-NaOH pH 7.8, 4.0 mM MgSO4, 0.1-10μg firefly luciferase and 0.1-20μg purified recombinant LRE according to the invention, or 0.1-40μg purified recombinant luciferase-LRE fusion protein also according to the invention, 0.5-5mM D-cysteine and 250μM D-luciferin. Light output from the reaction will be detected and measured using a luminometer or other light detecting instuments, as a measure of ATP content of the sample.

Although the invention has been described in connection with the specific preferred embodiments, it should be understood that the invention as claimed should not be unduly limited to such specific embodiments.

## Claims

1. A method for measuring intracellular ATP and/or gene expression, which method comprises transforming a cell with a construct which encodes a luciferase and a luciferin recycling protein, introducing luciferin into said cell, and monitoring bioluminescent signals from said cell.

2. The method according to claim 1 which is used to monitor intracellular ATP levels.

3. The method according to claim 1 which is used to monitor luciferase expression.

4. The method according to any one of the preceding claims, wherein the cell is contacted with luciferin and the pH reduced to less than 5 prior to the start of the assay, and thereafter, the cell is restored to physiological pH conditions.

5. The method according to any one of the preceding claims, wherein the cell is further transformed such that it expresses an L-cysteine racemase.

6. The method according to any one of the preceding claims, wherein the cell is capable of expressing a further luciferase which has a different $K_m$ value and a different wavelength output than said first luciferase.

7. A DNA construct comprising (i) a nucleic acid sequence which encodes a luciferase enzyme, and (ii) a nucleic acid sequence which encodes a luciferin recycling protein.

8. The DNA construct according to claim 7, wherein the nucleic acid sequences of (i) and (ii) are linked so as to express a fusion protein comprising a luciferase and a luciferin recycling protein.

9. The DNA construct according to claim 7 or claim 8 which further comprises one or more additional components selected from (iii) a nucleic acid sequence which encodes a further luciferase enzyme; and (iv) a nucleic acid sequence which encodes an L-cysteine racemase enzyme.

10. The DNA construct according to any one of claims 7 to 9 which comprises a single vector.

11. The DNA construct according to any one of claims 7 to 9 which comprises more than one vector which may be used in cotransformation of a cell.

12. A cell transformed with a DNA construct according to any one of claims 7 to 11.

13. The cell according to claim 12 which is a mammalian or plant cell.

14. The method according to any one of claims 1 to 6, the DNA construct according to any one of claims 7 to 11 or the cell according to any one of claims 12 to 13, wherein the luciferin recycling protein is a protein of SEQ ID NOS 1, 62 or 63 or a luciferin recycling fragment thereof.

15. The method according to any one of claims 1 to 6, the DNA construct according to any one of claims 7 to 11 or the cell according to any one of claims 12 to 13, wherein the luciferin recycling protein is a luciferin recycling protein comprising SEQ ID NO 3, SEQ ID NO 39, SEQ ID NO 59, or SEQ ID NO 61, or a luciferin recycling fragment thereof.

16. The method, DNA construct or cell according to claim 15, wherein the luciferin recycling protein is a luciferin recycling protein comprising SEQ ID NO 59, or a luciferin recycling fragment thereof.

17. A fusion protein comprising a luciferase enzyme and a luciferin recycling protein.

18. The fusion protein according to claim 17, wherein the luciferin recycling protein is obtainable from a glow-worm species, or a luciferin recycling fragment thereof, or a protein having at least 60% homology thereto.

19. The fusion protein according to claim 18, wherein the glow-worm species is *Lampyris noctiluca*.

20. The fusion protein according to claim 18 or claim 19, wherein the luciferin recycling protein comprises SEQ ID NO 3, SEQ ID NO 39, or SEQ ID NO 59 or a luciferin recycling fragment thereof.

**21.** The fusion protein according to any one of claims 18 to 20, wherein the luciferin recycling protein comprises SEQ ID NO 3 or SEQ ID NO 39 or a luciferin recycling fragment thereof.

**22.** The fusion protein according to any one of claims 18 to 21, wherein the luciferin protein comprises SEQ ID NO 59 or a luciferin recycling fragment thereof.

**23.** The fusion protein according to claim 22, wherein the luciferin recycling protein comprises SEQ ID NO 59.

**24.** The fusion protein according to claim 17, wherein the luciferase recycling protein is a chimeric luciferase recycling protein comprising fragments encoded by exons found in luciferase recycling protein genes of different species.

**25.** The fusion protein according to claim 24, wherein at least one of the exons is from a gene of a glowworm species.

**26.** The fusion protein according to claim 24 or claim 25, wherein at least one of the exons is from a gene of a firefly species.

**27.** The fusion protein according to any one of claims 24 to 26, wherein the chimeric luciferase recycling protein comprises a fragment encoded by exon 1 of *Photinus pyralis* luciferase recycling gene, linked to a fragment encoded by exons 2, 3, 4 and 5 of a *Lampyris noctiluca* luciferase recycling gene.

**28.** A nucleic acid which encodes a protein according to any one of claims 17 to 27.

**29.** The nucleic acid according to claim 28 which comprises SEQ ID NO 4 or any one of SEQ ID NOS 31 to 37.

**30.** The nucleic acid according to claim 28 which comprises SEQ ID NO 40.

**31.** The nucleic acid according to claim 28 which comprises SEQ ID NO 58.

**32.** The nucleic acid according to claim 28 which is a cDNA sequence.

**Patentansprüche**

**1.** Verfahren zur Messung der intrazellulären ATP- und/oder Genexpression, wobei das Verfahren das Transformieren einer Zelle mit einem Konstrukt, das für eine Luciferase und ein Luciferin-Recyclingpnatein kodiert, das Einführen von Luciferin in die Zelle und das Überwachen von Bioiumineszenzsignaien aus der Zelle umfasst.

**2.** Verfahren nach Anspruch 1, wobei das Verfahren zur Überwachung von intrazelluläre ATP-Konzentrationen verwendet wird.

**3.** Verfahren nach Anspruch 1, wobei das Verfahren zur Überwachung der Luciferase-Expression verwendet wird.

**4.** Verfahren nach einem der vorstehenden Ansprüche, wobei die Zelle vor Testbeginn mit Luciferin in Kontakt gebraucht wird und der pH-Wert auf weniger als 5 verringert wird und anschließend die Zelle wieder auf physiologische pH-Bedingungen gebraucht wird.

**5.** Verfahren nach einem der vorstehende Ansprüche, wobei die Zelle ferner so transformiert wird, dass sie eine L-Cystein-racemase exprimiert.

**6.** Nach einem der vorstehenden Ansprüche, wobei die Zelle zur Expression einer weiteren Luciferase befähigt ist, die einen im Vergleich zur ersten Luciferase unterschiedlichen $K_m$-Wert und eine unterschiedliche Ausgabewellenlänge aufweist.

**7.** DNA-Konstrukt, umfassend (i) eine Nucleinsäuresequenz, die für ein Luciferase-Enzym kodiert, und (ii) eine Nucleinsäuresequenz, die für ein Luciferin-Recyclingprotein kodiert.

**8.** DNA-Konstrukt nach Anspruch 7, wobei die Nucieinsäuresequenzen von (i) und (ii) so Verknüpft sind, dass sie ein Fusionsprotein exprimieren, das eine Luciferase und ein Luciferin-Recyclingprotein umfasst.

**9.** DNA-Konstrukt nach Anspruch 7 oder 8, ferner umfassend eine oder mehrere weitere Komponenten, die aus (iii) einer Nucleinsäuresequenz, die für ein weiteres Luciferase-Enzym kodiert; und (iv) einer Nucleinsäuresequenz, die für ein L-Cystein-racemase-Enzym kodiert, ausgewählt sind.

**10.** DNA-Konstrukt nach einem der Ansprüche 7 bis 9, umfassend einen einzigen Vektor.

**11.** nach einem der Ansprüche 7 bis 9, umfassend mehr als einen Vektor, die bei der Cotransformation einer Zelle verwendet werden können.

**12.** Zelle, die mit einem DNA-Konstrukt nach einem der Ansprüche 7 bis 11 transformiert ist.

**13.** Zelle nach Anspruch 12, bei der es sich um eine Säugetier- oder Pflanzenzelle' handelt.

**14.** Verfahren nach einem der Ansprüche 1 bis 6, DNA-Konstrukt nach einem der Anspräche 7 bis 11 oder Zelle nach einem der Ansprüche 12 bis 13, wobei es sich beim Luciferin-Recyclingprotein um ein Protein gemäß SEQ ID NOS: 1, 62 oder 63 order um ein Luciferin-Recyclingfragment davon handelt.

**15.** Verfahren nach einem der Ansprüche 1 bis 6, DNA-Konstrukt nach einem der Ansprüche 7 bis 11 oder Zelle nach einem der Ansprüche 12 bis 13, wobei es sich beim Luciferin-Recyclingprotein um ein Luciferin-Recyclingprotein handelt, das aus SEQ ID NO: 3, SEQ ID NO: 39, SEQ ID NO: 59 oder SEQ ID NO: 61 oder einem Luciferin-Recyclingfragment davon ausgewählt ist.

**16.** Verfahren, DNA-Konstrukt oder Zelle noch Anspruch 15, wobei es sich beim Luciferin-Recyclingprotein um ein Luciferin-Recyclingprotein handelt, das SEQ ID NO: 59 oder ein Luciferin-Recyclingfragment davon umfasst.

**17.** Fusionsprotein, umfassend ein Luciferase-Enzym und ein Luciferin-Recyclingprotein.

**18.** Fusionsprotein nach Anspruch 17, wobei das Luciferin-Recyclingprotein von einer Glühwürmchen-Spezies oder einem Luciferin-Recyclingfragment davon oder einem Protein mit einer mindestens 60%igen Homologie hierzu erhältich ist.

**19.** Fusionsprotein nach Anspruch 18, wobei es sich bei der Glühwürmchen-Spezies um *Lampyris noctiluca* handelt.

**20.** Fusionsprotein nach Anspruch 18 oder 19, wobei das Luciferin-Recyclingprotein SEQ ID NO: 3, SEQ ID NO: 39 oder SEQ ID NO: 59 oder ein Luciferin-Recyclingfragment davon umfasst.

**21.** Fusionsprotein nach einem der Ansprüche 18 bis 20, wobei das Luciferin-Recyclingprotein SEQ ID NO: 3 oder SEQ ID NO: 39 oder ein Luciferin-Recyclingfragment davon umfasst.

**22.** Fusionsprotein nach einem der Ansprüche 18 bis 21, wobei das Luciferin-Recyclingprotein SEQ ID NO: 59 oder ein Luciferin-Recyclingfragment davon umfasst.

**23.** Fusionsprotein nach Anspruch 22, wobei das Luciferin-Recyclingprotein SEQ ID NO: 59 umfasst.

**24.** Fusionsprotein nach Anspruch 17, wobei es sich beim Luciferin-Recyclingprotein um ein chimäres Luciferase-Recyclingprotein handelt, das Fragmente umfasst, die durch Exons kodiert werden, die in Luciferase-Recyclingprotein-Genen verschiedener Spezies auftreten.

**25.** Fusionsprotein nach Anspruch 24, wobei mindestens eines der Exons von einem Gen einer Glühwürmchen-Spezies stammt.

**26.** Fusionsprotein nach Anspruch 24 oder 25, wobei mindestens eines der Exons von einem Gen einer Leuchtkäfer-Spezies stammt.

**27.** Fusionsprotein nach einem der Ansprüche 24 bis 26, wobei das chimäre Luciferase-Recyclingprotein ein Fragment umfasst, das durch ein Exon 1 des *Photinus pyralis*-Luciferase-Recyclinggens kodiert wird, verknüpft mit einem Fragment, das durch die Exons 2,'3, 4 und 5 eines *Lampyris noctiluca*-Luciferase-Recyclinggens kodiert wird.

**28.** Nucleinsäure, die für ein Protein nach einem der Ansprüche 17 bis 27 kodiert.

**29.** Nucleinsäure nach Anspruch 28, die SEQ ID NO: 4 oder eine der Sequenzen SEQ ID NOS: 31 bis 37 umfasst.

**30.** Nucleinsäure nach Anspruch 28, die SEQ ID NO: 40 umfasst.

**31.** Nucleinsäure nach Anspruch 28, die SEQ ID NO: 58 umfasst.

**32.** Nucleinsäure nach Anspruch 28, bei der es sich um eine cDNA-Sequenz handelt

**Revendications**

**1.** Méthode de mesure de l'ATP intracellulaire et/ou l'expression génétique, laquelle méthode comprend la transformation d'une cellule avec un constituant qui code une protéine recyclant la luciférase et une protéine recyclant la luciférine, l'introduction de la luciférine dans ladite cellule, et la surveillance des signaux bioluminescents provenant de ladite cellule.

**2.** Méthode selon la revendication 1 qui est utilisée pour surveiller les niveaux de l'ATP intracellulaire.

**3.** Méthode selon la revendication 1 qui est utilisée pour surveiller l'expression de la luciférase.

**4.** Méthode selon l'une quelconque des revendications précédentes, dans laquelle la cellule est mise en contact avec la luciférine et le pH est réduit à moins de 5 avant le début de l'essai, et ensuite la cellule est restaurée dans les conditions du pH physiologique.

**5.** Méthode selon l'une quelconque des revendications précédentes, dans laquelle la cellule est en outre transformée de telle sorte qu'elle exprime une racémase de L-cystéine.

**6.** Méthode selon l'une quelconque des revendications précédentes, dans laquelle la cellule est capable d'exprimer une autre luciférase qui a une valeur $K_m$ différente et une sortie à une longueur d'onde différente de ladite première luciférase.

**7.** Constituant d'ADN comprenant (i) une séquence d'acide nucléique qui code une enzyme luciférase et (ii) une séquence d'acide nucléique qui code une protéine recyclant la luciférine.

**8.** Constituant d'ADN selon la revendication 7, dans lequel les séquences d'acide nucléique de (i) et (ii) sont liées de façon à exprimer une protéine de fusion comprenant une protéine recyclant la luciférase et une protéine recyclant la luciférine.

**9.** Constituant d'ADN selon la revendication 7 ou la revendication 8 qui comprend en outre un ou plusieurs composants supplémentaires choisis parmi (iii) une séquence d'acide nucléique qui code une autre enzyme luciférase ; et (iv) une séquence d'acide nucléique qui code une enzyme racémase de L-cystéine.

**10.** Constituant d'ADN selon l'une quelconque des revendications 7 à 9 qui comprend un seul vecteur.

**11.** Constituant d'ADN selon l'une quelconque des revendications 7 à 9 qui comprend plus d'un vecteur qui peut être utilisé dans la cotransformation d'une cellule.

**12.** Cellule transformée avec un constituant d'ADN selon l'une quelconque des revendications 7 à 11.

**13.** Cellule selon la revendication 12 qui est une cellule d'un mammifère ou d'un végétal.

**14.** Méthode selon l'une quelconque des revendications 1 à 6, constituant d'ADN selon l'une quelconque des revendications 7 à 11 ou cellule selon l'une quelconque des revendications 12 à 13, dans lesquels la protéine recyclant la luciférine est une protéine ayant les SEQ ID N° 1, 62 ou 63 ou un fragment recyclant la luciférine de celle-ci.

**15.** Méthode selon l'une quelconque des revendications 1 à 6, constituant d'ADN selon l'une quelconque des revendications 7 à 11 ou cellule selon l'une quelconque des revendications 12 à 13, dans lesquels la protéine recyclant la luciférine est une protéine recyclant la luciférine comprenant SEQ ID N° 3, SEQ ID N° 39, SEQ ID N° 59 ou SEQ ID N° 61 ou un fragment recyclant la luciférine de celle-ci.

**16.** Méthode, constituant d'ADN ou cellule selon la revendication 15 dans lesquels la protéine recyclant la luciférine est une protéine recyclant la luciférine comprenant SEQ ID N° 59, ou un fragment recyclant la luciférine de celle-ci.

**17.** Protéine de fusion comprenant une protéine recyclant l'enzyme luciférase et une protéine recyclant la luciférine.

**18.** Protéine de fusion selon la revendication 17, dans laquelle la protéine recyclant la luciférine peut être obtenue à partir d'une espèce de ver luisant, ou un fragment recyclant la luciférine de celle-ci, ou une protéine ayant au moins une homologie de 60 % par rapport à celle-ci.

**19.** Protéine de fusion selon la revendication 18, dans laquelle l'espèce de ver luisant est le *Lampyris noctiluca.*

**20.** Protéine de fusion selon la revendication 18 ou la revendication 19, dans laquelle la protéine recyclant la luciférine comprend SEQ ID N° 3, SEQ ID N° 39 ou SEQ ID N° 59 ou un fragment recyclant la luciférine de celle-ci.

**21.** Protéine de fusion selon l'une quelconque des revendications 18 à 20, dans laquelle la protéine recyclant la luciférine comprend SEQ ID N° 3 ou SEQ ID N° 39 ou un fragment recyclant la luciférine de celle-ci.

**22.** Protéine de fusion selon l'une quelconque des revendications 18 à 21, dans laquelle la protéine recyclant la luciférine comprend SEQ ID N° 59 ou un fragment recyclant la luciférine de celle-ci.

**23.** Protéine de fusion selon la revendication 22, dans laquelle la protéine recyclant la luciférine comprend SEQ ID N° 59.

**24.** Protéine de fusion selon la revendication 17, dans laquelle la protéine recyclant la luciférase est une protéine recyclant la luciférase chimère comprenant des fragments codés par des exons découverts dans les gènes de la protéine recyclant la luciférase de différentes espèces.

**25.** Protéine de fusion selon la revendication 24, dans laquelle au moins l'un des exons provient d'un gène d'une espèce de ver luisant.

**26.** Protéine de fusion selon la revendication 24 ou la revendication 25, dans laquelle au moins l'un des exons provient d'un gène d'une espèce de luciole.

**27.** Protéine de fusion selon l'une quelconque des revendications 24 à 26, dans laquelle la protéine recyclant la luciférase chimère comprend un fragment codé par l'exon 1 du gène recyclant la luciférase de *Photinus pyralis,* lié à un fragment codé par les exons 2, 3, 4 et 5 d'un gène recyclant la luciférase de *Lampyris noctiluca.*

**28.** Acide nucléique qui code une protéine selon l'une quelconque des revendications 17 à 27.

**29.** Acide nucléique selon la revendication 28 qui comprend SEQ ID N° 4 ou l'une quelconque des SEQ ID N° 31 à 37.

**30.** Acide nucléique selon la revendication 28 qui comprend SEQ ID N° 40.

**31.** Acide nucléique selon la revendication 28 qui comprend SEQ ID N° 58.

**32.** Acide nucléique selon la revendication 28 qui est une séquence d'ADN-c.

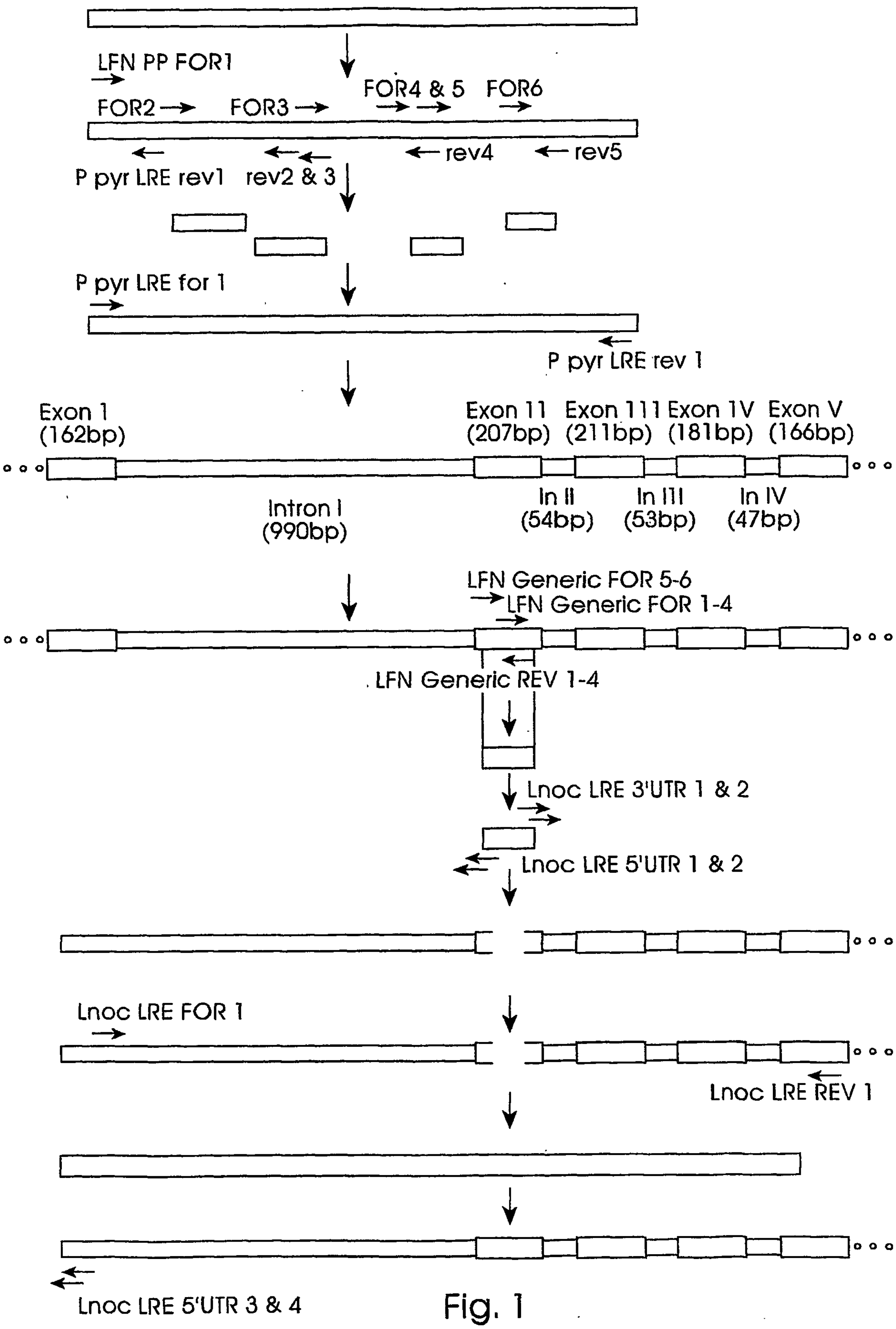
LFN PP FOR1
FOR2
FOR3
FOR4 & 5
FOR6
rev4
rev5
P pyr LRE rev1
rev2 & 3
P pyr LRE for 1
P pyr LRE rev 1
Exon 1 (162bp)
Exon 11 (207bp)
Exon 111 (211bp)
Exon 1V (181bp)
Exon V (166bp)
Intron I (990bp)
In II (54bp)
In I11 (53bp)
In IV (47bp)
LFN Generic FOR 5-6
LFN Generic FOR 1-4
LFN Generic REV 1-4
Lnoc LRE 3'UTR 1 & 2
Lnoc LRE 5'UTR 1 & 2
Lnoc LRE FOR 1
Lnoc LRE REV 1
Lnoc LRE 5'UTR 3 & 4

Fig. 1

Accession number AB062786 Species *Photinus pyralis* mRNA
for luciferin regenerating enzyme

ATGGGGCCAGTTGTTGAAAAAATTGCAGAACTTGGCAAGTATACGGTTGGAGAAGGTCCTCACTGGGATC
ATGAAACTCAGACCTTATATTTCGTCGACACCGTAGAGAAAACTTTTCATAAATATGTACCTTCTCAGAA
AAAATACACGTTTTGTAAAGTAGATAAACTGGTTTCTTTCATTATTCCCCTTGCTGGATCCCCTGGCCGT
TTTGTAGTCAGTTTGGAACGTGAAATAGCCATTCTTACATGGGATGGCGTTAGTGCTGCACCTACAAGCA
TAGAAGCTATTGTTAATGTCGAACCACACATTAAAAATAACAGACTCAATGATGGCAAAGCAGATCCCCT
TGGCAATCTATGGACAGGTACAATGGCTATTGACGCTGGTCTCCCCGTAGGACCGGTCACTGGCAGTTTA
TATCATTTAGGGGCTGATAAAAAGGTAAAAATGCACGAGAGCAACATAGCTATAGCAAATGGGCTCGCGT
GGAGTAATGATTTGAAGAAAATGTATTATATTGATTCGGGGAAAAGAAGAGTAGACGAGTACGATTATGA
TGCTTCTACATTATCCATCAGCAATCAACGGCCATTATTTACTTTTGAAAAGCATGAAGTGCCTGGATAT
CCAGATGGTCAAACAATTGATGAGGAGGGTAATTTATGGGTTGCCGTTTTCCAAGGACAGCGAATTATTA
AAATCAGTACCCAACAACCGGAAGTGTTACTGGATACCGTAAAAATACCAGATCCTCAGGTCACCTCTGT
AGCATTTGGCGGTCCGAATTTGGATGAACTGCATGTAACATCTGCTGGTCTTCAGCTTGACGACAGTTCT
TTGGACAAAAGTTTAGTTAATGGGCACGTCTACAGAGTAACAGGTTTAGGCGTCAAAGGTTTCGCGGGAG
TTAAAGTGAAGCTATGA (SEQ ID NO 2)

Accession number BAB60700 luciferin regenerating enzyme
*Photinus pyralis*

MGPVVEKIAELGKYTVGEGPHWDHETQTLYFVDTVEKTFHKYVPSQKKYTFCKVDKLVSFIIPLAGSPGR
FVVSLEREIAILTWDGVSAAPTSIEAIVNVEPHIKNNRLNDGKADPLGNLWTGTMAIDAGLPVGPVTGSL
YHLGADKKVKMHESNIAIANGLAWSNDLKKMYYIDSGKRRVDEYDYDASTLSISNQRPLFTFEKHEVPGY
PDGQTIDEEGNLWVAVFQGQRIIKISTQQPEVLLDTVKIPDPQVTSVAFGGPNLDELHVTSAGLQLDDSS
LDKSLVNGHVYRVTGLGVKGFAGVKVKL (SEQ ID NO 1)

Fig. 2

| | | | | | | SEQ ID NO |
|---|---|---|---|---|---|---|
| 1. | P.pyr LRE for1 | 5' | ATGGGGCCAGTTGTTGAAAAAATTG | 3' | | 5 |
| 2. | P.pyr LRE for2 | 5' | GCAATCTATGGACAGGTACAATGG | 3' | | 6 |
| 3. | P.pyr LRE for3 | 5' | GGGCTGATAAAAAGGTAAAAATGCA | 3' | | 7 |
| 4. | P.pyr LRE rev1 | 5' | TCATAGCTTCACTTTAACTCCCGCG | 3' | | 8 |
| 5. | P.pyr LRE rev2 | 5' | GCACTAACGCCATCCCA | 3' | | 9 |

**ATGGGGCCAGTTGTTGAAAAAATTG**CAGAACTTGGCAAGTATACGGTTGGAGAAGGTCCTCACTGGGATC LRE for1
ATGAAACTCAGACCTTATATTTCGTCGACACCGTAGAGAAAACTTTTCATAAATATGTACCTTCTCAGAA
AAAATACACGTTTTGTAAAGTAGATAAACTGGTTTCTTTCATTATTCCCCTTGCTGGATCCCCTGGCCGT
TTTGTAGTCAGTTTGGAACGTGAAATAGCCATTCTTACA**TGGGATGGCGTTAGTGC**TGCACCTACAAGCA LRE rev2
TAGAAGCTATTGTTAATGTCGAACCACACATTAAAAATAACAGACTCAATGATGGCAAAGCAGATCCCCT
TG**GCAATCTATGGACAGGTACAATGGC**TATTGACGCTGGTCTCCCCGTAGGACCGGTCACTGGCAGTTTA LRE for2
TATCATTTAG**GGGCTGATAAAAAGGTAAAAATGCA**CGAGAGCAACATAGCTATAGCAAATGGGCTCGCGT LRE for3
GGAGTAATGATTTGAAGAAAATGTATTATATTGATTCGGGGAAAAGAAGAGTAGACGAGTACGATTATGA
TGCTTCTACATTATCCATCAGCAATCAACGGCCATTATTTACTTTTGAAAAGCATGAAGTGCCTGGATAT
CCAGATGGTCAAACAATTGATGAGGAGGGTAATTTATGGGTTGCCGTTTTCCAAGGACAGCGAATTATTA
AAATCAGTACCCAACAACCGGAAGTGTTACTGGATACCGTAAAAATACCAGATCCTCAGGTCACCTCTGT
AGCATTTGGCGGTCCGAATTTGGATGAACTGCATGTAACATCTGCTGGTCTTCAGCTTGACGACAGTTCT
TTGGACAAAAGTTTAGTTAATGGGCACGTCTACAGAGTAACAGGTTTAGGCGTCAAAGGTTT**CGCGGGAG** LRE rev1
**TTAAAGTGAAGCTATGA**

Fig. 3

```
          ....:....| ....:....| ....:....| ....:....| ....:....| ....:....| ....:....| ....:....|
                 10         20         30         40         50         60         70         80
LRE gene  ATGGGGCCAG TTGTTGAAAA AATTGCAGAA CTTGGCAAGT ATACGAGTTG GAGAAGGTCC TCACTGGGAT CATGAAACTC
RNA       ATGGGGCCAG TTGTTGAAAA AATTGCAGAA CTTGGCAAGT ATACG-GTTG GAGAAGGTCC TCACTGGGAT CATGAAACTC

          ....:....| ....:....| ....:....| ....:....| ....:....| ....:....| ....:....| ....:....|
                 90        100        110        120        130        140        150        160
LRE gene  AGACCTTATA TTTCGTCGAC ACCGTAGAGA AAACTTTTCA TAAATATGTA CCTTCTCAGA AAAAATACAC GTTTTGTAAA
RNA       AGACCTTATA TTTCGTCGAC ACCGTAGAGA AAACTTTTCA TAAATATGTA CCTTCTCAGA AAAAATACAC GTTTTGTAAA

          ....:....| ....:....| ....:....| ....:....| ....:....| ....:....| ....:....| ....:....|
                170        180        190        200        210        220        230        240
LRE gene  GTAGGTAAGA ATCATTGTTC AAACTATCAC GCGTAACCGT TTTTACATAA GTACAATGTA AAGTAGCTAC GTACACCGTG
RNA       GTA....... .......... .......... .......... .......... .......... .......... ..........

          ....:....| ....:....| ....:....| ....:....| ....:....| ....:....| ....:....| ....:....|
                250        260        270        280        290        300        310        320
LRE gene  GATAGTAGCA TTGTATTTAT AAACGAAATA CAATGGTAGC ATTGTATTTA TATAGTAGGC ACTTTATAGT TAGAAATAGG
RNA       .......... .......... .......... .......... .......... .......... .......... ..........

          ....:....| ....:....| ....:....| ....:....| ....:....| ....:....| ....:....| ....:....|
                330        340        350        360        370        380        390        400
LRE gene  GGAAGTATTA GCCAAGAGTA TACAGGGTGG TGAGTTGCGT AACACATTAT AGGGATTTCA ATGTAAAATT CATAACATGC
RNA       .......... .......... .......... .......... .......... .......... .......... ..........

          ....:....| ....:....| ....:....| ....:....| ....:....| ....:....| ....:....| ....:....|
                410        420        430        440        450        460        470        480
LRE gene  GATTATTGGC TCCCCAAATT ATTTTAGAGC AAAAATGCAT TAAGGTTTTT TGTAGAAAAA CATTTTTTAC ATCAATCAAC
RNA       .......... .......... .......... .......... .......... .......... .......... ..........

          ....:....| ....:....| ....:....| ....:....| ....:....| ....:....| ....:....| ....:....|
                490        500        510        520        530        540        550        560
LRE gene  CGTATTAGTT TTAAAATTTT AAGCAAGTCA CCACCCTGTA TATACTCTTT GGTATTAGTA CAAGATCCCA CATAAGGCCA
RNA       .......... .......... .......... .......... .......... .......... .......... ..........

          ....:....| ....:....| ....:....| ....:....| ....:....| ....:....| ....:....| ....:....|
                570        580        590        600        610        620        630        640
LRE gene  ACCTCGCTCA TCTGTTTACT GAGTGAAATA CATCTTTGTT AGTAATCTTT TATGTGTAAA CAAACACACT AAAA::AACT
RNA       .......... .......... .......... .......... .......... .......... .......... ..........
```

Fig. 4

Fig 4 continued

```
                650        660        670        680        690        700        710        720
LRE gene TAAGTCAAGA CTATCCTTAA AATTTCTGTC TGCTTCTATT AGATAGAGAG GAGACACACG GAACAACACA CGTTTCTCTC
RNA      .......... .......... .......... .......... .......... .......... .......... ..........

                730        740        750        760        770        780        790        800
LRE gene CCGCGCGTTT CTTAAAATTA CGACAACCGT GTGAAACAGG ACGTTACTCG TGACGGCGGT GTACGCATTA ATGTTAAGAG
RNA      .......... .......... .......... .......... .......... .......... .......... ..........

                810        820        830        840        850        860        870        880
LRE gene AGTGCGGCGC ACTGAATTTT AATTGAGGGT AGTCTTGACT TAATTTTTTT AGGCAACCAT TTGCAATGTG TTTGATTACA
RNA      .......... .......... .......... .......... .......... .......... .......... ..........

                890        900        910        920        930        940        950        960
LRE gene TATTAAAATT GCTAAAACAT GTAATTCACT TGGTAAACAG TAGTCGGTAG GGCGAATAAT GCTGCTATCT ATTTTCTCTT
RNA      .......... .......... .......... .......... .......... .......... .......... ..........

                970        980        990       1000       1010       1020       1030       1040
LRE gene CTATAGCTTA TTGAGCTGAG TATGAAGGGT AGAATAGCAT TTAGATTAGC TAAAGAATGG CGTGAGAATT TAACCCATCT
RNA      .......... .......... .......... .......... .......... .......... .......... ..........

               1050       1060       1070       1080       1090       1100       1110       1120
LRE gene GTACATAAAC TGAAGTTGAT GCCATTTCAG ATAAACTGGT TTCATTCATT ATTCCCCTTG CTGGATCCCC TGGCCGTTTT
RNA      .......... .......... .........G ATAAACTGGT TTCTTTCATT ATTCCCCTTG CTGGATCCCC TGGCCGTTTT

               1130       1140       1150       1160       1170       1180       1190       1200
LRE gene GTAGTCAGTT TGGAACGTGA AATAGCCATT CTCACGTGGG ATGGCGTTAG TGCTGCACCT ACGAGCATAG AAGCTATTGT
RNA      GTAGTCAGTT TGGAACGTGA AATAGCCATT CTTACATGGG ATGGCGTTAG TGCTGCACCT ACAAGCATAG AAGCTATTGT
```

Fig. 4

Fig 4 continued

```
                1210       1220       1230       1240       1250       1260       1270       1280
LRE gene  TAATGTCGAA CCCCACATTA AAAATAACAG ACTCAATGAT GGCAAAGCAG ATCCTCTTGG CAATCTATGG ACAGGTATAA
RNA       TAATGTCGAA CCACACATTA AAAATAACAG ACTCAATGAT GGCAAAGCAG ATCCCCTTGG CAATCTATGG ACAGGT....

                1290       1300       1310       1320       1330       1340       1350       1360
LRE gene  CTGAAATCGA CTAACCAAAT TATAATAATT CTAGCTCGAA TTCTTTAGGT ACAATGGCTA TTGACGCTGG TCTCCCCGTA
RNA       .......... .......... .......... .......... .......... ACAATGGCTA TTGACGCTGG TCTCCCCGTA

                1370       1380       1390       1400       1410       1420       1430       1440
LRE gene  GGACCGGTCA CTGGCAGTTT ATATCATTTA GGGGCTGATA AAAAGGTAAA AATGCACGAG AGCAACATAG CTATAGCAAA
RNA       GGACCGGTCA CTGGCAGTTT ATATCATTTA GGGGCTGATA AAAAGGTAAA AATGCACGAG AGCAACATAG CTATAGCAAA

                1450       1460       1470       1480       1490       1500       1510       1520
LRE gene  TGGGCTCGCG TGGAGTAATG ATTTGAAGAA AATGTATTAT ATTGATTCGG GAAAAAGAAG AGTAGACGAG TACGATTATG
RNA       TGGGCTCGCG TGGAGTAATG ATTTGAAGAA AATGTATTAT ATTGATTCGG GGAAAAGAAG AGTAGACGAG TACGATTATG

                1530       1540       1550       1560       1570       1580       1590       1600
LRE gene  ATGCTTCTAC ATTATCCATC AGTAAACACT ATTTAAATAT ATTGCACTAT TTTTTGTTAG ATGAATATTT TCAGGCAATC
RNA       ATGCTTCTAC ATTATCCATC A......... .......... .......... .......... .......... ....GCAATC

                1610       1620       1630       1640       1650       1660       1670       1680
LRE gene  AACGGCCATT ATTTACTTTT GAAAAGCATG AAGTGCCTGG ATATCCAGAT GGTCAAACAA TTGATGCGGA GGGTAATTTA
RNA       AACGGCCATT ATTTACTTTT GAAAAGCATG AAGTGCCTGG ATATCCAGAT GGTCAAACAA TTGATGAGGA GGGTAATTTA

                1690       1700       1710       1720       1730       1740       1750       1760
LRE gene  TGGGTTGCCG TTTTCCAAGG ACAGCGAATT ATTAAAATCA GTACCGAACA ACCGGAAGTG TTACTGGATA CCGTAAAAAT
RNA       TGGGTTGCCG TTTTCCAAGG ACAGCGAATT ATTAAAATCA GTACCCAACA ACCGGAAGTG TTACTGGATA CCGTAAAAAT
```

Fig. 4

Fig 4 continued

```
         .........|.........|.........|.........|.........|.........|.........|.........|
                1770      1780      1790      1800      1810      1820      1830      1840
LRE gene ACCAGATCCT CAGGTAAATT AAGTAACTTT GTGTATTACA TTAATTACCT TTGGAGTCAG GTCACCTCTG TTGCATTTGG
RNA      ACCAGATCCT CAGG...... .......... .......... .......... .......... .TCACCTCTG TAGCATTTGG

         .........|.........|.........|.........|.........|.........|.........|.........|
                1850      1860      1870      1880      1890      1900      1910      1920
LRE gene CGGTCCGAAT TTGGATGAAC TGTATGTAAC ATCTGCTGGT CTTCAGCTTG ACGACAGTTC TTTTGACAAA AGTTTAGTTA
RNA      CGGTCCGAAT TTGGATGAAC TGCATGTAAC ATCTGCTGGT CTTCAGCTTG ACGACAGTTC TTTGGACAAA AGTTTAGTTA

         .........|.........|.........|.........|.........|.........|........
                1930      1940      1950      1960      1970      1980
LRE gene ATGGGCACGT CTACAGAGTA ACAGGTTTAG GCGTCAAAGG TTTCGCGGGA GTTAAAGTGA AGCTATGA   SEQ ID NO 11
RNA      ATGGGCACGT CTACAGAGTA ACAGGTTTAG GCGTCAAAGG TTTCGCGGGA GTTAAAGTGA AGCTATGA   SEQ ID NO 2
```

Fig. 4

```
        P.pyr LRE for1
1       ATG GGG CCA GTT GTT GAA AAA ATT GCA GAA CTT GGC AAG TAT ACG    45
1       Met Gly Pro Val Val Glu Lys Ile Ala Glu Leu Gly Lys Tyr Thr    15

46      GTT GGA GAA GGT CCT CAC TGG GAT CAT GAA ACT CAG ACC TTA TAT    90
16      Val Gly Glu Gly Pro His Trp Asp His Glu Thr Gln Thr Leu Tyr    30

91      TTC GTC GAC ACC GTA GAG AAA ACT TTT CAT AAA TAT GTA CCT TCT    135
31      Phe Val Asp Thr Val Glu Lys Thr Phe His Lys Tyr Val Pro Ser    45

                                        ↓ INTRON I (990bp)
136     CAG AAA AAA TAC ACG TTT TGT AAA GTA GAT AAA CTG GTT TCT TTC    180
46      Gln Lys Lys Tyr Thr Phe Cys Lys Val Asp Lys Leu Val Ser Phe    60

181     ATT ATT CCC CTT GCT GGA TCC CCT GGC CGT TTT GTA GTC AGT TTG    225
61      Ile Ile Pro Leu Ala Gly Ser Pro Gly Arg Phe Val Val Ser Leu    75
                                ATH CTN ACN TGG GAY GG   LRE Generic FOR5-6
226     GAA CGT GAA ATA GCC ATT CTT ACA TGG GAT GGC GTT AGT GCT GCA    270
76      Glu Arg Glu Ile Ala Ile Leu Thr Trp Asp Gly Val Ser Ala Ala    90

271     CCT ACA AGC ATA GAA GCT ATT GTT AAT GTC GAA CCA CAC ATT AAA    315
91      Pro Thr Ser Ile Glu Ala Ile Val Asn Val Glu Pro His Ile Lys    105

316     AAT ACC AGA CTC AAT GAT GGC AAA GCA GAT CCC CTT GGC AAT CTA    360
106     Asn Asn Arg Leu Asn Asp Gly Lys Ala Asp Pro Leu Gly Asn Leu    120

                    ↓ INTRON II 54 bp
361     TGG ACA GGT ACA ATG GCT ATT GAC GCT GGT CTC CCC GTA GGA CCG    405
121     Trp Thr Gly Thr Met Ala Ile Asp Ala Gly Leu Pro Val Gly Pro    135
                    LRE Generic FOR1-4                AAR AAR GTN AAR
406     GTC ACT GGC AGT TTA TAT CAT TTA GGG GCT GAT AAA AAG GTA AAA    450
136     Val Thr Gly Ser Leu Tyr His Leu Gly Ala Asp Lys Lys Val Lys    150
        ATG CAY GA
451     ATG CAC GAG AGC AAC ATA GCT ATA GCA AAT GGG CTC GCG TGG AGT    495
151     Met His Glu Ser Asn Ile Ala Ile Ala Asn Gly Leu Ala Trp Ser    165

496     AAT GAT TTG AAG AAA ATG TAT TAT ATT GAT TCG GGG AAA AGA AGA    540
166     Asn Asp Leu Lys Lys Met Tyr Tyr Ile Asp Ser Gly Lys Arg Arg    180
                            TAC ATR ATR TAD CTR AGR CC LRE Generic REV1-4

                                        INTRON III 53 bp ↓
541     GTA GAC GAG TAC GAT TAT GAT GCT TCT ACA TTA TCC ATC AGC AAT    585
181     Val Asp Glu Tyr Asp Tyr Asp Ala Ser Thr Leu Ser Ile Ser Asn    195

586     CAA CGG CCA TTA TTT ACT TTT GAA AAG CAT GAA GTG CCT GGA TAT    630
196     Gln Arg Pro Leu Phe Thr Phe Glu Lys His Glu Val Pro Gly Tyr    210

631     CCA GAT GGT CAA ACA ATT GAT GAG GAG GGT AAT TTA TGG GTT GCC    675
211     Pro Asp Gly Gln Thr Ile Asp Glu Glu Gly Asn Leu Trp Val Ala    225

676     GTT TTC CAA GGA CAG CGA ATT ATT AAA ATC AGT ACC CAA CAA CCG    720
226     Val Phe Gln Gly Gln Arg Ile Ile Lys Ile Ser Thr Gln Gln Pro    240
```

Fig. 5

Fig 5 continued

INTRON IV-47bp ↓

```
721  GGA GTG TTA CTG GAT ACC GTA AAA ATA CCA GAT CCT CAG GTC ACC  765
241  Glu Val Leu Leu Asp Thr Val Lys Ile Pro Asp Pro Gln Val Thr  255

766  TCT GTA GCA TTT GGC GGT CCG AAT TTG GAT GAA CTG CAT GTA ACA  810
256  Ser Val Ala Phe Gly Gly Pro Asn Leu Asp Glu Leu His Val Thr  270

     low complexity region could be problematic for primer design
811  TCT GCT GGT CTT CAG CTT GAC GAC AGT TCT TTG GAC AAA AGT TTA  855
271  Ser Ala Gly Leu Gln Leu Asp Asp Ser Ser Leu Asp Lys Ser Leu  285

856  GTT AAT GGG CAC GTC TAC AGA GTA ACA GGT TTA GGC GTC AAA GGT  900
286  Val Asn Gly His Val Tyr Arg Val Thr Gly Leu Gly Val Lys Gly  300
                   P. pyr LRE REV1
901  TTC GCG GGA GTT AAA GTG AAG CTA TGA   927
```

**LRE Generic FOR1-6**

| | | | SEQ ID NO | |
|---|---|---|---|---|
| 5' | AAR AAR GTN AAR ATG CAY GA | 3' | 12 | |
| 1. | AAR AAR GTN AAA ATG CAC GA | | 13 | >50°C |
| 2. | AAR AAR GTN AAG ATG CAC GA | | 14 | >52°C |
| 3. | AAR AAR GTN AAA ATG CAT GA | | 15 | >50°C |
| 4. | AAR AAR GTN AAG ATG CAT GA | | 16 | >52°C |
| 5. | ATH CTN ACN TGG GAT GG | | 17 | >48°C |
| 6. | ATH CTN ACN TGG GAC GG | | 18 | >50°C |

**LRE Generic rev1-4**

| | | | SEQ ID NO | |
|---|---|---|---|---|
| 5' | CCR GAR TCD ATR TAR TAC AT | 3' | 19 | |
| 1. | CCR GAR TCD ATA TAG TAC AT | | 20 | >52°C |
| 2. | CCR GAR TCD ATG TAG TAC AT | | 21 | >54°C |
| 3. | CCR GAR TCD ATA TAA TAC AT | | 22 | >50°C |
| 4. | CCR GAR TCD ATG TAA TAC AT | | 23 | >52°C |

Fig. 5

```
           ....|....|....|....|....|....|....|....|....|....|....|....|....|....|....|....|
                   10        20        30        40        50        60        70        80
PYRmRNA    ATTCTTACATGGGATGGCGTTAGTGCTGCACCTACAAGCATAGAA------GCTATTGTTAATGTCGAACCACACATTAA
PYR SEQ    ATTCTGACGTGGGATGGCGTTAGTGCTGCACCTACGAGCATAGAA------GCTATTGTTAATGTCGAACCCCACATTAA
NOC SEQ    ATCCTGACGTGGGATGGCATAAGTTCTACACCCACAAGTATAGAAACAATCGCTGTAGTTGACTATGAGCCAGGTCTGGA

           ....|....|....|....|....|....|....|....|....|....|....|....|....|....|....|....|
                   90       100       110       120       130       140       150       160
PYRmRNA    AAATAACAGACTCAATGATGGCAAAGCAGATCCCCTTGGCAATCTATGGACAGGT-------------------------
PYR SEQ    AAATAACAGACTCAATGATGGCAAAGCAGATCCTCTTGGCAATCTATGGACAGGTATAACTGAAATCGACTAACCAAATT
NOC SEQ    AAGTAATAGAATTAATGATGGTAAAGCAGATCATCTTGGTAATCTGTGGGCAGGTGAATGAAGAAATCTATAGAACTATA
                                                                                 INTRON

           ....|....|....|....|....|....|....|....|....|....|....|....|....|....|....|....|
                  170       180       190       200       210       220       230       240
PYRmRNA    ----------------------------ACAATGGCTATTGACGCTGGTCTCCCCGTAGGACCGGTCACTGGCAGTTTA
PYR SEQ    ATNATAATTCTAGCTCGAATTCTTTAGGTACAATGGCTATTGACGCTGGTCTCCCCGTAGGACCGGTCACTGGCAGTTTA
NOC SEQ    ACATGAAATCCAACGCACCTTNTTCAGGCACCATGGACGTAAATGC--ATATTCCAACGGGACCNATAACTGGTAGATTG
                 INTRON

           ....|....|....|....|....|....|....|....|....|....|....|....|....|....|....|....|
                  250       260       270       280       290       300       310       320
PYRmRNA    TATCATTTAGGGGCTGATAAAAAGGTAAAAATGCACGAGAGCAACATAGCTATAGCAAATGGGCTCGCGTGGAGTAATGA
PYR SEQ    TATCATNTAGGGGCTGATAAAAGGGTAAAGATGCACGAGAGNNACATAGCTATAGCAAATGGGCTCGCGTGGAGTAATNA
NOC SEQ    TTTAGTATGAATTCTACTAAAGACGTTAGATACTACAGAACNNACATAGCGGTGTCAAACGGAATCGCTTGGAGTAAAGA

           ....|....|....|....|....|....|
                  330       340       350
PYRmRNA    TTTGAAGAAAATGTATTATATTGATTCGGG      (SEQ ID NO 25)
PYR SEQ    TTTGAAGAAAATGTATNATATCGATTCCGG      (SEQ ID NO 26)
NOC SEQ    TCTGAAGANAATGTATNATATNGACTCCGG      (SEQ ID NO 24)
```

Fig. 6

```
                        10         20         30         40         50         60         70         80
P.  PYR     1   ATTCTTACAT GGGATGGCGT TAGTGCTGCA CCTACAAGCA TAGAA----- -GCTATTGTT AATGTCGAAC CACACATTAA
P.  PYR SE  1   ATTCTGACGT GGGATGGCGT TAGTGCTGCA CCTACGAGCA TAGAA----- -GCTATTGTT AATGTCGAAC CCCACATTAA
L   NOC  L  1   ATCCTGACGT GGGATGGCAT AAGTTCTACA CCCACAAGTA TAGAAACATT CGCTGTAGTT GACTATGAGC CAGGTCTGGA
                                            GA TCATCTTGGT AATCTGTGGG CAGGT   LNOC LRE 3'UTR1

                        90        100        110        120        130        140        150        160
P.  PYR     75  AAATAACAGA CTCAATGATG GCAAAGCAGA TCCCCTTGGC AATCTATGGA CAGGT----- ---------- ----------
P.  PYR SE  75  AAATAACAGA CTCAATGATG GCAAAGCAGA TCCTCTTGGC AATCTATGGA CAGGTATAAC TGAAATCGAC TAACCAAATT
L   NOC  L  81  AAGTAATAGA ATTAATGATG GTAAAGCAGA TCATCTTGGT AATCTGTGGG CAGGTGAATG AAGAAATCTA TAGAACTATA
                                            CT AGTAGAACCA TTAGACACCC GTCCA   LNOC LRE 5'UTR1
                 CATTATCT TAATTACTAC CATTTCGTCT  LNOC LRE 5'UTR2

                       170        180        190        200        210        220        230        240
P.  PYR     129 ---------- ---------- ---------A CAATGGCTAT TGACGCTGGT CTCCCCGTAG GACCGGTCAC TGGCAGTTTA
P.  PYR SE  155 ATNATAATTC TAGCTCGAAT TCTTTAGGTA CAATGGCTAT TGACGCTGGT CTCCCCGTAG GACCGGTCAC TGGCAGTTTA
L   NOC  L  161 ACATGAAATC CAACGCACCT TNTTCAGGCA CCATGGACGT AAATGC--AT ATTCCAACGG GACCNATAAC TGGTAGATTG
                                   LNOC LRE 3'UTR2 GC GGTGTCAAAC GGAATCGCTT GGAGT

                       250        260        270        280        290        300        310        320
P.  PYR     181 TATCATTTAG GGGCTGATAA AAAGGTAAAA ATGCACGAGA GCAACATAGC TATAGCAAAT GGGCTCGCGT GGAGTAATGA
P.  PYR SE  235 TATCATNTAG GGGCTGATAA AAGGGTAAAG ATGCACGAGA GCAACATAGC TATAGCAAAT GGGCTCGCGT GGAGTAATNA
L   NOC  L  239 TTTAGTATGA ATTCTACTAA AGACGTTAGA TACTACAGAA CNNACATAGC GGTGTCAAAC GGAATCGCTT GGAGTAAAGA

                       330        340        350
P.  PYR     261 TTTGAAGAAA ATGTATTATA TTGATTCGGG
P.  PYR SE  315 TTTGAAGAAA ATGTATNATA TCGATTCCGG
L   NOC  L  319 TCTGAAGANA ATGTATNATA TNGACTCCGG
```

| | | SEQ ID NO |
|---|---|---|
| LNOC LRE 3'UTR1 | 5'GATCATCTTGGTAATCTGTGGGCAGGT 3' | 27 |
| LNOC LRE 3'UTR2 | 5'GCGGTGTCAAACGGAATCGCTTGGAGT 3' | 28 |
| LNOC LRE 5'UTR1 | 5'ACCTGCCCACAGATTACCAAGATGATC 3' | 29 |
| LNOC LRE 5'UTR2 | 5'TCTGCTTTACCATCATTAATTCTATTAC 3' | 30 |

Fig. 7

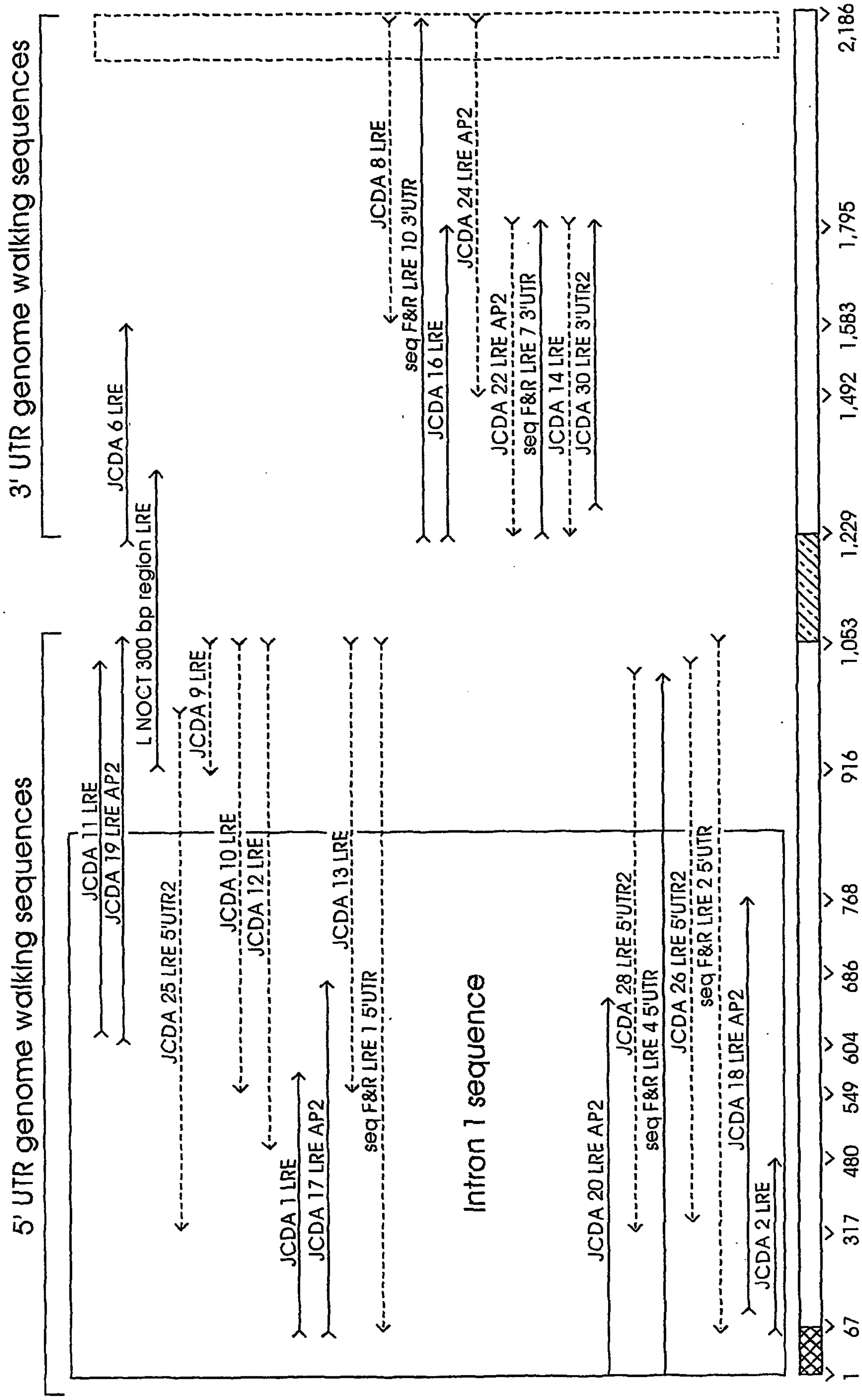
5' UTR genome walking sequences
3' UTR genome walking sequences
JCDA 11 LRE
JCDA 19 LRE AP2
L NOCT 300 bp region LRE
JCDA 6 LRE
JCDA 25 LRE 5'UTR2
JCDA 9 LRE
JCDA 10 LRE
JCDA 12 LRE
JCDA 1 LRE
JCDA 17 LRE AP2
JCDA 13 LRE
seq F&R LRE 1 5'UTR
JCDA 8 LRE
seq F&R LRE 10 3'UTR
JCDA 16 LRE
JCDA 24 LRE AP2
Intron 1 sequence
JCDA 22 LRE AP2
seq F&R LRE 7 3'UTR
JCDA 14 LRE
JCDA 30 LRE 3'UTR2
JCDA 20 LRE AP2
JCDA 28 LRE 5'UTR2
seq F&R LRE 4 5'UTR
JCDA 26 LRE 5'UTR2
seq F&R LRE 2 5'UTR
JCDA 18 LRE AP2
JCDA 2 LRE
1
67
317
480
549
604
686
768
916
1,053
1,229
1,492
1,583
1,795
2,186

Fig. 8

```
               ....|....|....|....|....|....|....|....|....|....|....|....|....|....|....|....|
                        10        20        30        40        50        60        70        80
LRE4 5'  GTACGTCTCCAAATGACTTCGTCATGAGATGTGAGCACTTCTGGTATGTGTTACGGGTAGTTAGATATCGCATTTAACGA
LRE2 5'  ------------------------------------------------------------------ATCGCATTTATCGA
LRE1 5'  ------------------------------------------------------------------AAATTACTTA--GT

               ....|....|....|....|....|....|....|....|....|....|....|....|....|....|....|....|
                        90       100       110       120       130       140       150       160
LRE4 5'  CGTTACCTAACATTTT-CTACTNATGGGGAGTGTTATATAGTAAAGTTTTGAATAATTTTCGTAAGCTAGTCACAGGTCT
LRE2 5'  CGTT-CCTAACATTTTACTACTTTTGGGGAGTGTTATATTGTAAAGTTTTGAATAATTTTCGTAAGCTAGTCACAGGTCT
LRE1 5'  CGTCTTTTGTAAGTTTATGAAAAAGTTGCATTTTTATTAATTTGAATTTTGAATAATTTTCGTAAGCTAGTCACAGGTCT

               ....|....|....|....|....|....|....|....|....|....|....|....|....|....|....|....|
                       170       180       190       200       210       220       230       240
LRE4 5'  CGATAGAAGTGTAAGCTTTAACCATAAATGTTAATGATATTATTCCATTCAATTGAGCTTCTATAAAATCTTCATTTTTT
LRE2 5'  CGATAGAAGTGTAAGCTTTAACCATAAATGTTAATGATATTATTCCATTCAATTGAGCTTCTATAAAATCTTCATTTTTT
LRE1 5'  CGATAGAAGTGTAAACTTTAACCATAAATGTTAATAATATTATTCTATTCAACTGAGCTTCTATAAAATCTTCATTTTT-

               ....|....|....|....|....|....|....|....|....|....|....|....|....|....|....|....|
                       250       260       270       280       290       300       310       320
LRE4 5'  GGTTTATAATACTGATGTGATCACTGCTAGATTACGTTNTTCGTAGAATTGGCAATATAATTATTTA------AGCCGTA
LRE2 5'  GGTTTATAATGCTGACGTGATCACTGCTAGATTACGTTATTCGTAGAATTTGCAATATAATTATTTA------AGCCG-A
LRE1 5'  GGTTTATAATACTGATGTAATCTCTGCTAGATTACGCT-TTGGTAGAAT-GGCAATATAATTAATAATTATTTAATTCTA

               ....|....|....|....|....|....|....|....|....|....|....|....|....|....|....|....|
                       330       340       350       360       370       380       390       400
LRE4 5'  AATATTTCATCA------------GTGTAATTGTCACTACGCATCACTTTTTTTTGTCTGTCTAGTACAGCTTACTTCCC
LRE2 5'  AATATTTCATCAATGAGTACCTCAGTGTAATTGTCACTACGCATCACTTTTTTTTGTCTGTCTAGTACAGCTTACTTCCC
LRE1 5'  AATACTTGATCAATGAGTACCTCCGTGTAATTGTCACTACGCATCACTTTTTTT-GTCTGTCTAGTACAGCTTACTTCCC

               ....|....|....|....|....|....|....|....|....|....|....|....|....|....|....|....|
                       410       420       430       440       450       460       470       480
LRE4 5'  TCGTTATTTGTGTAATTTCGCATAGTTATGGATAATATCTAGTATTATCTAAATTTACCTATNGTGTATTACGCCATGTA
LRE2 5'  TCGTTATTTGTGTAATTTCGTATAGTTATGGATAATATCTAGTATTATCTAAATTTACCTATNGTGTATTACGTCATGTA
LRE1 5'  TCGTTATTTGTGTAATTTCGTGTAGTTATGGATAATATCTAGTCTTATCTAAATTTA-------TGTACTACGTCATGTA
```

Fig. 9

```
                 490       500       510       520       530       540       550       560
LRE4 5' GTTAAAGGTTGCTGTATTATCTTATCATACATTGAAGTTAA-TACACAAGTAAAAACCTTGATTTTTATAGAAACCTACG
LRE2 5' GTTAAAGGTTGCTGTATTA-----TCATACATAGAAGTTAA-TACACAAGTAAAAACCTTGATTTTTATAGAAACCTACG
LRE1 5' GTTAAAGATTGCTGTATCA-----TCATACATTAAAGTTAAATGCAAAAGTAAAAACATTGATTTTTATAGAAAACTACG

                 570       580       590       600       610       620       630       640
LRE4 5' ACAGCATTGCAACGGCACTAGTTAATAGAAACAGTGGAAGAAGTGGCCAATATGCAGTTTGGAGAAGGACCTCATTGGAA
LRE2 5' ACAGCATTGCAACGGCACTAGTTA-TAGAAACAGTTGAAGAAGTGGCCAATATGCAGTTTGGAGAAGGACCTCATTGGGA
LRE1 5' GCAGCATTGCAACGGCACTAGTTA-TAGAAACAGTTGAAGAATTGGGCACTTTTAAGACTGGAGAAGGACCTCATTGGGA

                 650       660       670       680       690       700       710       720
LRE4 5' TGATAAGACACAAAGTTTATATTTGGTGGACCTTGTAGATAAATCTATTCACAAATATGTACCATCTCTTAAAAGACAAA
LRE2 5' TGATAAGACACAAAGTTTATATTTGGTGGACCTTGTAGATAAATCTATTCACAAATATGTACCATCTCTTAAAAGACATA
LRE1 5' TGATAAAACACAAAGTTTATATTTGGTGGACCTTGTAGATAAATCTATTCACAAATATGTACCATCTCTTAAAAGACATA

                 730       740       750       760       770       780       790       800
LRE4 5' CCCATATAAAAGTAGGCAAGTATTATTACAACTTGTTATATCCGAGTCAACCAAATAATGTAAAAAATTTACGTCAGTCC
LRE2 5' CCCATATAAAAGTAGGCAAGTATTATTACAACTTGTTATATCCGAGTCAACCAAATAATGTAAAAAATTTACGTCAGTCC
LRE1 5' CCCATATAAAAGTAGGCAAGTATTATTACAACTTGTTATATCCAAGTCAACCAAATAATGTAAAAAATTTACGTCAGTCC

                 810       820       830       840       850       860       870       880
LRE4 5' CACTATAA--GATTTAAGAAATTCATAAATATAAATTTCTATGCTCATTTTCAGATAAAATACCGTCTTTGATTATTGCC
LRE2 5' CACTATAA--GATTTAAGAAATTCATAAGTATAAATTTCTATGCTCATTTTCAGATAAAATACCGTCTTTGATTATTGCC
LRE1 5' CACATTCATAAATATAAATATATCATAAATATAAATTTCTATGCTCATTTTCAGATAAAATACCGTCTTTGATTATTCCC

                 890       900       910       920       930       940       950       960
LRE4 5' ATAAGCGGTTGTTCAAATCGTTTTCTTATTACTTTAGACCGAGAAATCGCCATCCTTACTTGGGATGGCATTAGTTCTAC
LRE2 5' ATAAGCGGTTGTTCAAATCGTTTTCTTATTACTTTAGACCGAGAAATCGCCATCCTTACTTGGGATGGCATTAGTTCTAC
LRE1 5' ATAAGCGGTTGTTCAAATCGTTTTCTTATTACTTTAGACCGAGAAATCGCCATCCTTACTTGGGATGGTATAAGTTCTAC

                 970       980       990      1000      1010      1020      1030      1040
LRE4 5' ACCCACAAGTATAGAAACAATCGCTGTAGTTGA-----------------------------------------------  SEQ ID NO 10
LRE2 5' ACCCACAAGTATAGAAACAATCGCTGTAGTTGACAATGAGCCAGGTCTTGAAAGTAATAGAATTAATGATGGTAAAGCAGA  SEQ ID NO 64
LRE1 5' ACCCACAAGTATAAAAACAATCGCTGTAGTTGACAATGAGCCAGGTCTGGAAAGTAATAGAATTAATGATGGTAAAGCAGA  SEQ ID NO 65
```

Fig 9 continued

Fig. 9

**L.noc AR5 pp 1**

1 GAT AAA ATA CCG TCT TTG ATT ATT CCC ATA AGC GGT TGT TCA AAT 45
1 Asp Lys Ile Pro Ser Leu Ile Ile Pro Ile Ser Gly Cys Ser Asn 15

46 CGT TTT CTT ATT ACT TTA GAC CGA GAA ATC GCC ATC CTT ACT TGG 90
16 Arg Phe Leu Ile Thr Leu Asp Arg Glu Ile Ala Ile Leu Thr Trp 30

91 GAT GGT ATA AGT TCT ACA CCC ACA AGT ATA AAA ACA ATC GCT GTA 135
31 Asp Gly Ile Ser Ser Thr Pro Thr Ser Ile Lys Thr Ile Ala Val 45

136 GTT GAC AAT GAG CCA GGT CTG GAA AGT AAT AGA ATT AAT GAT GGT 180
46 Val Asp Asn Glu Pro Gly Leu Glu Ser Asn Arg Ile Asn Asp Gly 60

181 AAA GCA GAT CAT CTT GGT AAT CTG TGG GCA GGT GAA TGA AGA AAT 225
61 Lys Ala Asp His Leu Gly Asn Leu Trp Ala Gly 75

226 CTA TAG AAC TAT AAC ATG AAA TCC AAC GCA CCT TTT TCA GGC ACC 270
76 **Intron 2** Thr 90

271 ATG GAC GTA AAT GCT TTT CCA ACG GGA CCA ATA ACT GGT AGA TTG 315
91 Met Asp Val Asn Ala Phe Pro Thr Gly Pro Ile Thr Gly Arg Leu 105

316 TTT AGT ATG AAT TCT ACT AAA GAC GTT AGA TAC TAC AGA ACA AAC 360
106 Phe Ser Met Asn Ser Thr Lys Asp Val Arg Tyr Tyr Arg Thr Asn 120

361 ATA GCG GTG TCA AAC GGA ATC GCT TGG AGT AAA GAT TTG AAG AAA 405
121 Ile Ala Val Ser Asn Gly Ile Ala Trp Ser Lys Asp Leu Lys Lys 135

406 ATG TAT TAT ATT GAT TCC AAG ACT AGA GTA ATA GAT CAA TAC GAT 450
136 Met Tyr Tyr Ile Asp Ser Lys Thr Arg Val Ile Asp Gln Tyr Asp 150

451 TTT GAT GCT TCC AAC AGA TTG ATC AGT TAC AGT AAA TGA TTT ACA 495
151 Phe Asp Ala Ser Asn Arg Leu Ile Ser 165

496 TTC GTT GCA TAT TTA TGT TGT TTA TTT TCA GGT AAT CGT CAA ACA 540
166 **Intron 3** Asn Arg Gln Thr 180

541 TTG TTT AGT CTG GAA AAG CAT CAA ATC CCG GGA TTT CCC GAT GGT 585
181 Leu Phe Ser Leu Glu Lys His Gln Ile Pro Gly Phe Pro Asp Gly 195

586 CAA ACA ATT GAT ACT GAT GAT AAT TTA TGG GTT GGT GTG TTC GAA 630
196 Gln Thr Ile Asp Thr Asp Asp Asn Leu Trp Val Gly Val Phe Glu 210

631 GGA AAC AGA GTG ATA AAA ATT AGT ACT CAT ATA CCT GAA ACA TTA 675
211 Gly Asn Arg Val Ile Lys Ile Ser Thr His Ile Pro Glu Thr Leu 225

676 CTG TAT ACT GTA AAC CTT CCA GAT TCG CTG GT GAG TAA AAG TAT 720
226 Leu Tyr Thr Val Asn Leu Pro Asp Ser Leu Val 240

721 TGA CAT TTT CAA ATA ATG TAG ATG TTT ATT AGA T A ACC TCG GTT 765
241 **Intron 4** Thr Ser Val 255

766 GCT TTT GGT GGA CCA AAC TTG GAT GAA TTG TAC GTA ACA GGT GGT 810
256 Ala Phe Gly Gly Pro Asn Leu Asp Glu Leu Tyr Val Thr Gly Gly 270

811 GGT AAC TTT CTG AAT GGG AAA GTT TAT AAA GTA TCT GGT CTA GGT 855
271 Gly Asn Phe Leu Asn Gly Lys Val Tyr Lys Val Ser Gly Leu Gly 285

856 GTT CAA GGT 864 (SEQ ID NO 4)
286 Val Gln Gly (SEQ ID NO 3)

Fig. 10

Start of Exon 2 ⟶

```
                  ....|....| ....|....| ....|....| ....|....| ....|....| ....|....| ....|....| ....|....|
                         10         20         30         40         50         60         70         80
L.noc AR5 pp1     GATAAAATAC CGTCTTTGAT TATTCCCATA AGCGGTTGTT CAAATCGTTT TCTTATTACT TTAGACCGAG AAATCGCCAT
L.noc AR5 pp2     .......... .......... .......... .......... .......... .......... .......... ..........
L.noc AR5 pp4     .......... .......... .G........ .......... .......... .......... .......... ..........
L.noc AR5 pp3     .......... .......... .......... .......... .......... .......... .......... ..........
L.noc AR5 pp5     .......... .......... .......... .......... .......... .......... .......... ..........
L.noc AR1         .......... .......... .......... .......... .......... .......... .......... ..........
L.noc AR3         .......... .......... .......... .......... .......... .......... .......... ..........
L.noc AR4         .......... .......... .......... .......... .......... .......... .......... ..........

                  ....|....| ....|....| ....|....| ....|....| ....|....| ....|....| ....|....| ....|....|
                         90        100        110        120        130        140        150        160
L.noc AR5 pp1     CCTTACTTGG GATGGTATAA GTTCTACACC CACAAGTATA AAAACAATCG CTGTAGTTGA CAATGAGCCA GGTCTGGAAA
L.noc AR5 pp2     .......... .......... .......... .......... .......... .......... .......... ..........
L.noc AR5 pp4     .......... .......... .......... .......... .......... .......... .......... ..........
L.noc AR5 pp3     .......... .....C..T. .......... .......... G......... .......... .......... .....T....
L.noc AR5 pp5     .......... .....C..T. .......... .......... G......... .......... .......... .....T....
L.noc AR1         .......... .....C..T. .......... .......... G......... .......... .......... .....T....
L.noc AR3         .......... .....C..T. .......... .......... G......... .......... .......... .....T....
L.noc AR4         .......... .....C..T. .......... .......... G......... .......... .......... .....T....

                  ....|....| ....|....| ....|....| ....|....| ....|....| ....|....| ....|....| ....|....|
                        170        180        190        200        210        220        230        240
L.noc AR5 pp1     GTAATAGAAT TAATGATGGT AAAGCAGATC ATCTTGGTAA TCTGTGGGCA GGTGAATGAA GAAATCTATA GAACTATAAC
L.noc AR5 pp2     .......... .......... .......... .......... C......... .......... .......... ..........
L.noc AR5 pp4     .......... .......... .......... .......... .......... .......... .......... ..........
L.noc AR5 pp3     A......... .......... .......... .......... .......... .......... .......... A.........
L.noc AR5 pp5     A......... .......... .......... .......... .......... .......... .......... A.........
L.noc AR1         A......... .......... .......... .......... .......... .......... .......... A.........
L.noc AR3         A......... .......... .......... .......... .......... .......... .......... A.........
L.noc AR4         A......... .......... .......... .......... .......... .......... .......... A.........
```

Fig. 11

Fig 11 continued

```
                    ....|....| ....|....| ....|....| ....|....| ....|....| ....|....| ....|....| ....|....|
                           250        260        270        280        290        300        310        320
L.noc AR5 pp1       ATGAAATCCA ACGCACCTTT TTCAGGCACC ATGGACGTAA ATGCTTTTCC AACGGGACCA ATAACTGGTA GATTGTTTAG
L.noc AR5 pp2       .......... .......... .......... .......... .......... .......... .......... ..........
L.noc AR5 pp4       .......... .......... .......... .......... .......... .......... G......... ..........
L.noc AR5 pp3       ........A. .......... .......... .......... ...GA.A... .......... .....C.... ..........
L.noc AR5 pp5       ........A. .......... .......... .......... ...GA.A... .......... .....C.... ..........
L.noc AR1           ........A. .......... .......... .......... ...GA.A... .......... T....C.... ..........
L.noc AR3           ........A. .......... .......... .......... ...GA.A... .......... .....C.... ..........
L.noc AR4           ........A. .......... .......... .......... ...GA.A... .......... .....C.... ..........

                    ....|....| ....|....| ....|....| ....|....| ....|....| ....|....| ....|....| ....|....|
                           330        340        350        360        370        380        390        400
L.noc AR5 pp1       TATGAATTCT ACTAAAGACG TTAGATACTA CAGAACAAAC ATAGCGGTGT CAAACGGAAT CGCTTGGAGT AAAGATTTGA
L.noc AR5 pp2       .......... .......... .......... .......... .......... .......... .......... ..........
L.noc AR5 pp4       .......... .......... .......... .......... .......... .......... .......... ..........
L.noc AR5 pp3       ...A...... .......... .......... .......... .......... .......... ...C...... ..........
L.noc AR5 pp5       ...A...... .......... .......... .......... .......... .......... .........C ..........
L.noc AR1           ...A...... .......... .......... .......... .......... .......... .......... ..........
L.noc AR3           ...A...... .......... .......... .......... .......... .......... .......... ..........
L.noc AR4           ...A...... .......... .......... .......... .......... .......... .......... ..........
                       Diagnostic Eco RI site

                    ....|....| ....|....| ....|....| ....|....| ....|....| ....|....| ....|....| ....|....|
                           410        420        430        440        450        460        470        480
L.noc AR5 pp1       AGAAAATGTA TTATATTGAT TCCAAGACTA GAGTAATAGA TCAATACGAT TTTGATGCTT CCAACAGATT GATCAGTATA
L.noc AR5 pp2       .......... .......... .......... .......... .......... .......... .......... ..........
L.noc AR5 pp4       .......... .......... .......... .......... .......... .......... .......... ..........
L.noc AR5 pp3       .......... .......... ....T..... A......... .......... .......... .......... ..........
L.noc AR5 pp5       .......... .......... ....T..... A......... .......... .......... .......... ..........
L.noc AR1           .......... .......... ....T..... A......... .......... .......... .......... ..........
L.noc AR3           .......... .......... ....T..... A........G .......... .......... .......... ..........
L.noc AR4           .......... .......... ....T..... A......... .......... .......... .......... ..........
```

Fig. 11

Fig 11 continued

```
                 ....|....| ....|....| ....|....| ....|....| ....|....| ....|....| ....|....| ....|....|
                        490        500        510        520        530        540        550        560
L.noc AR5 pp1    CAGTAAATGA TTTACATTCG TTGCATATTT ATGTTGTTTA TTTTCAGGTA ATCGTCAAAC ATTGTTTAGT CTGGAAAAGC
L.noc AR5 pp2    .......... .......... .......... .......... .......... .......... .......... ..........
L.noc AR5 pp4    .......... .......... .......... .......... .......... .......... .......... ..........
L.noc AR5 pp3    .......... .AA....... ..A....... .......A.. .......... .......... C......... ..T.....TA
L.noc AR5 pp5    .......... .AA....... ..A....... .......A.. .......... .......... C......... ..T.....TA
L.noc AR1        .......... .AA....... ..A....... .......A.. .......... .......... C......... ..T.....TA
L.noc AR3        .......... .AA....... ..A....... .......A.. .......... .......... C......... ..T......A
L.noc AR4        .......... .AA....... ..A....... .......A.. .......... .......... C......... ..T......A

                 ....|....| ....|....| ....|....| ....|....| ....|....| ....|....| ....|....| ....|....|
                        570        580        590        600        610        620        630        640
L.noc AR5 pp1    ATCAAATCCC GGGATTTCCC GATGGTCAAA CAATTGATAC TGATGATAAT TTATGGGTTG GTGTGTTCGA AGGAAACAGA
L.noc AR5 pp2    .......... .......... .......... .......... .......... .......... .......... ..........
L.noc AR5 pp4    .......... .......... .......... .......... .......... .......... .......... ..........
L.noc AR5 pp3    .......... .........A .......... .......... .......... .......... C........G ..........
L.noc AR5 pp5    .......... .........A .......... .......... .......... .......... C........G ..........
L.noc AR1        .......... .........A .......... .......... .......... .......... .......... ..........
L.noc AR3        ...G...... .........A .........M ....A..... .......... .......... C........G ..........
L.noc AR4        .......... .........A .......... .......... .......... .......... C........G ..........

                 ....|....| ....|....| ....|....| ....|....| ....|....| ....|....| ....|....| ....|....|
                        650        660        670        680        690        700        710        720
L.noc AR5 pp1    GTGATAAAAA TTAGTACTCA TATACCTGAA ACATTACTGT ATACTGTAAA CCTTCCAGAT TCGCTGGTGA GTAAAAGTAT
L.noc AR5 pp2    .......... .......... .......... .......... .......... ..C....... .......... ..........
L.noc AR5 pp4    .......... .......... .......... .......... .......... .......... .......... ..........
L.noc AR5 pp3    .......... ........G. .......... .......... .......... .A........ ....A..... .....T.G..
L.noc AR5 pp5    .......... ........G. .......... .......... .......... .A........ ....A..... .....T.G..
L.noc AR1        .......... .......... .......... .......... .......... .......... .......... ..........
L.noc AR3        .......... ........G. .......... .......... .......... .A........ ....A..... .....T.G..
L.noc AR4        .......... ........G. .......... .......... .......... .A........ ....A..... .....T.G..
```

Fig. 11

Fig 11 continued

```
                   ....|....| ....|....| ....|....| ....|....| ....|....| ....|....| ....|....| ....|....|
                          730        740        750        760        770        780        790        800
L.noc AR5 pp1      TGACATTTTC AAATAATGTA GATGTTTATT AGATAACCTC GGTTGCTTTT GGTGGACCAA ACTTGGATGA ATTGTACGTA
L.noc AR5 pp2      .......... .......... .......... .......... .......... .......... .......... ..........
L.noc AR5 pp4      .......... .......... .......... .......... .......... .......... .......... ..........
L.noc AR5 pp3      .......... .......... .......... .......... .......... .......... .......... ..........
L.noc AR5 pp5      .......... .......... .......... .......... .......... .......... .......... ..........
L.noc AR1          .......... .......... .......... .......... .......... .......... .......... ..........
L.noc AR3          .......... .......... .......... .......... .......... .......... .......... ..........
L.noc AR4          .......... .......... .......... .......... .......... .......... .......... ..........

                   ....|....| ....|....| ....|....| ....|....| ....|....| ....|....| ....
                          810        820        830        840        850        860
L.noc AR5 pp1      ACAGGTGGTG GTAACTTTCT GAATGGGAAA GTTTATAAAG TATCTGGTCT AGGTGTTCAA GGT.
L.noc AR5 pp2      .......... .......... .......... .......... ..A....... .......... ....
L.noc AR5 pp4      .......... .......... .......... .......... .......... .......... ....
L.noc AR5 pp3      ...A...... ..-------- ----...... ........G. ..A....... .......... ....
L.noc AR5 pp5      ...A...... ..-------- ----...... ........G. ..A....... .......... ....
L.noc AR1          .......... .......... .......... .......... .......... .......... ....
L.noc AR3          ...A...... ..-------- ----...... ........G. ..A....... .......... ....
L.noc AR4          ...A...... ..-------- ----...... ........G. ..A....... .......... ...@
```

Lnoc LRE REV 1 (5' ACCTTGAACACCTAGACCAGWT 3')

Fig. 11

Complete Gene sequence for the luciferin recycling enzyme from *Lampyris noctiluca* and the putative mRNA and protein sequence

```
              ....|....|....|....|....|....|....|....|....|....|....|....|....|....|....|....|....|....|
                      10        20        30        40        50        60        70        80        90
L. noct gene  ATGCAGTTTGGAGAAGGACCTCATTGGGATGATAAGACACAAAGTTTATATTTGGTGGACCTTGTAGATAAATCTATTCACAAATATGTA
L. noct mRNA  ATGCAGTTTGGAGAAGGACCTCATTGGGATGATAAGACACAAAGTTTATATTTGGTGGACCTTGTAGATAAATCTATTCACAAATATGTA
              MetGlnPheGlyGluGlyProHisTrpAspAspLysThrGlnSerLeuTyrLeuValAspLeuValAspLysSerIleHisLysTyrVal

              ....|....|....|....|....|....|....|....|....|....|....|....|....|....|....|....|....|....|
                     100       110       120       130       140       150       160       170       180
L. noct gene  CCATCTCTTAAAAGACATACCCATATAAAAGTAGGCAAGNANTATATACAACTTGTTATATCCGAGTCAACCAAATAATGTAAAAAATTT
L. noct mRNA  CCATCTCTTAAAAGACATACCCATATAAAAGTA---------------------------------------------------------
              ProSerLeuLysArgHisThrHisIleLysVal

              ....|....|....|....|....|....|....|....|....|....|....|....|....|....|....|....|....|....|
                     190       200       210       220       230       240       250       260       270
L. noct gene  ACGTCAGTCCCACTATAAGATTTAAGAAATTCATAAATATAAATTTCTATGCTCATTTTCAGATAAAATACCGTCTTTGATTATTCCCAT
L. noct mRNA  -------------------------------------------------------------GATAAAATACCGTCTTTGATTATTCCCAT
                                                                           AspLysIleProSerLeuIleIleProIl
              ....|....|....|....|....|....|....|....|....|....|....|....|....|....|....|....|....|....|
                     280       290       300       310       320       330       340       350       360
L. noct gene  AAGCGGTTGTTCAAATCGTTTTCTTATTACTTTAGACCGAGAAATCGCCATCCTTACTTGGGATGGCATTAGTTCTACACCCACAAGTAT
L. noct mRNA  AAGCGGTTGTTCAAATCGTTTTCTTATTACTTTAGACCGAGAAATCGCCATCCTTACTTGGGATGGCATTAGTTCTACACCCACAAGTAT
              eSerGlyCysSerAsnArgPheLeuIleThrLeuAspArgGluIleAlaIleLeuThrTrpAspGlyIleSerSerThrProThrSerIl
              ....|....|....|....|....|....|....|....|....|....|....|....|....|....|....|....|....|....|
                     370       380       390       400       410       420       430       440       450
L. noct gene  AGAAACAATCGCTGTAGTTGACAATGAGCCAGGTCTTGAAAATAATAGAATTAATGATGGTAAAGCAGATCATCTTGGTAATCTGTGGGC
L. noct mRNA  AGAAACAATCGCTGTAGTTGACAATGAGCCAGGTCTTGAAAATAATAGAATTAATGATGGTAAAGCAGATCATCTTGGTAATCTGTGGGC
              eGluThrIleAlaValValAspAsnGluProGlyLeuGluAsnAsnArgIleAsnAspGlyLysAlaAspHisLeuGlyAsnLeuTrpAl
```

Fig. 12

Fig 12 continued

```
               ....|....|....|....|....|....|....|....|....|....|....|....|....|....|....|....|....|....|
                       460       470       480       490       500       510       520       530       540
L. noct gene   AGGTGAATGAAGAAATCTATAAAACTATAACATGAAATCAAACGCACCTTTTTCAGGCACCATGGACGTAAATGGATATCCAACGGGACC
L. noct mRNA   AGGT------------------------------------------------------ACCATGGACGTAAATGGATATCCAACGGGACC
               aGly                                                     ThrMetAspValAsnGlyTyrProThrGlyPr

               ....|....|....|....|....|....|....|....|....|....|....|....|....|....|....|....|....|....|
                       550       560       570       580       590       600       610       620       630
L. noct gene   AATAACCGGTAGATTGTTTAGTATAAATTCTACTAAAGACGTTAGATACTACAGAACAAACATAGCGGTGTCAAACGGAATCGCTTGGAG
L. noct mRNA   AATAACCGGTAGATTGTTTAGTATAAATTCTACTAAAGACGTTAGATACTACAGAACAAACATAGCGGTGTCAAACGGAATCGCTTGGAG
               oIleThrGlyArgLeuPheSerIleAsnSerThrLysAspValArgTyrTyrArgThrAsnIleAlaValSerAsnGlyIleAlaTrpSe

               ....|....|....|....|....|....|....|....|....|....|....|....|....|....|....|....|....|....|
                       640       650       660       670       680       690       700       710       720
L. noct gene   TAAAGATTTGAAGAAAATGTATTATATTGATTCCATGACTAAAGTAATAGGTCAATACGATTTTGATGCTTCCAACAGATTGATCAGTAT
L. noct mRNA   TAAAGATTTGAAGAAAATGTATTATATTGATTCCATGACTAAAGTAATAGGTCAATACGATTTTGATGCTTCCAACAGATTGATCAGTA-
               rLysAspLeuLysLysMetTyrTyrIleAspSerMetThrLysValIleGlyGlnTyrAspPheAspAlaSerAsnArgLeuIleSerA

               ....|....|....|....|....|....|....|....|....|....|....|....|....|....|....|....|....|....|
                       730       740       750       760       770       780       790       800       810
L. noct gene   ACAGTAAATGATAAACATTCGTTACATATTTATGTTGTATATTTTCAGGTAATCGTCAAACCTTGTTTAGTCTTGAAAAGAATCGAATCC
L. noct mRNA   ---------------------------------------------------ATCGTCAAACCTTGTTTAGTCTTGAAAAGAATCGAATCC
                                                                  snArgGlnThrLeuPheSerLeuGluLysAsnArgIleP

               ....|....|....|....|....|....|....|....|....|....|....|....|....|....|....|....|....|....|
                       820       830       840       850       860       870       880       890       900
L. noct gene   CGGGATTTCCAGATGGTCAAACAATAGATACTGATGATAATTTATGGGTTGCTGTGTTCGGAGGAAACAGAGTGATAAAAATTAGTACTG
L. noct mRNA   CGGGATTTCCAGATGGTCAAACAATAGATACTGATGATAATTTATGGGTTGCTGTGTTCGGAGGAAACAGAGTGATAAAAATTAGTACTG
               roGlyPheProAspGlyGlnThrIleAspThrAspAspAsnLeuTrpValAlaValPheGlyGlyAsnArgValIleLysIleSerThrA
```

Fig. 12

Fig 12 continued

```
               ....|....|....|....|....|....|....|....|....|....|....|....|....|....|....|....|....|....|
                       910       920       930       940       950       960       970       980       990
L. noct gene   ATATACCTGAAACATTACTGTATACTGTAAACATTCCAGATTCGCAGGTGAGTAAATGGATTGACATTTTCAAATAATGTAGATGTTTAT
L. noct mRNA   ATATACCTGAAACATTACTGTATACTGTAAACATTCCAGATTCGCAGGT-----------------------------------------
               spIleProGluThrLeuLeuTyrThrValAsnIleProAspSerGlnVa

               ....|....|....|....|....|....|....|....|....|....|....|....|....|....|....|....|....|....|
                      1000      1010      1020      1030      1040      1050      1060      1070      1080
L. noct gene   TAGATAACCTCGGTTGCTTTTGGTGGACCAAACTTGGATGAATTGTACGTAACAAGTGGTGGTGGGAAAGTTTATAAGGTAACTGGTCTA
L. noct mRNA   -----AACCTCGGTTGCTTTTGGTGGACCAAACTTGGATGAATTGTACGTAACAAGTGGTGGTGGGAAAGTTTATAAGGTAACTGGTCTA
                    LThrSerValAlaPheGlyGlyProAsnLeuAspGluLeuTyrValThrSerGlyGlyGlyLysValTyrLysValThrGlyLeu

               ....|....|....|....|....|....|....|....|
                      1090      1100      1110      1120 1125
L. noct gene   GGTGTTCAAGGTTTACCTGCAAATAGGATTAAAATACATAATTAA                SEQ ID NO 40
L. noct mRNA   GGTGTTCAAGGTTTACCTGCAAATAGGATTAAAATACATAATTAA                SEQ ID NO 41
               GlyValGlnGlyLeuProAlaAsnArgIleLysIleHisAsnEnd                SEQ ID NO 39
```

Fig. 12

```
                   ....|....|....|....|....|....|....|....|....|....|....|....|....|....|....|....|....|....|
                            10        20        30        40        50        60        70        80        90
L. noct mRNA       M------------- FGEGPHWDDKTQSLY VD VDKS HKYVPS KRHTH KVDKI S IIPISGCSNRFLITLDREIAILTWDGISST
P. pyralis mRNA    MG   EK   E GKYT GEGPHWDHETQTLYFVDTVEKTFHKYVPS KKYTFCKVDKL SFIIPLAGSPGRFVVSLEREIAILTWDGVSAA

                   ....|....|....|....|....|....|....|....|....|....|....|....|....|....|....|....|....|....|
                           100       110       120       130       140       150       160       170       180
L. noct mRNA       PTSIETI  VDNEPGLENNRINDGKADHLGNLWAGTMDVN-GYPTGPITGRLFSINSTKDVRYYRTNIAVSNGIAWSKDLKKMYYIDS T
P. pyralis mRNA    PTSIEAI NV--EPHIKNNRLNDGKAD LGNLWTGTM ID G P GPVTGSLYHLGADKKVK HESNIAIANGLAWSNDLKKMYYIDSGK

                   ....|....|....|....|....|....|....|....|....|....|....|....|....|....|....|....|....|....|
                           190       200       210       220       230       240       250       260       270
L. noct mRNA       K IGQYDFDASNR ISNRQTLFS EKNRIPGFPDGQTIDTDDNLWVAVFGGNRVIKISTD PETLLYTVNIPDSQVTSVAFGGPNLDELY
P. pyralis mRNA    RRVDEYDYDAST SISNQR LFTFEKHEVPGYPDGQTIDEEGNLWVAVF G RIIKISTQ PE LLDTVKIPDPQVTSVAFGGPNLDELH

                   ....|....|....|....|....|....|....|....|..
                           280       290       300       310
L. noct mRNA       VTSGG--------------GKVYKVTGLGV G PANRIKIHN
P. pyralis mRNA    VTSAG   DDSS DKS  NGHVYRVTGLGVKGFAG KVKL--
```

Fig. 13

```
                  ....|....|....|....|....|....|....|....|....|....|....|....|....|....|....|....|....|....|
                           10        20        30        40        50        60        70        80        90
L. noct gene      ATGCAGTTTGGAGAAGGACCTCATTGGGATGATAAGACACAAAGTTTATATTTGGTGGACCTTGTAGATAAATCTATTCACAAATATGTA
L. noct mRNA      ATGCAGTTTGGAGAAGGACCTCATTGGGATGATAAGACACAAAGTTTATATTTGGTGGACCTTGTAGATAAATCTATTCACAAATATGTA
                  MetGlnPheGlyGluGlyProHisTrpAspAspLysThrGlnSerLeuTyrLeuValAspLeuValAspLysSerIleHisLysTyrVal

                  ....|....|....|....|....|....|....|....|....|....|....|....|....|....|....|....|....|....|
                          100       110       120       130       140       150       160       170       180
L. noct gene      CCATCTCTTAAAAGACATACCCATATAAAAGTAGGCAAGNANTATATACAACTTGTTATATCCGAGTCAACCAAATAATGTAAAAAATTT
L. noct mRNA      CCATCTCTTAAAAGACATACCCATATAAAAGTAG--------------------------------------------------------
                  ProSerLeuLysArgHisThrHisIleLysValA

                  ....|....|....|....|....|....|....|....|....|....|....|....|....|....|....|....|....|....|
                          190       200       210       220       230       240       250       260       270
L. noct gene      ACGTCAGTCCCACTATAAGATTTAAGAAATTCATAAATATAAATTTCTATGCTCATTTTCAGATAAAATACCGTCTTTGATTATTCCCAT
L. noct mRNA      --------------------------------------------------------------ATAAAATACCGTCTTTGATTATTCCCAT
                                                                                spLysIleProSerLeuIleIleProIl

                  ....|....|....|....|....|....|....|....|....|....|....|....|....|....|....|....|....|....|
                          280       290       300       310       320       330       340       350       360
L. noct gene      AAGCGGTTGTTCAAATCGTTTTCTTATTACTTTAGACCGAGAAATCGCCATCCTTACTTGGGATGGCATTAGTTCTACACCCACAAGTAT
L. noct mRNA      AAGCGGTTGTTCAAATCGTTTTCTTATTACTTTAGACCGAGAAATCGCCATCCTTACTTGGGATGGCATTAGTTCTACACCCACAAGTAT
                  eSerGlyCysSerAsnArgPheLeuIleThrLeuAspArgGluIleAlaIleLeuThrTrpAspGlyIleSerSerThrProThrSerIl

                  ....|....|....|....|....|....|....|....|....|....|....|....|....|....|....|....|....|....|
                          370       380       390       400       410       420       430       440       450
L. noct gene      AGAAACAATCGCTGTAGTTGACAATGAGCCAGGTCTTGAAAATAATAGAATTAATGATGGTAAAGCAGATCATCTTGGTAATCTGTGGGC
L. noct mRNA      AGAAACAATCGCTGTAGTTGACAATGAGCCAGGTCTTGAAAATAATAGAATTAATGATGGTAAAGCAGATCATCTTGGTAATCTGTGGGC
                  eGluThrIleAlaValValAspAsnGluProGlyLeuGluAsnAsnArgIleAsnAspGlyLysAlaAspHisLeuGlyAsnLeuTrpAl
```

Fig. 14

Fig 14 continued

```
              ....|....|....|....|....|....|....|....|....|....|....|....|....|....|....|....|....|....|
                       460       470       480       490       500       510       520       530       540
L. noct gene  AGGTGAATGAAGAAATCTATAAAACTATAACATGAAATCAAACGCACCTTTTTCAGGCACCATGGACGTAAATGGATATCCAACGGGACC
L. noct mRNA  AG------------------------------------------------------GTACCATGGACGTAAATGGATATCCAACGGGACC
              aG                                                        lyThrMetAspValAsnGlyTyrProThrGlyPr
              TC
              ....|....|....|....|....|....|....|....|....|....|....|....|....|....|....|....|....|....|
                       550       560       570       580       590       600       610       620       630
                                                      LNOC LRE 3'UTR2-->
L. noct gene  AATAACCGGTAGATTGTTTAGTATAAATTCTACTAAAGACGTTAGATACTACAGAACAAACATAGCGGTGTCAAACGGAATCGCTTGGAG
L. noct mRNA  AATAACCGGTAGATTGTTTAGTATAAATTCTACTAAAGACGTTAGATACTACAGAACAAACATAGCGGTGTCAAACGGAATCGCTTGGAG
              oIleThrGlyArgLeuPheSerIleAsnSerThrLysAspValArgTyrTyrArgThrAsnIleAlaValSerAsnGlyIleAlaTrpSe

              ....|....|....|....|....|....|....|....|....|....|....|....|....|....|....|....|....|....|
                       640       650       660       670       680       690       700       710       720
L. noct gene  TAAAGATTTGAAGAAAATGTATTATATTGATTCCATGACTAAAGTAATAGGTCAATACGATTTTGATGCTTCCAACAGATTGATCAGTAT
L. noct mRNA  TAAAGATTTGAAGAAAATGTATTATATTGATTCCATGACTAAAGTAATAGGTCAATACGATTTTGATGCTTCCAACAGATTGATCAGTA-
              rLysAspLeuLysLysMetTyrTyrIleAspSerMetThrLysValIleGlyGlnTyrAspPheAspAlaSerAsnArgLeuIleSerA

              ....|....|....|....|....|....|....|....|....|....|....|....|....|....|....|....|....|....|
                       730       740       750       760       770       780       790       800       810
L. noct gene  ACAGTAAATGATAAACATTCGTTACATATTTATGTTGTATATTTTCAGGTAATCGTCAAACCTTGTTTAGTCTTGAAAAGAATCGAATCC
L. noct mRNA  ---------------------------------------------------ATCGTCAAACCTTGTTTAGTCTTGAAAAGAATCGAATCC
                                                                 snArgGlnThrLeuPheSerLeuGluLysAsnArgIleP

              ....|....|....|....|....|....|....|....|....|....|....|....|....|....|....|....|....|....|
                       820       830       840       850       860       870       880       890       900
L. noct gene  CGGGATTTCCAGATGGTCAAACAATAGATACTGATGATAATTTATGGGTTGCTGTGTTCGGAGGAAACAGAGTGATAAAAATTAGTACTG
L. noct mRNA  CGGGATTTCCAGATGGTCAAACAATAGATACTGATGATAATTTATGGGTTGCTGTGTTCGGAGGAAACAGAGTGATAAAAATTAGTACTG
              roGlyPheProAspGlyGlnThrIleAspThrAspAspAsnLeuTrpValAlaValPheGlyGlyAsnArgValIleLysIleSerThrA
```

Fig. 14

Fig 14 continued

```
              ....|....|....|....|....|....|....|....|....|....|....|....|....|....|....|....|....|....|
                       910       920       930       940       950       960       970       980       990
L. noct gene  ATATACCTGAAACATTACTGTATACTGTAAACATTCCAGATTCGCAGGTGAGTAAATGGATTGACATTTTCAAATAATGTAGATGTTTAT
L. noct mRNA  ATATACCTGAAACATTACTGTATACTGTAAACATTCCAGATTCGCAGGT-----------------------------------------
              spIleProGluThrLeuLeuTyrThrValAsnIleProAspSerGlnVa

              ....|....|....|....|....|....|....|....|....|....|....|....|....|....|....|....|....|....|
                      1000      1010      1020      1030      1040      1050      1060      1070      1080
L. noct gene  TAGATAACCTCGGTTGCTTTTGGTGGACCAAACTTGGATGAATTGTACGTAACAAGTGGTGGTGGGAAAGTTTATAAGGTAACTGGTCTA
L. noct mRNA  -----AACCTCGGTTGCTTTTGGTGGACCAAACTTGGATGAATTGTACGTAACAAGTGGTGGTGGGAAAGTTTATAAGGTAACTGGTCTA
                   lThrSerValAlaPheGlyGlyProAsnLeuAspGluLeuTyrValThrSerGlyGlyGlyLysValTyrLysValThrGlyLeu

              ....|....|....|....|....|....|....|....|....|
                      1090      1100      1110      1120   1125
L. noct gene  GGTGTTCAAGGTTTACCTGCAAATAGGATTAAAATACATAATTAA                 SEQ ID NO 40
L. noct mRNA  GGTGTTCAAGGTTTACCTGCAAATAGGATTAAAATACATAATTAA                 SEQ ID NO 38
              GlyValGlnGlyLeuProAlaAsnArgIleLysIleHisAsnEnd                 SEQ ID NO 39
```

Fig. 14

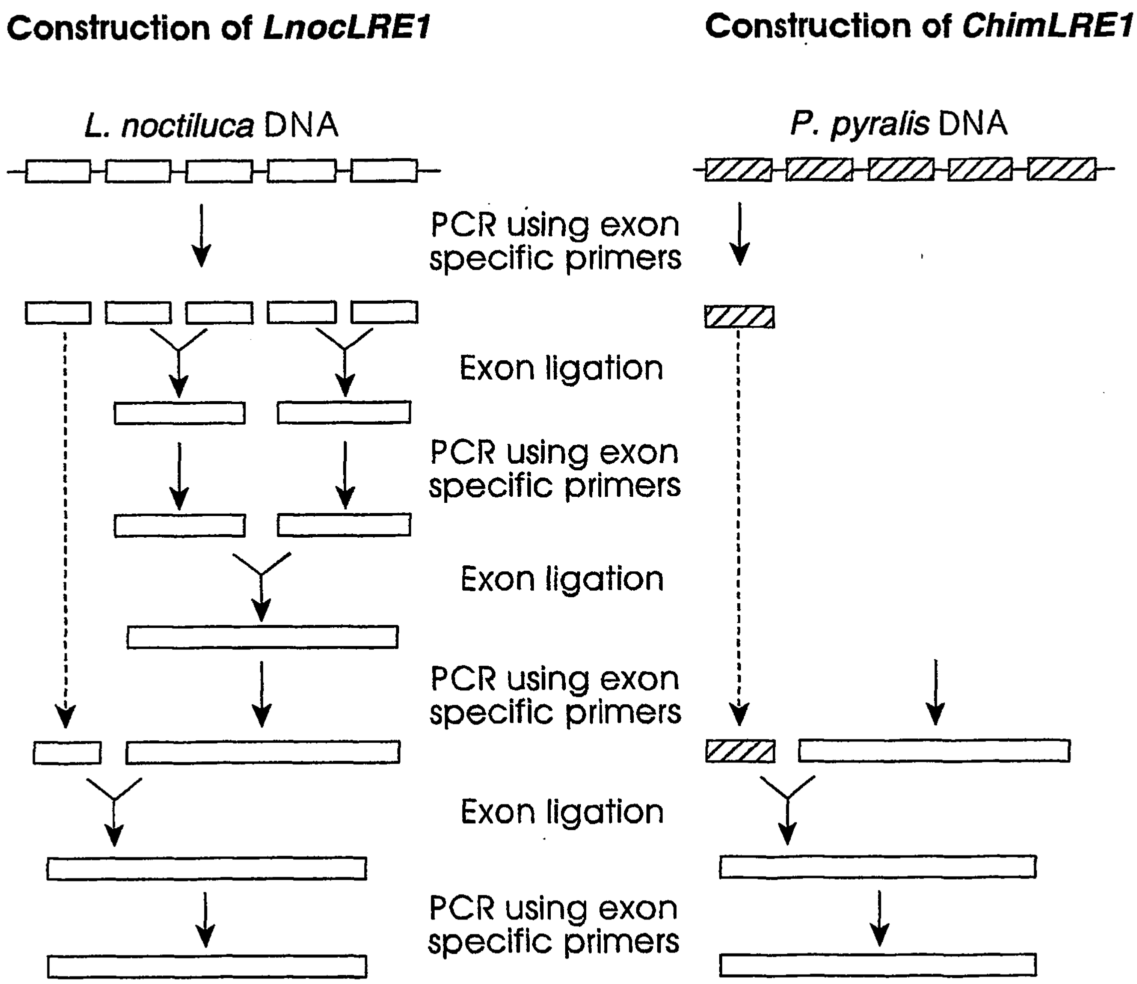
Construction of LnocLRE1
Construction of ChimLRE1
L. noctiluca DNA
P. pyralis DNA
PCR using exon specific primers
Exon ligation
PCR using exon specific primers
Exon ligation
PCR using exon specific primers
Exon ligation
PCR using exon specific primers

Fig. 15

```
1     ATG CAG TTT GGA GAA GGA CCT CAT TGG GAT GAT AAG ACA CAA AGT   45
1     Met Gln Phe Gly Glu Gly Pro His Trp Asp Asp Lys Thr Gln Ser   15

46    TTA TAT TTG GTG GAC CTT GTA GAT AAA TCT ATT CAC AAA TAT GTA   90
16    Leu Tyr Leu Val Asp Leu Val Asp Lys Ser Ile His Lys Tyr Val   30

91    CCA TCT CTT AAA AGA CAT ACC CAT ATA AAA GTA GAT AAA ATA CCG   135
31    Pro Ser Leu Lys Arg His Thr His Ile Lys Val Asp Lys Ile Pro   45

136   TCT TTG ATT ATT CCC ATA AGC GGT TGT TCA AAT CGT TTT CTT ATT   180
46    Ser Leu Ile Ile Pro Ile Ser Gly Cys Ser Asn Arg Phe Leu Ile   60

181   ACT TTA GAC CGA GAA ATC GCC ATC CTT ACT TGG GAT GGT ATA AGT   225
61    Thr Leu Asp Arg Glu Ile Ala Ile Leu Thr Trp Asp Gly Ile Ser   75

226   TCT ACA CCC ACA AGT ATA AAA ACA ATC GCT GTA GTT GAC AAT GAG   270
76    Ser Thr Pro Thr Ser Ile Lys Thr Ile Ala Val Val Asp Asn Glu   90

271   CCA GGT CTG GAA AGT AAT AGA ATT AAT GAT GGT AAA GCA GAT CAT   315
91    Pro Gly Leu Glu Ser Asn Arg Ile Asn Asp Gly Lys Ala Asp His   105

316   CTT GGT AAT CTG TGG GCA GGC ACC ATG GAC GTA AAT GGA TAT CCA   360
106   Leu Gly Asn Leu Trp Ala Gly Thr Met Asp Val Asn Gly Tyr Pro   120

361   ACG GGA CCA ATA ACC GGT AGA TTG TTT AGT ATA AAT TCT ACT AAA   405
121   Thr Gly Pro Ile Thr Gly Arg Leu Phe Ser Ile Asn Ser Thr Lys   135

406   GAC GTT AGA TAC TAC AGA ACA AAC ATA GCG GTG TCA AAC GGA ATC   450
136   Asp Val Arg Tyr Tyr Arg Thr Asn Ile Ala Val Ser Asn Gly Ile   150

451   GCT TGG AGT AAA GAT TTG AAG AAA ATG TAT TAT ATT GAT TCC ATG   495
151   Ala Trp Ser Lys Asp Leu Lys Lys Met Tyr Tyr Ile Asp Ser Met   165

496   ACT AAA GTA ATA GAT CAA TAC GAT TTT GAT GCT TCC AAC AGA TTG   540
166   Thr Lys Val Ile Asp Gln Tyr Asp Phe Asp Ala Ser Asn Arg Leu   180

541   ATC AGT AAT CGT CAA ACA TTG TTT AGT CTT GAA AAT AAT CAA ATC   585
181   Ile Ser Asn Arg Gln Thr Leu Phe Ser Leu Glu Asn Asn Gln Ile   195

586   CCG GGA TTT CCA GAT GGT CAA ACA ATT GAT ACT GAT GAT AAT TTA   630
196   Pro Gly Phe Pro Asp Gly Gln Thr Ile Asp Thr Asp Asp Asn Leu   210

631   TGG GTT GCT GTG TTC GAA GGA AAC AGA GTG ATA AAA ATT AGT ACT   675
211   Trp Val Ala Val Phe Glu Gly Asn Arg Val Ile Lys Ile Ser Thr   225

676   CAT ATA CCT GAA ACA TTA CTG TAT ACT GTA AAC ATT CCA GAT TCG   720
226   His Ile Pro Glu Thr Leu Leu Tyr Thr Val Asn Ile Pro Asp Ser   240

721   CTG ATA ACC TCG GTT GCT TTT GGT GGA CCA AAC TTG GAT GAA TTG   765
241   Leu Ile Thr Ser Val Ala Phe Gly Gly Pro Asn Leu Asp Glu Leu   255

766   TAC GTA ACA GGT GGT GGT AAC TTT CTG AAT GGG AAA GTT TAT AAA   810
256   Tyr Val Thr Gly Gly Gly Asn Phe Leu Asn Gly Lys Val Tyr Lys   270

811   GTA TCT GGT CTA GGT GTT CAA GGT TTA CCT GCA AAT AGG ATT AAA   855
271   Val Ser Gly Leu Gly Val Gln Gly Leu Pro Ala Asn Arg Ile Lys   285

856   ATA CAT AAT   864          SEQ ID NO 58
286   Ile His Asn                SEQ ID NO 59
```

Fig. 16

## DNA sequence for *ChimLRE1* and punative amino acid sequence

1 ATG GGG CCA GTT GTT GAA AAA ATT GCA GAA CTT GGC AAG TAT ACA 45
1 Met Gly Pro Val Val Glu Lys Ile Ala Glu Leu Gly Lys Tyr Thr 15

46 GTT GGA GAA GGT CCT CAC TGG GAT CAT GAA ACT CAG ACC TTA TAT 90
16 Val Gly Glu Gly Pro His Trp Asp His Glu Thr Gln Thr Leu Tyr 30

91 TTC GTC GAC ACC GTA GAG AAA ACT TTT CAT AAA TAT GTA CCT TCT 135
31 Phe Val Asp Thr Val Glu Lys Thr Phe His Lys Tyr Val Pro Ser 45

136 CAG AAA AAA TAC ACG TTT TGT AAA GTA GAT AAA ATA CCG TCT TTG 180
46 Gln Lys Lys Tyr Thr Phe Cys Lys Val Asp Lys Ile Pro Ser Leu 60

181 ATT ATT CCC ATA AGC GGT TGT TCA AAT CGT TTT CTT ATT ACT TTA 225
61 Ile Ile Pro Ile Ser Gly Cys Ser Asn Arg Phe Leu Ile Thr Leu 75

226 GAC CGA GAA ATC GCC ATC CTT ACT TGG GAT GGT ATA AGT TCT ACA 270
76 Asp Arg Glu Ile Ala Ile Leu Thr Trp Asp Gly Ile Ser Ser Thr 90

271 CCC ACA AGT ATA AAA ACA ATC GCT GTA GTT GAC AAT GAG CCA GGT 315
91 Pro Thr Ser Ile Lys Thr Ile Ala Val Val Asp Asn Glu Pro Gly 105

316 CTG GAA AGT AAT AGA ATT AAT GAT GGT AAA GCA GAT CAT CTT GGT 360
106 Leu Glu Ser Asn Arg Ile Asn Asp Gly Lys Ala Asp His Leu Gly 120

361 AAT CTG TGG GCA GGC ACC ATG GAC GTA AAT GGA TAT CCA ACG GGA 405
121 Asn Leu Trp Ala Gly Thr Met Asp Val Asn Gly Tyr Pro Thr Gly 135

406 CCA ATA ACC GGT AGA TTG TTT AGT ATA AAT TCT ACT AAA GAC GTT 450
136 Pro Ile Thr Gly Arg Leu Phe Ser Ile Asn Ser Thr Lys Asp Val 150

451 AGA TAC TAC AGA ACA AAC ATA GCG GTG TCA AAC GGA ATC GCT TGG 495
151 Arg Tyr Tyr Arg Thr Asn Ile Ala Val Ser Asn Gly Ile Ala Trp 165

496 AGT AAA GAT TTG AAG AAA ATG TAT TAT ATT GAT TCC ATG ACT AAA 540
166 Ser Lys Asp Leu Lys Lys Met Tyr Tyr Ile Asp Ser Met Thr Lys 180

541 GTA ATA GAT CAA TAC GAT TTT GAT GCT TCC AAC AGA TTG ATC AGT 585
181 Val Ile Asp Gln Tyr Asp Phe Asp Ala Ser Asn Arg Leu Ile Ser 195

586 AAT CGT CAA ACA TTG TTT AGT CTT GAA AAT AAT CAA ATC CCG GGA 630
196 Asn Arg Gln Thr Leu Phe Ser Leu Glu Asn Asn Gln Ile Pro Gly 210

631 TTT CCA GAT GGT CAA ACA ATT GAT ACT GAT GAT AAT TTA TGG GTT 675
211 Phe Pro Asp Gly Gln Thr Ile Asp Thr Asp Asp Asn Leu Trp Val 225

676 GCT GTG TTC GAA GGA AAC AGA GTG ATA AAA ATT AGT ACT CAT ATA 720
226 Ala Val Phe Glu Gly Asn Arg Val Ile Lys Ile Ser Thr His Ile 240

721 CCT GAA ACA TTA CTG TAT ACT GTA AAC ATT CCA GAT TCG CTG ATA 765
241 Pro Glu Thr Leu Leu Tyr Thr Val Asn Ile Pro Asp Ser Leu Ile 255

766 ACC TCG GTT GCT TTT GGT GGA CCA AAC TTG GAT GAA TTG TAC GTA 810
256 Thr Ser Val Ala Phe Gly Gly Pro Asn Leu Asp Glu Leu Tyr Val 270

Fig. 17

Fig 17 continued

```
811   ACA GGT GGT GGT AAC TTT CTG AAT GGG AAA GTT TAT AAA GTA TCT   855
271   Thr Gly Gly Gly Asn Phe Leu Asn Gly Lys Val Tyr Lys Val Ser   285

856   GGT CTA GGT GTT CAA GGT TTA CCT GCA AAT AGG ATT AAA ATA CAT   900
286   Gly Leu Gly Val Gln Gly Leu Pro Ala Asn Arg Ile Lys Ile His   300

901   AAT   903                SEQ ID NO 60
301   Asn                      SEQ ID NO 61
```

Fig. 17

```
          ....|....| ....|....| ....|....| ....|....| ....|....| ....|....| ....|....| ....|....| ....|....| ....|....|
                  10         20         30         40         50         60         70         80         90        100
lnoc LRE  M--------- ----QFGEGP HWDDKTQSLY LVDLVDKSIH KYVPSLKRHT HIKVDKIPSL IIPISGCSNR FLITLDREIA ILTWDGISST PTSIKTIAVV
chim LRE  MGPVVEKIAE LGKYTVGEGP HWDHETQTLY FVDTVEKTFH KYVPSQKKYT FCKVDKIPSL IIPISGCSNR FLITLDREIA ILTWDGISST PTSIKTIAVV
Lcru LRE  MAPTVEQIVE LGTYLLAESP HWDDETQSLY FVDIVGRSVN KYVPTTKTHT QLKFDKNPSF IIPVKGCSDR FIVSLEREIN LLTWDGASSA PSKIEKIAVF
Llat LRE  MSPVIEQITE VDNFQIGEGP HWDTETQSLY FVDILEKSIH KYVPSTKQHT KMILNKRPSF IIPIKETSDR FVISLERDIC VLTWDGVSAT PSHLETIVTV
Ppyr LRE  MGPVVEKIAE LGKYTVGEGP HWDHETQTLY FVDTVEKTFH KYVPSQKKYT FCKVDKLVSF IIPLAGSPGR FVVSLEREIA ILTWDGVSAA PTSIE--AIV

          ....|....| ....|....| ....|....| ....|....| ....|....| ....|....| ....|....| ....|....| ....|....| ....|....|
                 110        120        130        140        150        160        170        180        190        200
lnoc LRE  DNEPGLESNR INDGKADHLG NLWAGTMDVN -GYPTG-PIT GRLFSINSTK DVRYYRTNIA VSNGIAWSKD LKKMYYIDSM TKVIDQYDFD ASNRLISNRQ
chim LRE  DNEPGLESNR INDGKADHLG NLWAGTMDVN -GYPTG-PIT GRLFSINSTK DVRYYRTNIA VSNGIAWSKD LKKMYYIDSM TKVIDQYDFD ASNRLISNRQ
Lcru LRE  DNTPEKSENR LNDGKADPLG NLWAGTMNMG SDHTTGTPVR GTLSSL-SNK QVKEHVSEVC ISNGLAWSKD LKKFYYIDSA VRQVDQFDFD AKNLSLSNRQ
Llat LRE  DTGIEG--NT FNDGKADAFG NLWAGTLYSK FDIEKQGPNT GTLYSL-SNK QLRKHISNIF LSNGLAWNKD SKKFYFIDSN KRTIDQFDYD SENLIISNCQ
Ppyr LRE  NVEPHIKNNR LNDGKADPLG NLWTGTMAID AGLPVG-PVT GSLYHLGADK KVKMHESNIA IANGLAWSND LKKMYYIDSG KRRVDEYDYD ASTLSISNQR

          ....|....| ....|....| ....|....| ....|....| ....|....| ....|....| ....|....| ....|....| ....|....| ....|....|
                 210        220        230        240        250        260        270        280        290        300
lnoc LRE  TLFSLENNQI PGFPDGQTID TDDNLWVAVF EGNRVIKIST HIPETLLYTV NIPDSLITSV AFGGPNLDEL YVTGGG---- ------NFLN GKVYKVSGLG
chim LRE  TLFSLENNQI PGFPDGQTID TDDNLWVAVF EGNRVIKIST HIPETLLYTV NIPDSLITSV AFGGPNLDEL YVTGGG---- ------NFLN GKVYKVSGLG
Lcru LRE  PLFTFDKHGI MGSPDGQTID AEGNLWVATC QGDKVLKIDT STPETLLGIV EIPEHQVTSV CIGGAELNVL YVTTASIKLP GAD-ETKPMK GAIYKVTGLG
Llat LRE  PLFTLDKHGI QGLPDAQTID ENDNLWVAIV RGGKVINIGT KQPESLLGVI NMPESLITSV CFGGSKLDEL YVTTSGIKEY ETD-STKLVK GGLYRVTGLG
Ppyr LRE  PLFTFEKHEV PGYPDGQTID EEGNLWVAVF QGQRIIKIST QQPEVLLDTV KIPDPQVTSV AFGGPNLDEL HVTSAGLQLD DSSLDKSLVN GHVYRVTGLG

          ....|....| ...
                 310
lnoc LRE  VQGLPANRIK IHN          SEQ ID NO 59
chim LRE  VQGLPANRIK IHN          SEQ ID NO 61
Lcru LRE  VKGLPGDRVK L--          SEQ ID NO 62
Llat LRE  VKGLPAHRFS L--          SEQ ID NO 63
Ppyr LRE  VKGFAGVKVK L--          SEQ ID NO 1
```

Fig. 18

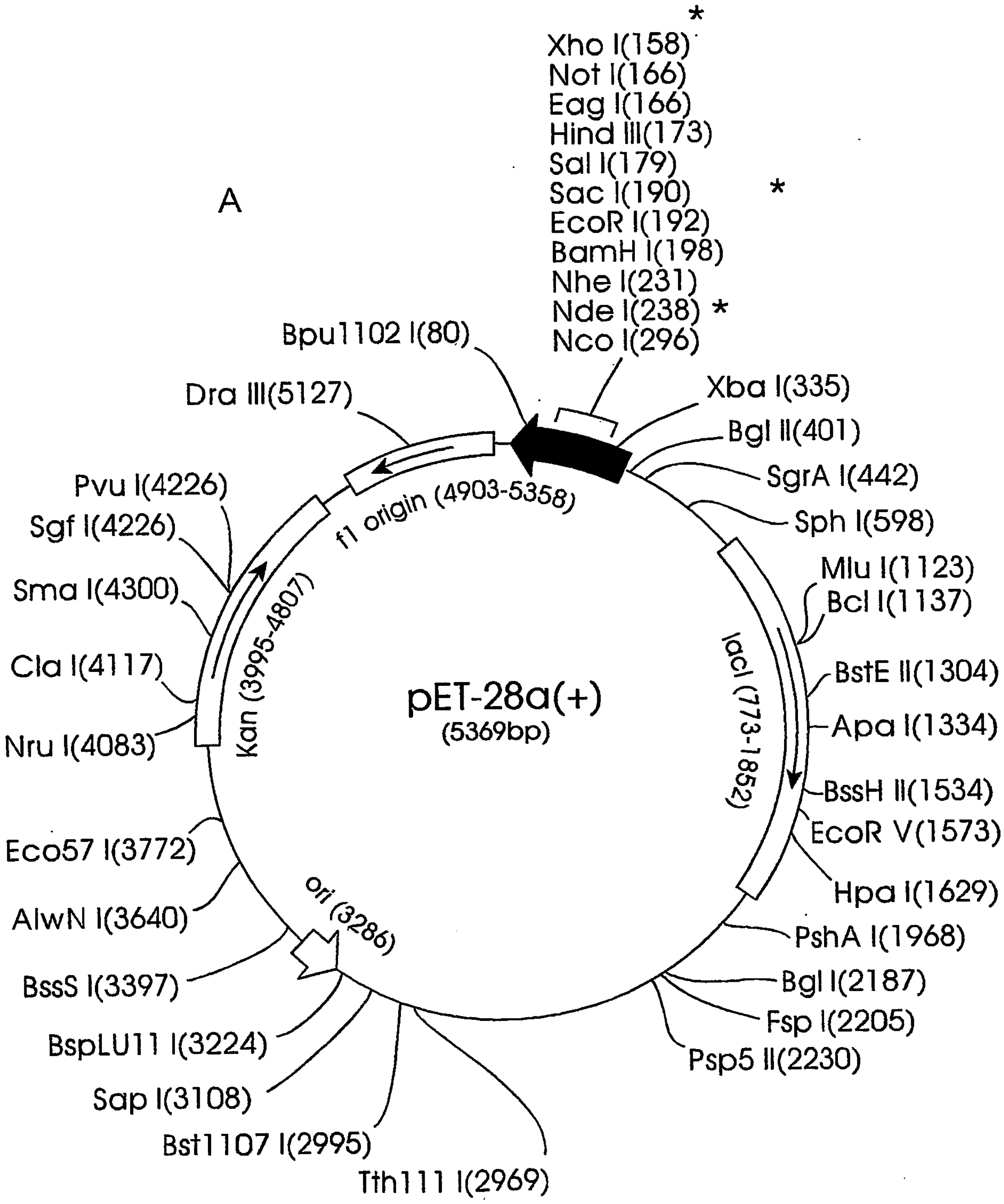
A
*
Xho I(158)
Not I(166)
Eag I(166)
Hind III(173)
Sal I(179)
Sac I(190)
*
EcoR I(192)
BamH I(198)
Nhe I(231)
Nde I(238) *
Nco I(296)
Bpu1102 I(80)
Xba I(335)
Dra III(5127)
Bgl II(401)
SgrA I(442)
Pvu I(4226)
Sgf I(4226)
f1 origin (4903-5358)
Sph I(598)
Mlu I(1123)
Bcl I(1137)
Sma I(4300)
Kan (3995-4807)
lacI (773-1852)
Cla I(4117)
BstE II(1304)
pET-28a(+)
(5369bp)
Apa I(1334)
Nru I(4083)
BssH II(1534)
EcoR V(1573)
Eco57 I(3772)
Hpa I(1629)
AlwN I(3640)
ori (3286)
PshA I(1968)
BssS I(3397)
Bgl I(2187)
Fsp I(2205)
BspLU11 I(3224)
Psp5 II(2230)
Sap I(3108)
Bst1107 I(2995)
Tth111 I(2969)

Fig. 19

Fig 19 continued

B

T7 Promoter primer #69348-3

pET upstream primer #69214-3

*Bgl*11 — T7 promoter — lac operator — *Xba*1 — rbs

AGATCTCGATCCCGCGAAATTAATACGACTCACTATAGGGGAATTGTGAGCGGATAACAATTCCCCTCTAGAAATAATTTTGTTTAACTTTAAGAAGGAGA

*Nco*1 — His*Tag — *Nde*1 — *Nhe*1 — T7*Tag

TATACCATGGGCAGCAGCCATCATCATCATCATCACAGCAGCGGCCTGGTGCCGCGCGGCAGCCATATGGCTAGCATGACTGGTGGACAGCAA

**MetGlySerSerHisHisHisHisHisHisSerSerGlyLeuValProArgGlySerHisMetAlaSerMetThrGlyGlyGlnGln**

thrombin

*Bam*H1 — *Eco*R1 — *Sac*1 — *Sal*1 — *Hind*111 — *Eag*1 *Not*1 — *Xho*1 — His*Tag

ATGGGTCGCGGATCCGAATTCGAGCTCCGTCGACAAGCTTGCGGCCGCACTCGAGCACCACCACCACCACCACTGAGATCCGGCTGCTAACAAAGCCC pET-28a(*)

**MetGlyArgGlySerGluPheGluLeuArgArgGlnAlaCysGlyArgThrArgAlaProProProProProLeuArgSerGlyCysEnd**

...GGTCGGGATCCGAATTCGAGCTCCGTCGACAAGCTTGCGGCCGCACTCGAGCACCACCACCACCACCACTGAGATCCGGCTGCTAACAAAGCCC pET-28b(*)

**...GlyArgAspProAsnSerSerSerValAspLysLeuAlaAlaAlaLeuGluHisHisHisHisHisHisEnd**

...GGTCGGATCCGAATTCGAGCTCCGTCGACAAGCTTGCGGCCGCACTCGAGCACCACCACCACCACCACTGAGATCCGGCTGCTAACAAAGCCC pET-28c(*)

**...GlyArgIleArgIleArgAlaProSerThrSerLeuArgProHisSerSerThrThrThrThrThrThrGluIleArgLeuLeuThrLysPro...**

*Bpu*11021 — T7 terminator

GAAAGGAAGCTGAGTTGGCTGCTGCCACCGCTGAGCAATAACTAGCATAACCCCTTGGGGCCTCTAAACGGGTCTTGAGGGGTTTTTTG

T7 terminator primer #69337-3

Fig. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9417202 A
- WO 9602665 A
- WO 9622376 A
- WO 9846729 A
- US 5814504 A
- US P5814504 A
- EP 0528448 A
- WO 9525798 A
- WO 0024878 A
- WO 0131028 A
- WO 9914336 A
- WO 0120002 A


### Non-patent literature cited in the description


- **DEMENTIEVA et al.** *Biochemistry (Moscow,* 1996, vol. 61 (7
- **LIPMAN, D.J. ; PEARSON, W.R.** Rapid and Sensitive Protein Similarity Searches. *Science,* 1985, vol. 227, 1435-1441
- *Nature,* vol. 227 (259), 680-85
- **BRADFORD, M. M.** A rapid and sensitive method for the quantification of microgram quantities of protein utilizing the principle of protein-dye binding. *Anal. Biochem,* 1976, vol. 72, 248-54